(19) Europäisches Patentamt
European Patent Office
Office européen des brevets

(11) EP 1 756 089 B1

(12) **EUROPEAN PATENT SPECIFICATION**

(45) Date of publication and mention of the grant of the patent:
**22.10.2008 Bulletin 2008/43**

(21) Application number: **05749653.1**

(22) Date of filing: **24.05.2005**

(51) Int Cl.:
*C07D 403/04* (2006.01) *C07D 233/84* (2006.01)
*C07D 405/04* (2006.01) *C07D 413/04* (2006.01)
*C07D 417/04* (2006.01) *C07D 401/04* (2006.01)
*A61K 31/4439* (2006.01) *A61K 31/506* (2006.01)
*A61K 31/427* (2006.01) *A61K 31/4164* (2006.01)
*A61K 31/4245* (2006.01) *A61K 31/4178* (2006.01)

(86) International application number:
**PCT/EP2005/052373**

(87) International publication number:
**WO 2005/118574 (15.12.2005 Gazette 2005/50)**

(54) **MERCAPTOIMIDAZOLES AS CCR2 RECEPTOR ANTAGONISTS**

MERCAPTOIMIDAZOLE ALS CCR2-REZEPTORANTAGONISTEN

MERCAPTOIMIDAZOLES UTILISES COMME ANTAGONISTES DU RECEPTEUR CCR2

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **26.05.2004 PCT/EP2004/050935**

(43) Date of publication of application:
**28.02.2007 Bulletin 2007/09**

(73) Proprietor: **Janssen Pharmaceutica NV**
**2340 Beerse (BE)**

(72) Inventors:
- **BOECKX, Gustaaf Maria**
**2340 Beerse (BE)**
- **VAN LOMMEN, Guy Rosalia Eugeen**
**2340 Beerse (BE)**
- **DOYON, Julien Georges Pierre-Olivier**
**2340 Beerse (BE)**
- **COESEMANS, Erwin**
**2340 Beerse (BE)**

(74) Representative: **Vervoort, Liesbeth**
**Janssen Pharmaceutica N.V.**
**Turnhoutseweg 30**
**2340 Beerse (BE)**

(56) References cited:
**WO-A-02/066458**
**WO-A-03/097633**
**WO-A-20/04069809**
**WO-A-20/04069810**
**US-A- 3 850 944**
**US-A1- 2003 149 081**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 756 089 B1

## Description

The present invention concerns mercaptoimidazole derivatives having CCR2 receptor antagonistic properties. The invention further relates to methods for their preparation and pharmaceutical compositions comprising them. The invention also relates to the use of said compounds for the manufacture of a medicament for the prevention or the treatment of diseases mediated through activation of the CCR2 receptor, in particular the CCR2B receptor.

WO 02/066458 and WO 03/097633 describe 2-thio-substituted imidazole derivatives having immunomodulating and/or inhibiting activity on the release of cytokines, especially TNF-α and IL-β.
FR 1,487,326 relates to thio-imidazole derivatives useful as analgetic and for its vasodilatation activity.
FR 6,751 M describes thio-imidazole derivatives as sedatives and analgesics.
US 3,850,944 describes 2-mercapto-5-(3-pyridyl)-imidazole derivatives having antiinflammatory activity.
EP 0,277,384 describes 1H-imidazole-5-carboxylic acid derivatives for controlling weeds.
W02004/069809 and WO 2004/069810 relate to mercaptoimidazoles as CCR2 receptor antagonists.
US2003/0149081 relates to pyrrolodinone derivatives that antagonize CCR2.

The compounds of the invention differ from the prior art compounds in structure, in their pharmacological activity and/or pharmacological potency.

One aspect of the present invention relates to a compound of formula

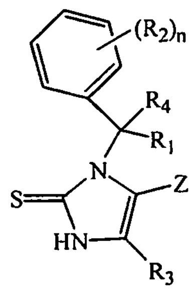

(I)

,

a *N*-oxide, a pharmaceutically acceptable addition salt, a quaternary amine or a stereochemically isomeric form thereof, wherein

| | |
|---|---|
| $R_1$ | represents $C_{1-6}$alkyl; |
| each $R_2$ | independently represents halo or polyhalo$C_{1-6}$alkyl; |
| $R_3$ | represents hydrogen, cyano, $C_{1-6}$alkyl optionally substituted with hydroxy or $C_{1-6}$alkyloxy, C(=O)-O-$R_5$, C(=O)-N$R_{6a}R_{6b}$, C(=S)-N$R_{6a}R_{6b}$, $S(=C)_2$-N$R_{6a}R_{6b}$ or C(=O)-$R_7$; |
| $R_4$ | represents hydrogen; |
| $R_5$ | represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-}$6alkyl, mono-or di($C_{1-4}$alkyl)aminocarbonyl$C_{1-6}$alkyl or aryl; |
| $R_{6a}$ and $R_{6b}$ | each independently represent hydrogen, $C_{1-6}$alkyl, amino, mono-or di($C_{1-4}$alkyl)amino, arylNH-, amino$C_{1-6}$alkyl, mono-or di($C_{1-4}$alkyl)amino-$C_{1-6}$alkyl, $C_{1-6}$alkylcarbonylamino, aminocarbonylamino, $C_{1-6}$alkyloxy, carbonylamino or hydroxy$C_{1-6}$alkyl; or |
| $R_{6a}$ and $R_{6b}$ | taken together with the nitrogen to which they are attached form pyrrolidinyl, imidazolidinyl, pyrazolidinyl, piperidinyl, piperazinyl, morpholinyl, thiomorpholinyl or piperazinyl substituted with $C_{1-6}$alkyl; |
| $R_7$ | represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl, aryl or heteroaryl; |
| Z | represents a cyclic ring system selected from |

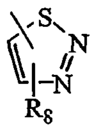

(a-1)

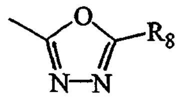

(a-2)

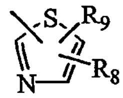

(a-3)

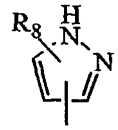

(a-4)

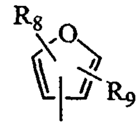

(a-5)

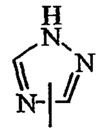

(a-6)

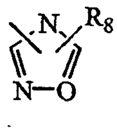

(a-7)

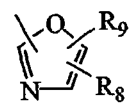

(a-8)

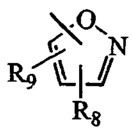

(a-9)

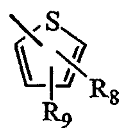

(a-10)

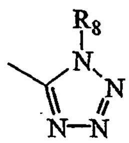

(a-11)

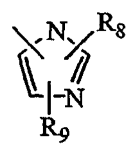

(a-12)

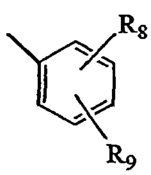

(a-13)

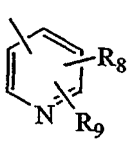

(a-14)

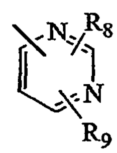

(a-15)

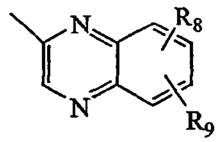

(a-16)

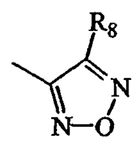

(a-17)

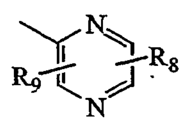

(a-18)

each $R_8$ independently represents hydrogen, halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di ($C_{1-4}$alkyl)amino, hydroxy$C_{1-6}$alkylamino, aryl, aryloxy, piperidinyl, piperidinylamino, morpholinyl, piperazinyl or nitro;

each $R_9$ independently represents hydrogen, halo or $C_{1-6}$alkyl;

n is 1, 2, 3, 4 or 5;

aryl represents phenyl or phenyl substituted with one, two, three, four or five substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino, phenyloxy or nitro;

heteroaryl represents furanyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, each of said heterocycles optionally being substituted with one or two substituents each independently selected from halo,

$C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino or nitro.

The present invention also relates to the use of a compound for the manufacture of a medicament for preventing or treating diseases mediated through activation of the CCR2 receptor, in particular for preventing or treating inflammatory diseases, wherein said compound is a compound of formula (I)

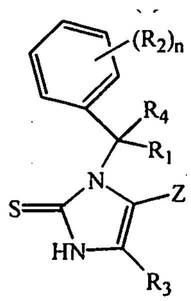

(I)

,

a *N*-oxide, a pharmaceutically acceptable addition salt, a quaternary amine or a stereochemically isomeric form thereof, wherein

| | |
|---|---|
| $R_1$ | represents $C_{1-6}$alkyl; |
| each $R_2$ | independently represents halo or polyhalo$C_{1-6}$alkyl; |
| $R_3$ | represents hydrogen, cyano, $C_{1-6}$alkyl optionally substituted with hydroxy or $C_{1-6}$alkyloxy, C(=O)-O-$R_5$, C(=O)-$NR_{6a}R_{6b}$, C(=S)-$NR_{6a}R_{6b}$, $S(=O)_2$-$NR_{6a}R_{6b}$ or C(=O)-$R_7$; |
| $R_4$ | $R_5$ represents hydrogen; represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl or aryl; |
| $R_{6a}$ and $R_{6b}$ | each independently represent hydrogen, $C_{1-6}$alkyl, amino, mono-or di($C_{1-4}$alkyl)amino, arylNH-, amino$C_{1-6}$alkyl, mono-or di($C_{1-4}$alkyl)amino $C_{1-6}$alkyl, $C_{1-6}$alkylcarbonylamino, aminocarbonylamino, $C_{1-6}$alkyloxy, carbonylamino or hydroxy$C_{1-6}$alkyl; or |
| $R_{6a}$ and $R_{6b}$ | taken together with the nitrogen to which they are attached form pyrrolidinyl, imidazolidinyl, pyrazolidinyl, piperidinyl, piperazinyl, morpholinyl, thiomorpholinyl or piperazinyl substituted with $C_{1-6}$alkyl; |
| $R_7$ | represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl, aryl or heteroaryl; |
| Z | represents a cyclic ring system selected from |

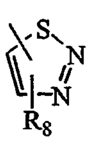
(a-1)

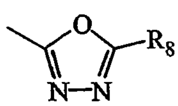
(a-2)

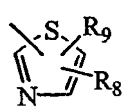
(a-3)

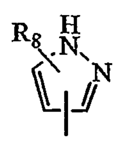
(a-4)

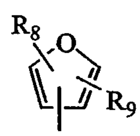
(a-5)

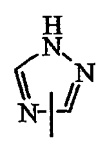
(a-6)

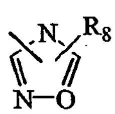
(a-7)

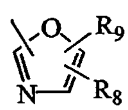
(a-8)

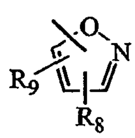
(a-9)

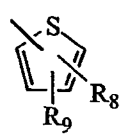
(a-10)

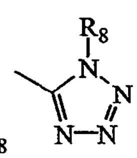
(a-11)

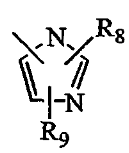
(a-12)

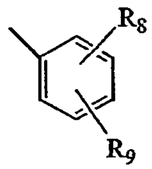

(a-13)

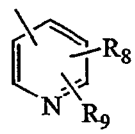

(a-14)

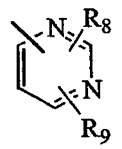

(a-15)

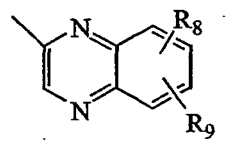

(a-16)

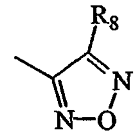

(a-17)

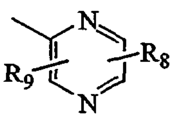

(a-18)

each $R_8$ independently represents hydrogen, halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di ($C_{1-4}$alkyl)amino, hydroxy$C_{1-6}$alkylamino, aryl, aryloxy, piperidinyl, piperidinylamino, morpholinyl, piperazinyl or nitro;

each $R_9$ independently represents hydrogen, halo or $C_{1-6}$alkyl;

n is 1, 2, 3, 4 or 5;

aryl represents phenyl or phenyl substituted with one, two, three, four or five substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino, phenyloxy or nitro;

heteroaryl represents furanyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, each of said heterocycles optionally being substituted with one or two substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino or nitro.

As used hereinbefore or hereinafter $C_{1-4}$alkyl as a group or part of a group defines straight or branched chain saturated hydrocarbon radicals having from 1 to 4 carbon atoms such as methyl, ethyl, propyl, 1-methylethyl, butyl; $C_{1-6}$alkyl as a group or part of a group defines straight or branched chain saturated hydrocarbon radicals having from 1 to 6 carbon atoms such as the group defined for $C_{1-4}$alkyl and pentyl, hexyl, 2-methylbutyl and the like; $C_{3-7}$cycloalkyl is generic to cyclopropyl, cyclobutyl, cyclopentyl, cyclohexyl and cycloheptyl; $C_{2-6}$alkenyl defines straight and branched chain hydrocarbon radicals having from 2 to 6 carbon atoms containing a double bond such as ethenyl, propenyl, butenyl, pentenyl, hexenyl and the like; $C_{2-6}$alkynyl defines straight and branched chain hydrocarbon radicals having from 2 to 6 carbon atoms containing a triple bond such as ethynyl, propynyl, butynyl, pentynyl, hexynyl and the like.

As used hereinbefore, the term (=O) forms a carbonyl moiety when attached to a carbon atom, a sulfoxide moiety when attached to a sulfur atom and a sulfonyl moiety when two of said terms are attached to a sulfur atom.

The term halo is generic to fluoro, chloro, bromo and iodo. As used in the foregoing or hereinafter, polyhalomethyl as a group or part of a group is defined as mono- or polyhalosubstituted methyl, in particular methyl with one or more fluoro atoms, for example, difluoromethyl or trifluoromethyl; polyhalo$C_{1-6}$alkyl as a group or part of a group is defined as mono- or polyhalosubstituted $C_{1-6}$alkyl, for example, the groups defined in polyhalomethyl, 1,1-difluoro-ethyl and the like. In case more than one halogen atoms are attached to an alkyl group within the definition of polyhalomethyl or polyhalo$C_{1-6}$alkyl, they may be the same or different.

The term heteroaryl in the definition of $R_1$ or $R_7$ is meant to include all the possible isomeric forms of the heterocycles, for instance, pyrrolyl comprises 1*H*-pyrrolyl and 2*H*-pyrrolyl.

The aryl, heteroaryl, heterocyclic ring systems or cyclic ring systems listed in the definitions of the substituents of the compounds of formula (I) (see for instance $R_1$, $R_5$, $R_7$ and Z) as mentioned hereinabove or hereinafter may be attached to the remainder of the molecule of formula (I) through any ring carbon or heteroatom as appropriate, if not otherwise specified. Thus, for example, when heteroaryl is imidazolyl, it may be 1-imidazolyl, 2-imidazolyl, 4-imidazolyl

and the like.

When any variable (eg. $R_{6a}$, $R_{6b}$) occurs more than one time in any constituent, each definition is independent.

Lines drawn from substituents into ring systems indicate that the bond may be attached to any of the suitable ring atoms. When the lines are drawn into bicyclic ring systems, it indicates that the bond may be attached to any of the suitable ring atoms of any one of the two cycles of the bicyclic ring system.

For therapeutic use, salts of the compounds of formula (I) are those wherein the counterion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not are included within the ambit of the present invention.

The pharmaceutically acceptable addition salts as mentioned hereinabove are meant to comprise the therapeutically active non-toxic acid addition salt forms which the compounds of formula (I) are able to form. The latter can conveniently be obtained by treating the base form with such appropriate acids as inorganic acids, for example, hydrohalic acids, e.g. hydrochloric, hydrobromic and the like; sulfuric acid; nitric acid; phosphoric acid and the like; or organic acids, for example, acetic, propanoic, hydroxyacetic, 2-hydroxypropanoic, 2-oxopropanoic, oxalic, malonic, succinic, maleic, fumaric, malic, tartaric, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. Conversely the salt form can be converted by treatment with alkali into the free base form.

The compounds of formula (I) containing acidic protons may be converted into their therapeutically active non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-*n*-butylamine, pyrrolidine, piperidine, morpholine, trimethylamine, triethylamine, tripropylamine, quinuclidine, pyridine, quinoline and isoquinoline, the benzathine, *N*-methyl-D-glucamine, 2-amino-2-(hydroxymethyl)-1,3-propanediol, hydrabamine salts, and salts with amino acids such as, for example, arginine, lysine and the like. Conversely the salt form can be converted by treatment with acid into the free acid form.

The term addition salt also comprises the hydrates and solvent addition forms which the compounds of formula (I) are able to form. Examples of such forms are e.g. hydrates, alcoholates and the like.

The term "quaternary amine" as used hereinbefore defines the quaternary ammonium salts which the compounds of formula (I) are able to form by reaction between a basic nitrogen of a compound of formula (I) and an appropriate quaternizing agent, such as, for example, an optionally substituted alkylhalide, arylhalide or arylalkylhalide, e.g. methyliodide or benzyliodide. Other reactants with good leaving groups may also be used, such as alkyl trifluoromethanesulfonates, alkyl methanesulfonates, and alkyl p-toluenesulfonates. A quaternary amine has a positively charged nitrogen. Pharmaceutically acceptable counterions include chloro, bromo, iodo, trifluoroacetate and acetate. The counterion of choice can be introduced using ion exchange resins.

The *N*-oxide forms of the present compounds are meant to comprise the compounds of formula (I) wherein one or several tertiary nitrogen atoms are oxidized to the so-called *N*-oxide.

It will be appreciated that some of the compounds of formula (I) and their *N*-oxides, addition salts, quaternary amines and stereochemically isomeric forms may contain one or more centers of chirality and exist as stereochemically isomeric forms.

The term "stereochemically isomeric forms" as used hereinbefore defines all the possible stereoisomeric forms which the compounds of formula (I), and their *N*-oxides, addition salts, quaternary amines or physiologically functional derivatives may possess. Unless otherwise mentioned or indicated, the chemical designation of compounds denotes the mixture of all possible stereochemically isomeric forms, said mixtures containing all diastereomers and enantiomers of the basic molecular structure as well as each of the individual isomeric forms of formula (I) and their *N*-oxides, salts, solvates or quaternary amines substantially free, *i.e.* associated with less than 10%, preferably less than 5%, in particular less than 2% and most preferably less than 1% of the other isomers. Thus, when a compound of formula (I) is for instance specified as (E), this means that the compound is substantially free of the (Z) isomer.

In particular, stereogenic centers may have the R- or S-configuration; substituents on bivalent cyclic (partially) saturated radicals may have either the *cis*- or *trans*-configuration. Compounds encompassing double bonds can have an E (entgegen) or Z (zusammen) -stereochemistry at said double bond. The terms cis, trans, R, S, E and Z are well known to a person skilled in the art.

Stereochemically isomeric forms of the compounds of formula (I) are obviously intended to be embraced within the scope of this invention.

Some of the compounds of formula (I) may also exist in their tautomeric form. Such forms although not explicitly indicated in the above formula (I) are intended to be included within the scope of the present invention. For instance, it is intended that formula (I) includes the tautomeric form of

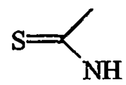

being

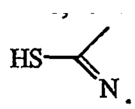

Thus, the compounds of the present invention include compounds of formula

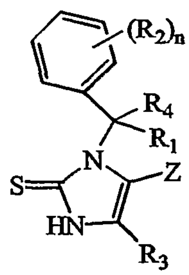

and

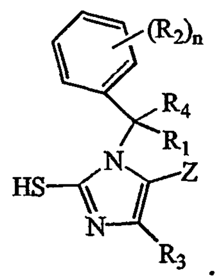

Whenever used hereinafter, the term "compounds of formula (I)" is meant to also include their *N*-oxide forms, their addition salts, their quaternary amines and their stereochemically isomeric forms. Of special interest are those compounds of formula (I) which are stereochemically pure.

Whenever used hereinbefore or hereinafter that substituents can be selected each independently out of a list of numerous definitions, such as for example for $R_{6a}$ or $R_{6b}$, all possible combinations are intended which are chemically possible.

A first interesting embodiment of the present invention relates to a compound of formula

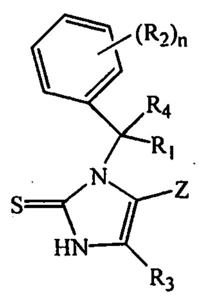

(I)

,

wherein

| | |
|---|---|
| $R_1$ | represents $C_{1-6}$alkyl; |
| each $R_2$ | independently represents halo or polyhalo$C_{1-6}$alkyl; |
| $R_3$ | represents hydrogen, cyano, $C_{1-6}$alkyl optionally substituted with hydroxy or $C_{1-6}$alkyloxy, C(=O)-O-$R_5$, C(=O)-$NR_{6a}R_{6b}$, C(=S)-$NR_{6a}R_{6b}$, $S(=O)_2$-$NR_{6a}R_{6b}$ or C(=O)-$R_7$; |
| $R_4$ | represents hydrogen; |
| $R_5$ | represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl or aryl; |
| $R_{6a}$ and $R_{6b}$ | each independently represent hydrogen, $C_{1-6}$alkyl, amino, mono-or di($C_{1-4}$alkyl)amino, arylNH-, amino$C_{1-6}$alkyl, mono-or di($C_{1-4}$alkyl)amino-$C_{1-6}$alkyl, $C_{1-6}$alkylcarbonylamino, aminocarbonylamino, $C_{1-6}$alkyloxy, carbonylamino or hydroxy$C_{1-6}$alkyl; or |

$R_{6a}$ and $R_{6b}$ taken together with the nitrogen to which they are attached form pyrrolidinyl, imidazolidinyl, pyrazolidinyl, piperidinyl, piperazinyl, morpholinyl, thiomorpholinyl or piperazinyl substituted with $C_{1-6}$alkyl;

$R_7$ represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl, aryl or heteroaryl;

Z represents a cyclic ring system selected from

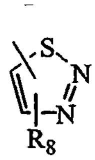
(a-1)

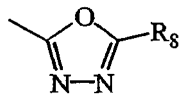
(a-2)

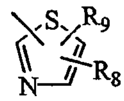
(a-3)

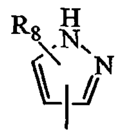
(a-4)

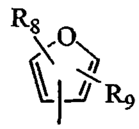
(a-5)

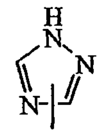
(a-6)

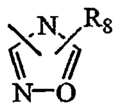
(a-7)

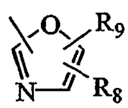
(a-8)

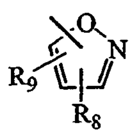
(a-9)

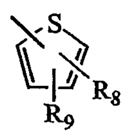
(a-10)

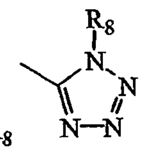
(a-11)

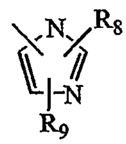
(a-12)

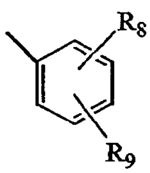
(a-13)

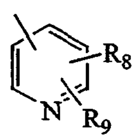
(a-14)

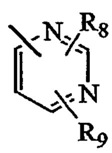
(a-15)

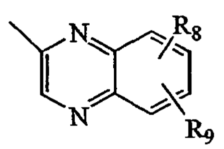
(a-16)

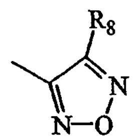
(a-17)

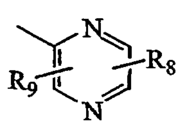
(a-18)

each $R_8$ independently represents hydrogen, halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di ($C_{1-4}$alkyl)amino, hydroxy$C_{1-6}$alkylamino, aryl, aryloxy, piperidinyl, piperidinylamino, morpholinyl, piperazinyl or nitro;

each $R_9$ independently represents hydrogen, halo or $C_{1-6}$alkyl;

n is 1, 2, 3, 4 or 5;

aryl represents phenyl or phenyl substituted with one, two, three, four or five substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino, phenyloxy or nitro;

heteroaryl represents furanyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, each of said heterocycles optionally being substituted with one or two substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino or nitro.

A second interesting embodiment are those compounds of formula (I) or any subgroup thereof as mentioned hereinbefore as interesting embodiment wherein $R_3$ represents hydrogen, cyano, C(=O)-O-$R_5$, C(=O)-$NR_{6a}R_{6b}$, C (=S)-$NR_{6a}R_{6b}$, $S(=O)_2$-$NR_{6a}R_{6b}$ or C(=O)-$R_7$.

A third interesting embodiment are the compounds of formula (I) or any subgroup thereof as mentioned hereinbefore as interesting embodiment wherein $R_3$ represents cyano, $C_{1-6}$alkyl optionally substituted with hydroxy or $C_{1-6}$alkyloxy, C(=O)-O-$R_5$, C(=O)-$NR_{6a}R_{6b}$, C(=S)-$NR_{6a}R_{6b}$, $S(=O)_2$-$NR_{6a}R_{6b}$ or C(=O)-$R_7$.

A fourth interesting embodiment are the compounds of formula (I) or any subgroup thereof as mentioned hereinbefore as interesting embodiment wherein $R_3$ represents cyano, C(=O)-O-$R_5$, C(=O)-$NR_{6a}R_{6b}$, C(=S)-$NR_{6a}R_{6b}$, $S(=O)_2$-$NR_{6a}R_{6b}$ or C(=O)-$R_7$.

A fifth interesting embodiment are the compounds of formula (I) or any subgroup thereof as mentioned hereinbefore as interesting embodiment wherein $R_3$ represents hydrogen, cyano, C(=O)-O-$R_5$, C(=O)-$NR_{6a}R_{6b}$ or C(=O)-$R_7$; preferably hydrogen, $C_{1-6}$alkyl substituted with $C_{1-6}$alkyloxy, or C(=O)-O-$R_5$; more preferably hydrogen or C(=O)-O-$R_5$; even more preferably C(=O)-O-$R_5$, in particular C(=O)-O-$C_{1-6}$alkyl; most preferred C(=O)-O-$CH_3$.

A sixth interesting embodiment are those compounds of formula (I) or any subgroup thereof as mentioned hereinbefore as interesting embodiment wherein Z is other than 3-pyridyl.

A seventh interesting embodiment are those compounds of formula (I) or any subgroup thereof as mentioned hereinbefore as interesting embodiment wherein n is 2 or 3, in particular n is 2.

An eighth interesting embodiment are those compounds of formula (I) or any subgroup thereof as mentioned hereinbefore as interesting embodiment wherein n is 2 and said two substituents are placed in meta and para postion.

A ninth interesting embodiment are those compounds of formula (I) or any subgroup thereof as mentioned hereinbefore as interesting embodiment wherein Z is a cyclic ring system selected from (a-1), (a-2), (a-3), (a-4), (a-5), (a-6), (a-7), (a-9), (a-10), (a-11), (a-12), (a-13), (a-14), (a-15), (a-16) or (a-18); preferably a cyclic ring system selected from (a-2), (a-3), (a-4), (a-5), (a-6), (a-7), (a-11), (a-12), (a-13), (a-14) or (a-15); more preferably a cyclic ring system selected from (a-2), (a-3), (a-4), (a-5), (a-6), (a-7), (a-11), (a-13), (a-14) or (a-15); even more preferably a cyclic ring system selected from (a-2), (a-11) or (a-15); most preferred a cyclic ring system selected from (a-2) or (a-15). Also interesting are those compounds of formula (I) or any subgroup thereof as mentioned hereinbefore as interesting embodiment wherein Z is a cyclic ring system of (a-9).

A tenth interesting embodiment are those compounds of formula (I) or any subgroup thereof as mentioned hereinbefore as interesting embodiment wherein Z represents a cyclic ring system selected from (a-2) or (a-15) and $R_3$ represents hydrogen.

An eleventh interesting embodiment are those compounds of formula (I) or any subgroup thereof as mentioned hereinbefore as interesting embodiment wherein $R_2$ represents chloro, fluoro or trifluoromethyl, most preferred halo, in particular chloro or fluoro.

A twelfth interesting embodiment are the compounds of formula (I) or any subgroup thereof as mentioned hereinbefore as interesting embodiment wherein $R_1$ is methyl, ethyl, n-propyl or methoxymethyl; more preferably $R_1$ is $C_{1-6}$alkyl, in particular methyl, ethyl and propyl, more in particular methyl, ethyl or n-propyl; most preferred $R_1$ is ethyl.

A thirteenth interesting embodiment are the compounds of formula (I) or any subgroup thereof as mentioned hereinbefore as interesting embodiment wherein $R_4$ is hydrogen.

A fourteenth interesting embodiment are those compounds of formula (I) or any subgroup thereof as mentioned hereinbefore as interesting embodiment which are stereochemically pure.

A fifteenth interesting embodiment are those compounds of formula (I) or any subgroup thereof as mentioned hereinbefore as interesting embodiment wherein the carbon atom carrying the $R_1$ and $R_4$ substituent has the (S) configuration, i.e. a compound of formula (I')

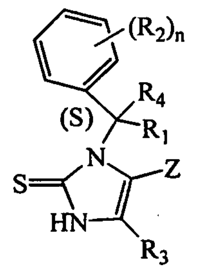

(I')

,

a *N*-oxide, a pharmaceutically acceptable addition salt, a quaternary amine or a stereochemically isomeric form thereof.

Also interesting compounds are those compounds of formula (I) wherein one or more, preferably all of the following restrictions apply:

a) $R_1$ represents $C_{1-6}$alkyl, especially methyl, ethyl or propyl;
b) $R_2$ represents halo or polyhalo$C_{1-6}$alkyl, especially halo, e.g. chloro or fluoro;
c) $R_3$ represents hydrogen, cyano, C(=O)-O-$R_5$, C(=O)-N$R_{6a}R_{6b}$ or C(=O)-$R_7$;
d) Z represents a ring system selected from (a-1), (a-2), (a-3), (a-4), (a-5), (a-6), (a-7), (a-9), (a-10), (a-11), (a-12), (a-13), (a-14), (a-15), (a-16) or (a-18);
e) $R_4$ represents hydrogen;
f) n is 2 or 3.

Also interesting compounds consists of those compounds of formula (I) wherein one or more of the following restrictions apply

a) $R_1$ represents $C_{1-6}$alkyl, especially ethyl or n-propyl, more especially ethyl;
b) $R_2$ represents halo or polyhalo$C_{1-6}$alkyl, especially halo, e.g. chloro and fluoro;
c) $R_3$ represents hydrogen, $C_{1-6}$alkyl substituted with $C_{1-6}$alkyloxy, or C(=O)-O-$R_5$;
d) Z represents a ring system selected from (a-2), (a-3), (a-4), (a-5), (a-6), (a-7), (a-11), (a-13), (a-14), (a-15);
e) $R_4$ represents hydrogen;
f) n is 1, 2 or 3, in particular 2 or 3, more in particular 2.

Further interesting compounds consists of those compounds of formula (I) wherein one or more of the following restrictions apply:

a) $R_1$ represents $C_{1-6}$alkyl, especially ethyl;
b) $R_2$ represents halo or polyhalo$C_{1-6}$alkyl;
c) $R_3$ represents hydrogen;
d) Z represents a ring system selected from (a-2), (a-7) or (a-15) or (a-2), (a-11) or (a-15);
e) $R_4$ represents hydrogen;
f) n is 2 or 3, in particular 2.

Further interesting compounds consists of those compounds of formula (I) wherein one or more of the following restrictions apply:

a) $R_1$ represents $C_{1-6}$alkyl, especially ethyl;
b) $R_2$ represents halo, especially chloro or fluoro;
c) $R_3$ represents hydrogen;
d) Z represents a ring system selected from (a-2) or (a-15);
e) $R_4$ represents hydrogen;
f) n is 2 or 3, in particular 2.

Preferred compounds of formula (I) are compounds 5, 26, 1, 20, 21 and 9.

Most preferred compounds of formula (I) are compounds 1, 20, 21 and 9.

In general, compounds of formula (I) can be prepared by reacting an intermediate of formula (II) with an appropriate acid, such as hydrochloric acid or acetic acid, optionally in the presence of a suitable solvent, such as for example 1,4-dioxane.

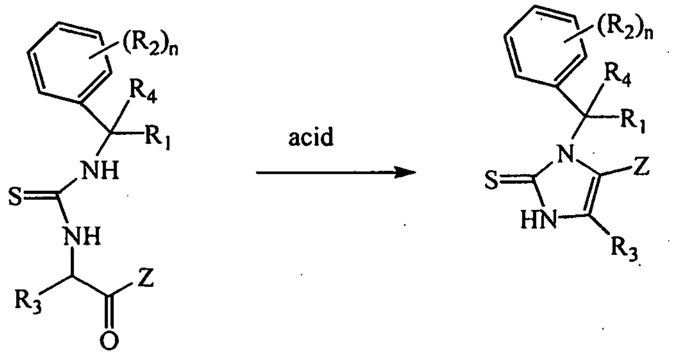


(II)

(I)

Intermediates of formula (I) wherein $R_3$ is hydrogen, said compounds being represented by formula (I-a), can be prepared by reacting an intermediate of formula (III) with an intermediate of formula (IV) in the presence of a suitable solvent, such as for example 1,4-dioxane and water.

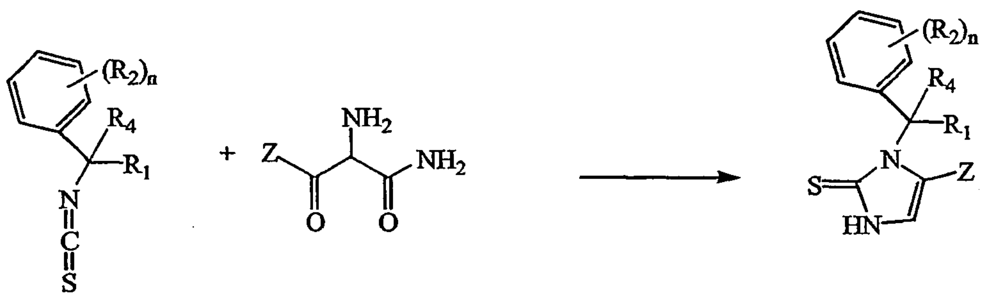

(III) (IV) (I-a)

Compounds of formula (I) can also be prepared by reacting an intermediate of formula (V) with a suitable acid, such as for example trifluoroacetic acid.

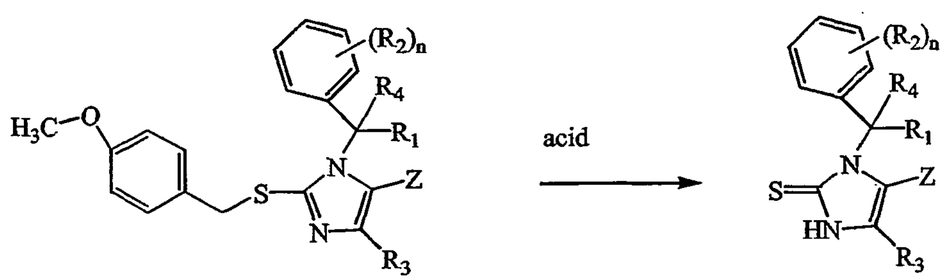


(V) (I)

Compounds of formula (I) wherein Z represents optionally substituted 1,3,4-oxadiazole, said compounds being represented by formula (I-b), can be prepared by reacting an intermediate of formula (VI) with phosphoric trichloride ($POCl_3$) or Burgess'reagent in the presence of a suitable solvent, such as for example acetonitrile or tetrahydrofuran.

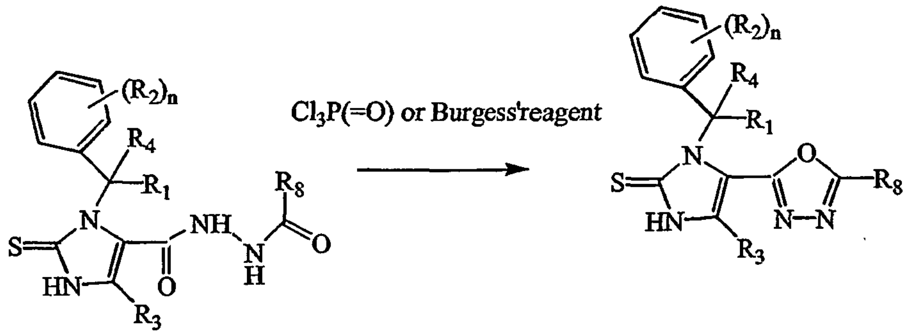


(VI)

(I-b)

Compounds of formula (I) wherein Z represents 1,3,4-oxadiazole, said compounds being represented by formula (I-b-1), can be prepared by reacting an intermediate of formula (VII) with $SOCl_2$ and HC(=O)NH-$NH_2$ in the presence of a suitable solvent, such as for example tetrahydrofuran.

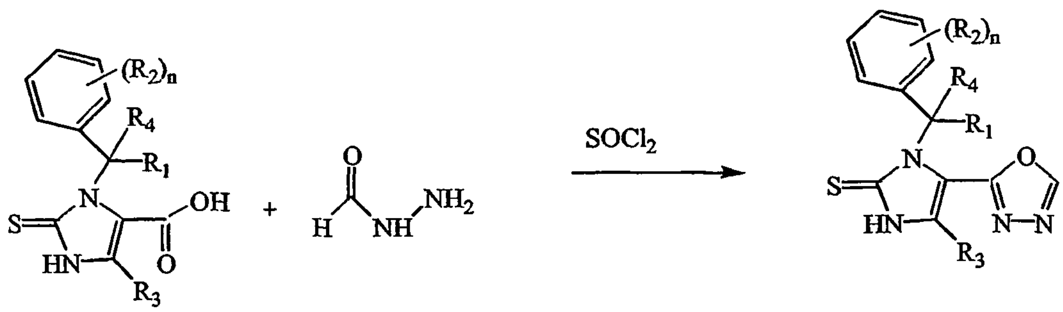


(VII)

(I-b-1)

Compounds of formula (I) wherein Z represents optionally substituted 1,2,4-oxadiazole, said compounds being represented by formula (I-c), can be prepared by reacting an intermediate of formula (VIII) with an intermediate of formula (IX) in the presence of a suitable base, such as for example $NaOCH_3$, and a suitable solvent, such as an alcohol, e.g. methanol.

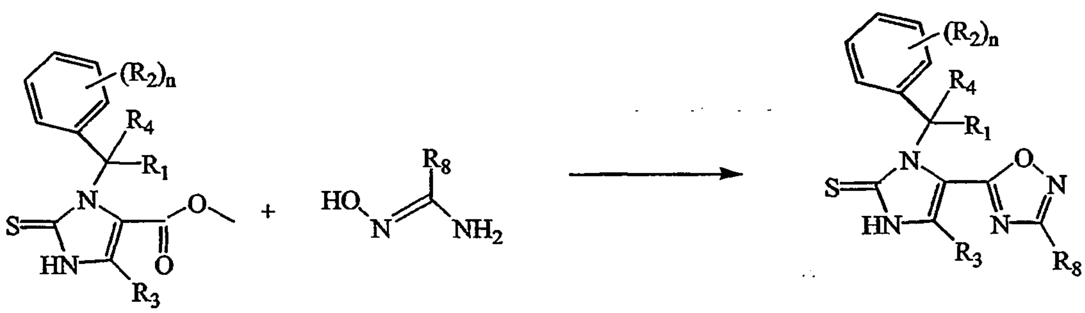

(VIII)

(IX)

(I-c)

Compounds of formula (I) wherein Z represents tetrazolyl and wherein $R_3$ is hydrogen, said compounds being represented by formula (I-a-1), can be prepared by reacting an intermediate of formula (X) with methyl formate in the presence of a suitable base, such as for example t-BuOK, $NaOCH_3$ or $NaOC(CH_3)_3$, KSCN in the presence of a suitable acid, such as for example hydrochloric acid and the like, and in the presence of a suitable solvent, such as for example tetrahydrofuran, an alcohol, such as for example methanol, and water, followed by reacting the thus obtained intermediate of formula (X-a) with $Bu_3SnN_3$ in the presence of a suitable solvent, such as for example toluene.

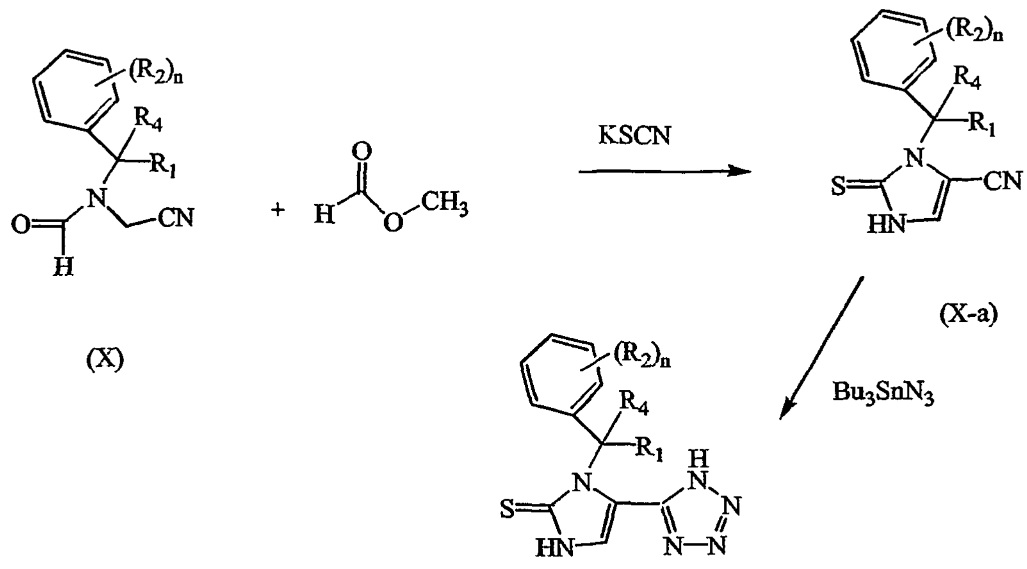


(X)

(X-a)

(I-a-1)

Compounds of formula (I') can be prepared according to the above described reactions but starting from an intermediate wherein the carbon atom carrying the $R_1$ and $R_4$ substituent has the (S) configuration.
Alternatively, compounds of formula (I) wherein the carbon atom carrying the $R_1$ and $R_4$ substituent has the (R) configuration can be prepared according to the above described reactions but starting from an intermediate wherein the carbon atom carrying the $R_1$ and $R_4$ substituent has the (R) configuration.

The compounds of formula (I) may further be prepared by converting compounds of formula (I) into each other according to art-known group transformation reactions.

The compounds of formula (I) may be converted to the corresponding *N*-oxide forms following art-known procedures for converting a trivalent nitrogen into its *N*-oxide form. Said *N*-oxidation reaction may generally be carried out by reacting the starting material of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. tert.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

Compounds of formula (I) wherein $R_3$ represents C(=O)-O-$C_{1-6}$alkyl, may be converted into a compound of formula (I) wherein $R_3$ represents $CH_2$-OH by reaction with a suitable reducing agent, such as for example $LiHBEt_3$ in the presence of a suitable solvent, such as for example tetrahydrofuran.

Compounds of formula (I) wherein $R_3$ represents C(=O)-O-$C_{1-6}$alkyl, can also be converted into a compound of formula (I) wherein $R_3$ represents C(=O)-OH by reaction with a suitable base, such as NaOH, in the presence of a suitable solvent, such as for example $H_2O$, tetrahydrofuran or an appropriate alcohol, e.g. methanol and the like.

Compounds of formula (I) wherein $R_3$ represents C(=O)-O-$C_{1-6}$alkyl, can also be converted into a compound of formula (I) wherein $R_3$ represents C(=O)-$NR_{6a}R_{6b}$, by reaction with the appropriate base of formula $NHR_{6a}R_{6b}$ in a suitable solvent, such as for example $H_2O$.

Compounds of formula (I) wherein $R_3$ represents C(=O)-O-H, can be converted into a compound of formula (I) wherein $R_3$ represents C(=)-$NR_{6a}R_{6b}$, by reaction with the appropriate base of formula $NHR_{6a}R_{6b}$ in the presence of *N'*-(ethylcarbonimidoyl)-*N,N*-dimethyl-1,3-propanediamine, 1-hydroxy-1H-benzotriazole and a suitable solvent, such as for example *N,N*-dimethylformamide.

Compounds of formula (I) wherein $R_3$ represents C(=O)-O-H, can also be converted into a compound of formula (I) wherein $R_3$ represents C(=O)-$NH_2$ by reaction with $NH_4OH$ in the presence of $SOCl_2$.

Compounds of formula (I) wherein $R_3$ represents C(=O)-O-$C_{1-6}$alkyl, can also be converted into a compound of formula (I) wherein $R_3$ represents C(=O)-O-$C_{1-6}$alkyl-O-$C_{1-6}$alkyl, by reaction with HO-$C_{1-6}$alkyl-O-$C_{1-6}$alkyl in the

presence of $NaBH_4$.

Compounds of formula (I) wherein $R_3$ represents cyano or C(=O)-O-$C_{1-6}$alkyl, can be converted into a compound of formula (I) wherein $R_3$ represents aminocarbonyl by reaction with $NH_4OH$.

Compounds of formula (I) wherein $R_3$ represents cyano, can also be converted into a compound of formula (I) wherein $R_3$ represents C(=S)$NR_{6a}R_{6b}$ by reaction with hydrogen sulfide in the presence of *N*-ethyl-*N*-(1-methylethyl)-2-propanamine in a suitable solvent such as pyridine.

Compounds of formula (I) wherein $R_3$ represents C(=O)-$NR_{6a}R_{6b}$ can be converted into a compound of formula (I) wherein $R_3$ represents C(=O)-$C_{1-6}$alkyl by reaction with chloro$C_{1-6}$alkyMg in a suitable solvent such as tetrahydrofuran.

Compounds of formula (I) wherein $R_3$ represents C(=O)-$C_{1-6}$alkyl can be converted into compounds of formula (I) wherein $R_3$ represents hydroxy$C_{1-6}$alkyl by reaction with a suitable reducing agent such as $NaBH_4$, in the presence of a suitable solvent such as methanol.

Some of the compounds of formula (I) and some of the intermediates in the present invention may contain an asymmetric carbon atom. Pure stereochemically isomeric forms of said compounds and said intermediates can be obtained by the application of art-known procedures. For example, diastereoisomers can be separated by physical methods such as selective crystallization or chromatographic techniques, e.g. counter current distribution, liquid chromatography and the like methods. Enantiomers can be obtained from racemic mixtures by first converting said racemic mixtures with suitable resolving agents such as, for example, chiral acids, to mixtures of diastereomeric salts or compounds; then physically separating said mixtures of diastereomeric salts or compounds by, for example, selective crystallization or chromatographic techniques, e.g. liquid chromatography and the like methods; and finally converting said separated diastereomeric salts or compounds into the corresponding enantiomers. Pure stereochemically isomeric forms may also be obtained from the pure stereochemically isomeric forms of the appropriate intermediates and starting materials, provided that the intervening reactions occur stereospecifically.

An alternative manner of separating the enantiomeric forms of the compounds of formula (I) and intermediates involves liquid chromatography, in particular liquid chromatography using a chiral stationary phase.

Some of the intermediates and starting materials are known compounds and may be commercially available or may be prepared according to art-known procedures.

Intermediates of formula (II) can be prepared by reacting an intermediate of formula (III) with an intermediate of formula (XI) in the presence of a suitable base, such as for example dipotassium carbonate, and a suitable solvent, such as for example dioxane or tetrahydrofuran and water.

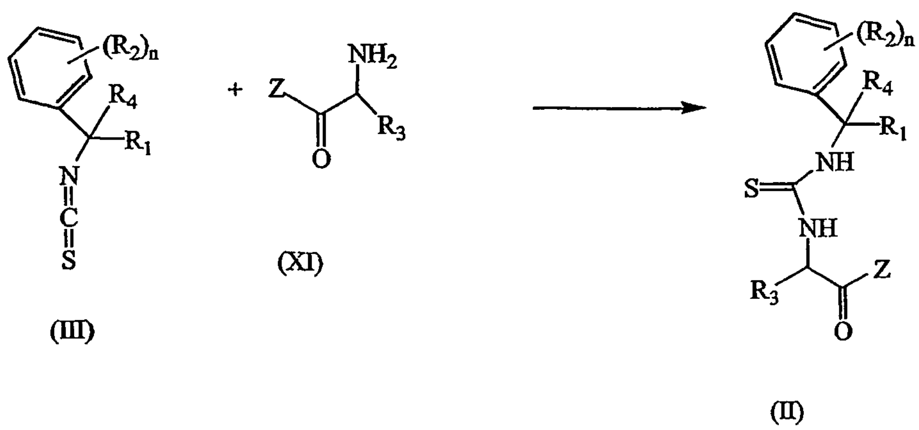

Intermediates of formula (III) can be prepared by reacting an intermediate of formula (XII) with $C(=S)Cl_2$ in the presence of a suitable base, such as for example *N,N*-diisopropylethanamine, and a suitable solvent, such as for example methylene chloride.

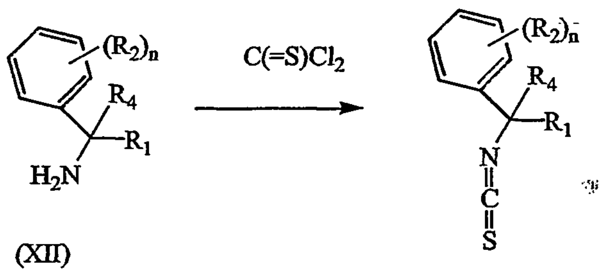


(XII)

(III)

Intermediates of formula (XII) wherein $R_4$ represents hydrogen, said intermediates being represented by formula (XII-a), may be prepared by reacting an intermediate of formula (XIII) with a suitable reducing agent, such as for example $H_2$, in the presence of a suitable catalyst, such as for example Raney Nickel, optionally a suitable catalyst poison, such as for example a thiophene solution, a suitable base, such as for example $NH_3$, and a suitable solvent, such as for example an alcohol, e.g. methanol.

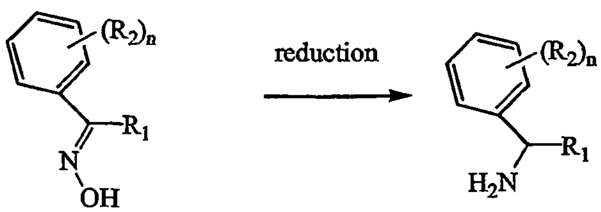


(XIII) (XII-a)

Intermediates of formula (XIII) may be prepared by reacting an intermediate of formula (XIV) with HO-$NH_2$ in the presence of a suitable base, such as for example $Na_2CO_3$ or sodium acetate and a suitable solvent, such as for example an alcohol, e.g. ethanol, and water.

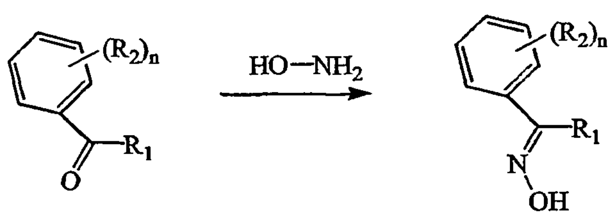


(XIV) (XIII)

Intermediates of formula (XII) can be prepared as described hereinabove. The intermediates of formula (XII) may contain a chiral center at the carbon atom carrying the $R_1$ and $R_4$ substituent depending on the substituents representing $R_1$ and $R_4$. In case said carbon atom represents a chiral center, stereospecific intermediates of formula (XII) represented by formula (XII-b), can be prepared by reacting an intermediate of formula (XV) with triphenylphosphine, in the presence of a suitable solvent, such as for example tetrahydrofuran and water or by reacting an intermediate of formula (XV) with a suitable reducing agent, such as for example $H_2$, in the presence of a suitable catalyst, such as for example Pt on charcoal or Pd on charcoal, and a suitable solvent, such as for example an alcohol, e.g. methanol.

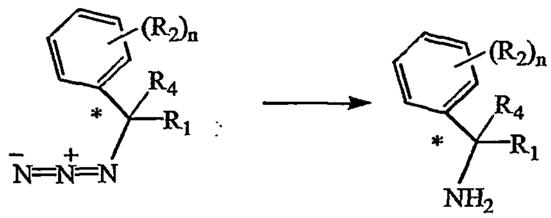

(XV) (XII-b)

* indicates the chiral center and may be (R) or (S) depending on the $R_1$ and $R_4$ substituents

When a stereospecific intermediate of formula (XII-b) is reacted further according to the methods described hereinabove, the resulting intermediates are also stereospecific and finally the resulting final compounds are also stereospecific.

Intermediates of formula (XV) can be prepared by reacting an intermediate of formula (XVI) with diphenylphosphoryl azide in the presence of 2,3,4,6,7,8,9,10-octahydropyrimido[1,2-a]azepine and in the presence of a suitable solvent, such as for example toluene.

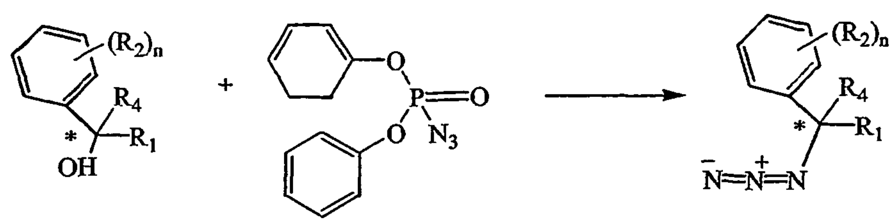

(XVI) (XV)

* indicates the chiral center and may be (R) or (S) depending on the $R_1$ and $R_4$ substituents

Stereospecific intermediates of formula (XVI) wherein $R_4$ is hydrogen and $R_1$ is methyl, ethyl, or *n*-propyl, said $R_1$ being represented by Alk and said intermediates being represented by formula (XVI-a) and (XVI-b), can be prepared by reacting an intermediate of formula (XVII) with $ZnAlk_2$ wherein Alk represents methyl, ethyl or *n*-propyl, in the presence of a stereospecific catalyst, such as for example *N,N'*-(1R,2R)-1,2-cyclohexanediylbis[1,1,1-trifluoro]-methanesulfonamide respectively *N,N'*-(1S,2S)-1,2-cyclohexanediylbis[1,1,1-trifluoro]-methanesulfonamide, $Ti(iPrO)_4$ and a suitable solvent, such as for example toluene.

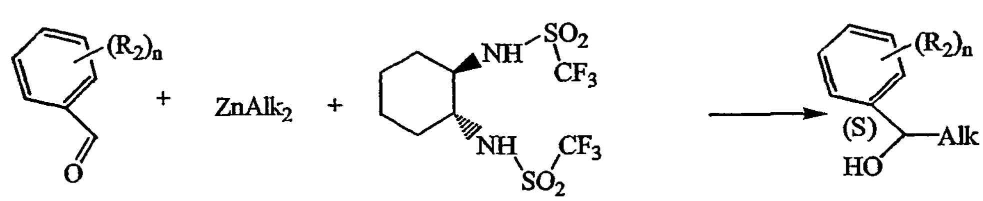


(XVII) 1R,2R (XVI-a)

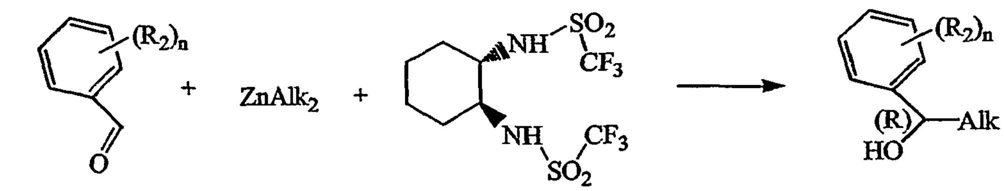

(XVII) 1S,2S (XVI-b)

Intermediates of formula (V) wherein Z represents optionally substituted thiazolyl, said intermediates being represented by formula (V-a), can be prepared by reacting an intermediate of formula (XVIII) with an intermediate of formula (XIX) wherein $W_1$ represents a suitable leaving group, such as for example halo, e.g. chloro, bromo and the like, in the presence of a suitable solvent, such as for example an alcohol, e.g. ethanol.

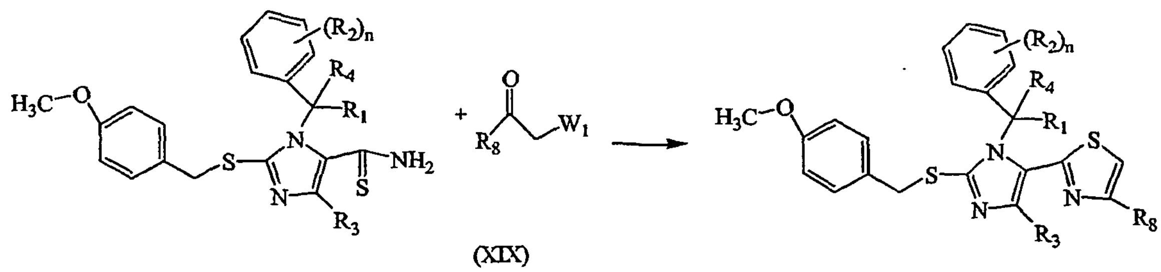

(XVIII) (XIX) (V-a)

Intermediates of formula (XVIII) can be prepared by reacting an intermediate of formula (XX) with $H_2S$ in the presence of a suitable base, such as for example *N,N*-diisopropylethanamine, and a suitable solvent, such as for example pyridine.

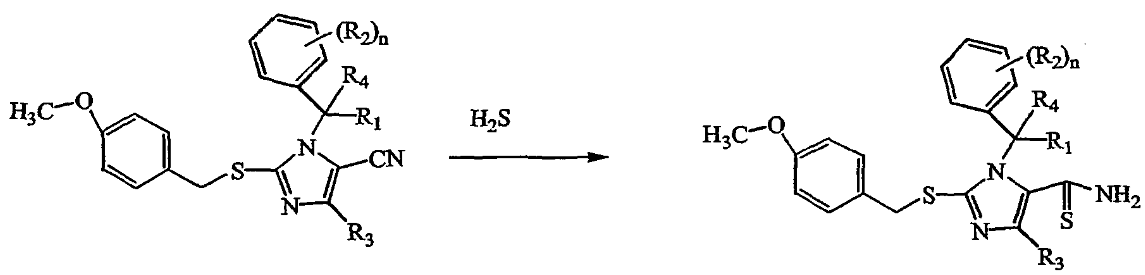


(XX) (XVIII)

Intermediates of formula (XX) can be prepared by reacting an intermediate of formula (XXI) with 4-methoxy-benzenemethanol in the presence of a suitable acid, such as for example trifluoroacetic acid, and a suitable solvent, such as for example methylene chloride.

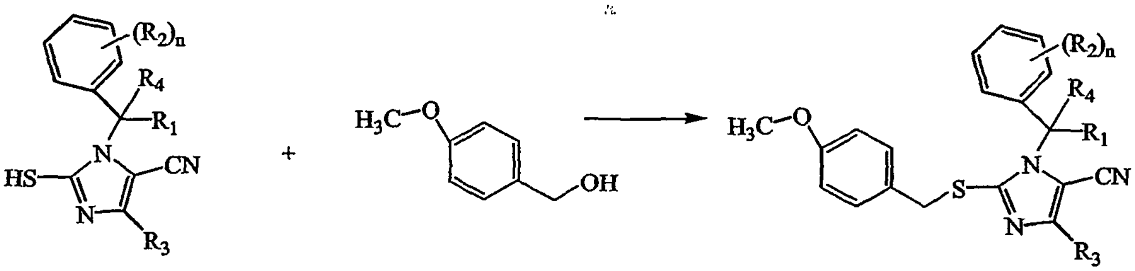

(XXI) (XX)

Intermediates of formula (XXI) can be prepared by reacting an intermediate of formula (X) with $CH_3$-CH(=O) in the presence of KSCN, in the presence of a suitable base, such as for example sodium methanolate, and a suitable solvent, such as for example tetrahydrofuran.

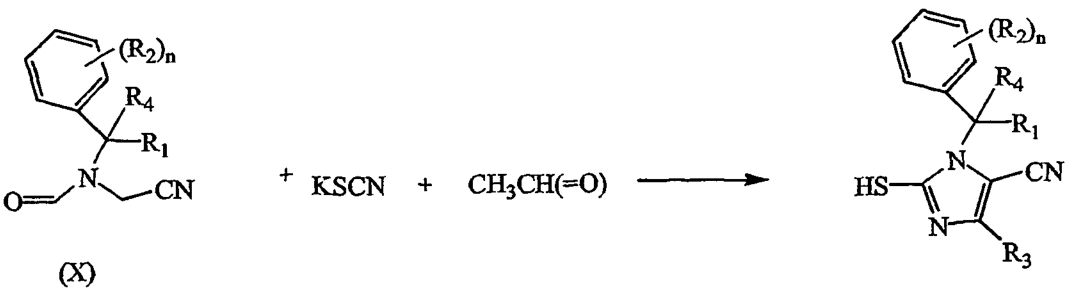


(X) (XXI)

Intermediates of formula (X) can be prepared by reacting an intermediate of formula (XXII) with *n*-butyl formate.

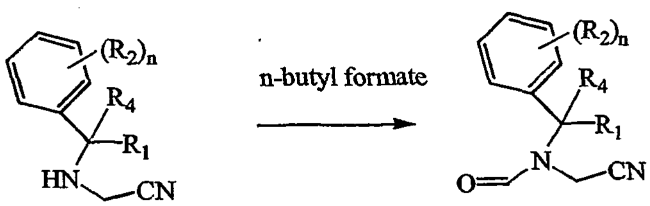


(XXII) (X)

Intermediates of formula (XXII) can be prepared by reacting an intermediate of formula (XII) with an intermediate of formula (XXIII) wherein $W_2$ represents a suitable leaving group, such as for example halo, e.g. chloro and the like, in the presence of a suitable base, such as for example *N,N*-diethylethanamine, and a suitable solvent, such as for example *N,N*-dimethylformamide or tetrahydrofuran.

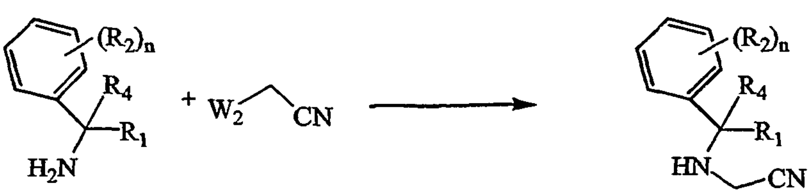

(XII) (XXIII) (XXII)

Intermediates of formula (V) wherein Z represents pyrazolyl or optionally substituted pyrimidine, said intermediates being represented by formula (V-b) or (V-c), can be prepared by reacting an intermediate of formula (XXIV) with $NH_2$-$NH_2$ or $R_8$-C(=NH)$NH_2$ in the presence of Na and a suitable solvent, such as for example an alcohol, e.g. ethanol.

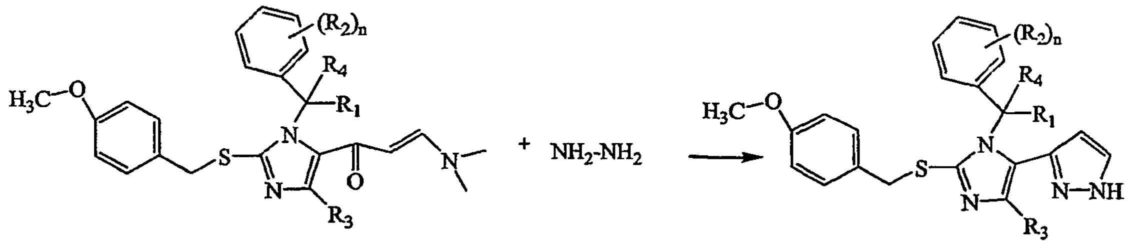


(XXIV) (V-b)

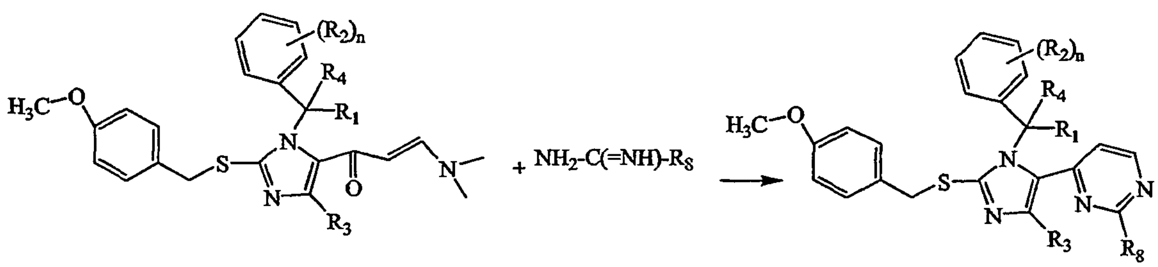


(XXIV) (V-c)

Intermediates of formula (XXIV) can be prepared by reacting an intermediate of formula (XXV) with $N(CH_3)_2$-$CH(OCH_3)_2$.

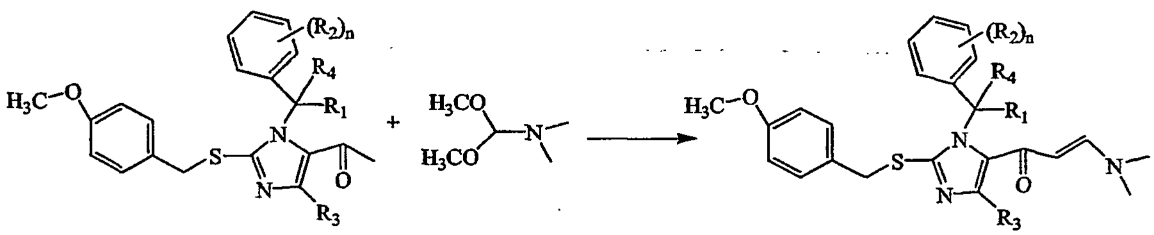

(XXV) (XXIV)

Intermediates of formula (XXV) can be prepared by reacting an intermediate of formula (XXVI) with an intermediate of formula (XXVII) wherein $W_3$ represents a suitable leaving group, such as for example halo, e.g. chloro, in the presence of NaH and a suitable solvent, such as for example tetrahydrofuran.

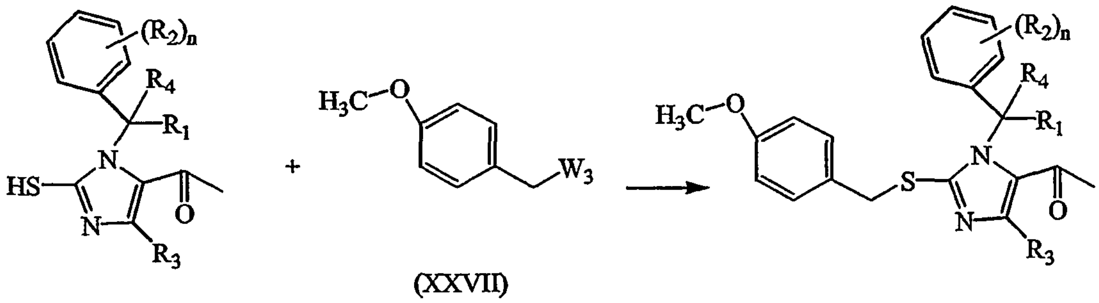

(XXVI) (XXVII) (XXV)

Intermediates of formula (XXVI) can be prepared by reacting an intermediate of formula (XXVIII) with $CH_3MgCl$ in the presence of a suitable solvent, such as for example tetrahydrofuran.

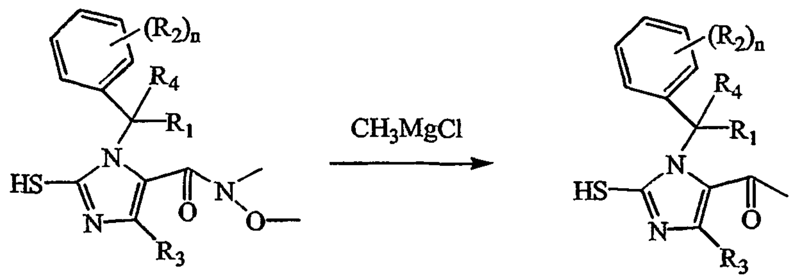


(XXVIII) (XXVI)

Intermediates of formula (XXVIII) can be prepared by reacting an intermediate of formula (XXIX) wherein $W_4$ represents a suitable leaving group, such as for example halo, e.g. chloro and the like, with $NH(CH_3)(OCH_3)$ in the presence of a suitable base, such as for example *N,N*-diethylethanamine, and a suitable solvent, such as for example tetrahydrofuran.

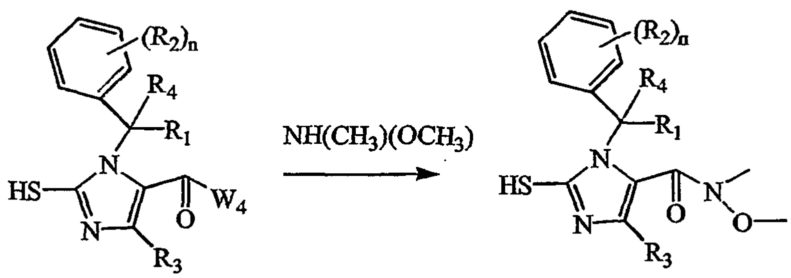


(XXIX) (XXVIII)

Intermediates of formula (XXIX) wherein $W_4$ represents chloro, said intermediates being represented by formula (XXIX-a), can be prepared by reacting an intermediate of formula (VII) with $SOCl_2$.

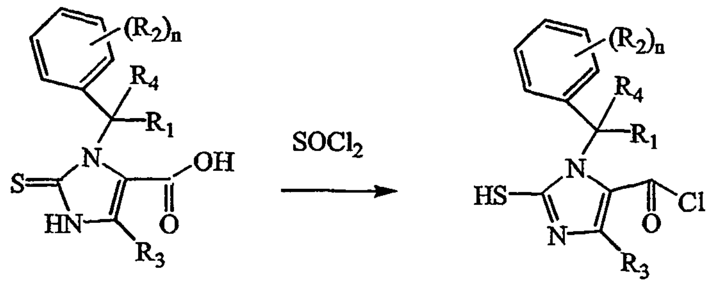


(VII) (XXIX-a)

Intermediates of formula (VII) can be prepared by reacting an intermediate of formula (VIII) with a suitable base, such as for example sodium hydroxide, in the presence of a suitable solvent, such as for example an alcohol, e.g. methanol, or tetrahydrofuran.

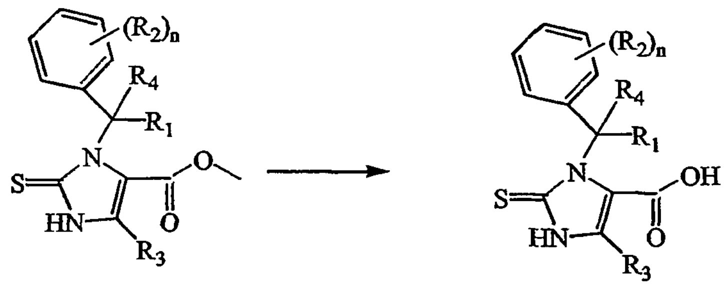

(VIII) (VII)

Intermediates of formula (VIII) wherein $R_3$ is hydrogen, said intermediates being represented by formula (VIII-a), can be prepared by reacting an intermediate of formula (XXX) with formic acid methyl ester or acetic acid methyl ester in the presence of KSCN, a suitable base, such as for example $NaOCH_3$ or *t*-BuONa, and a suitable solvent, such as for example tetrahydrofuran or an alcohol, e.g. methanol.

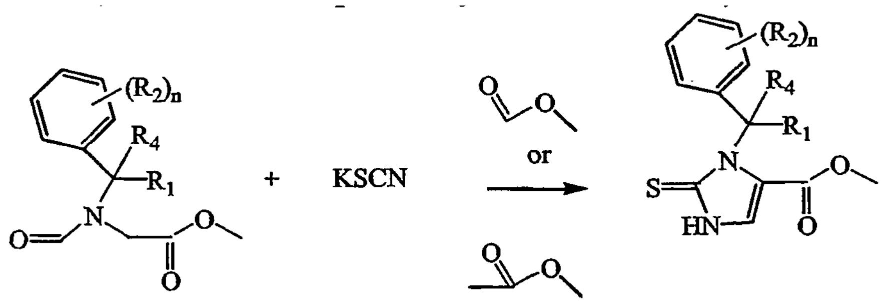


(VIII-a)

(XXX)

Intermediates of formula (XXX) can be prepared from an intermediate of formula (XXXI) in the presence of formic acid or formate, such as for example *n*-butylformate, and in the presence of a suitable solvent, such as for example xylene or diethyl ether.

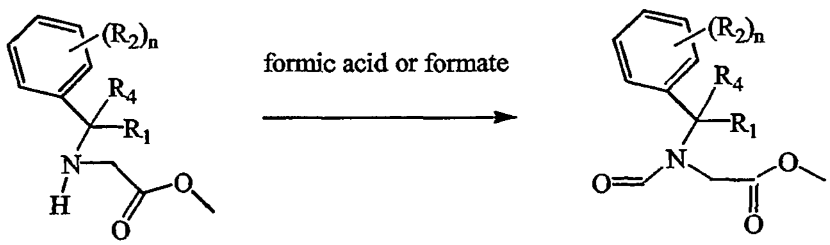


(XXXI)

(XXX)

Intermediates of formula (XXXI) can be prepared by reacting an intermediate of formula (XII) with an intermediate of formula (XXXII) wherein $W_5$ represents a suitable leaving group, such as for example halo, e.g. chloro or bromo, in the presence of a suitable solvent, such as for example tetrahydrofuran or *N,N*-dimethylformamide, and optionally in the presence of a suitable base, such as for example *N,N*-diethylethanamine and *N,N*-dimethyl-4-pyridinamine.

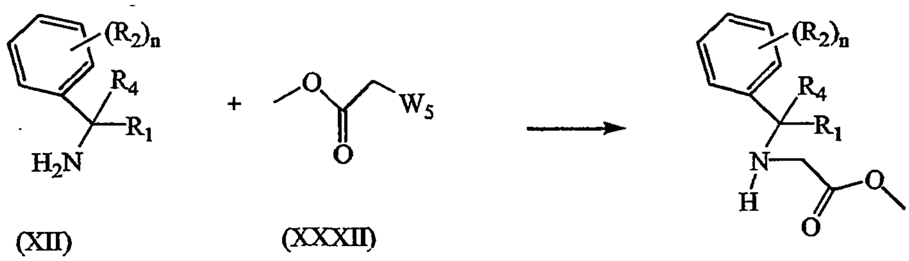

(XII) (XXXII) (XXXI)

Intermediates of formula (V) wherein Z represents triazolyl, said intermediates being represented by formula (V-d), can be prepared by reacting an intermediate of formula (XXXIII) with $NH_2$-$NH_2$ in the presence of a suitable acid, such as for example acetic acid.

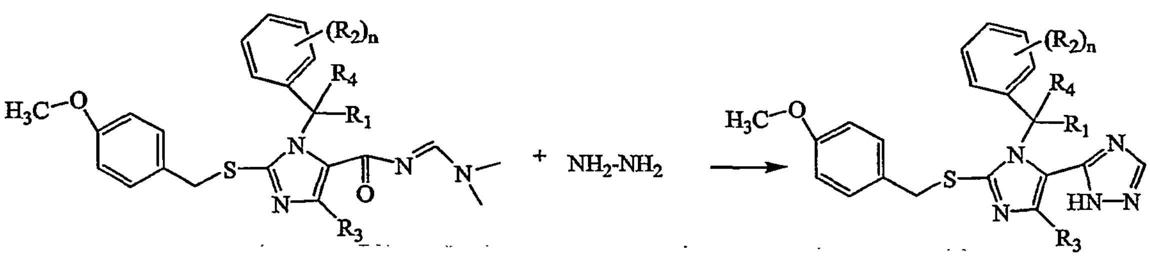

(XXXIII) (V-d)

Intermediates of formula (XXXIII) can be prepared by reacting an intermediate of formula (XXXIV) with *N*, *N*-dimethylformamide/*N*,*N*-dimethylacetamide.

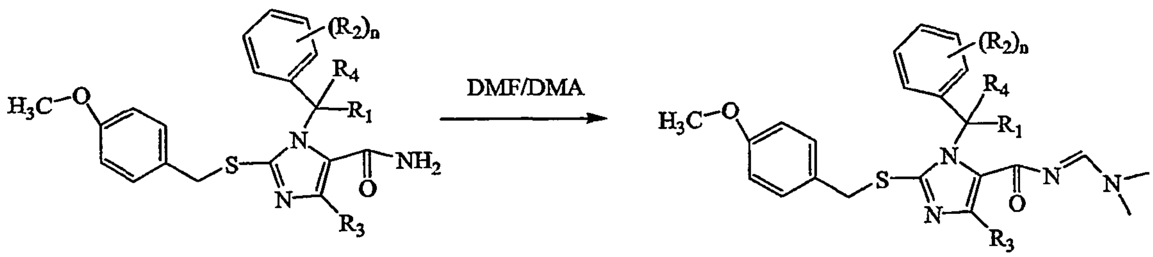


(XXXIV) (XXXIII)

Intermediates of formula (XXXIV) can be prepared by reacting an intermediate of formula (XXXV) wherein $W_6$ represents a suitable leaving group, such as for example halo, e.g. chloro, with $NH_3$ in the presence of a suitable solvent, such as for example tetrahydrofuran.

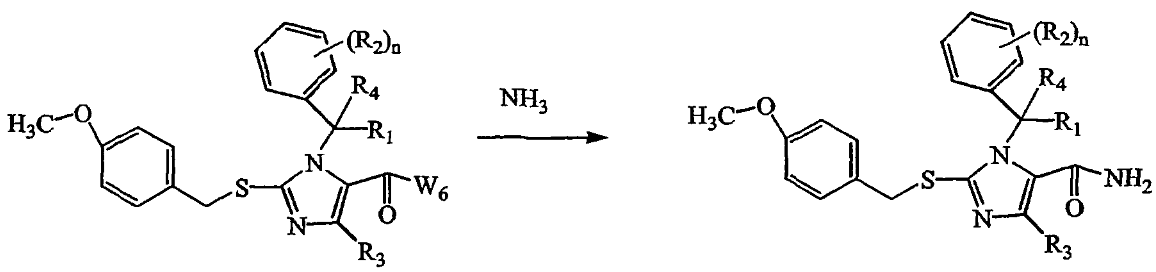


(XXXV) (XXXIV)

Intermediates of formula (XXXV) wherein $W_6$ represents chloro, said intermediates being represented by formula (XXXV-a), can be prepared by reacting an intermediate of formula (XXXVI) with $SOCl_2$.

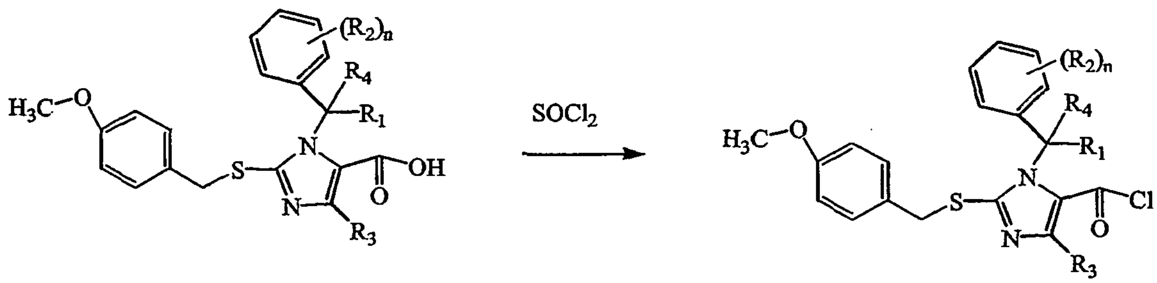


(XXXVI) (XXXV-a)

Intermediates of formula (XXXVI) can be prepared by hydrolysis of the corresponding ester (XXXVII) with a suitable base, such as for example sodium hydroxide, in the presence of a suitable solvent, such as for example an alcohol, e,g. methanol.

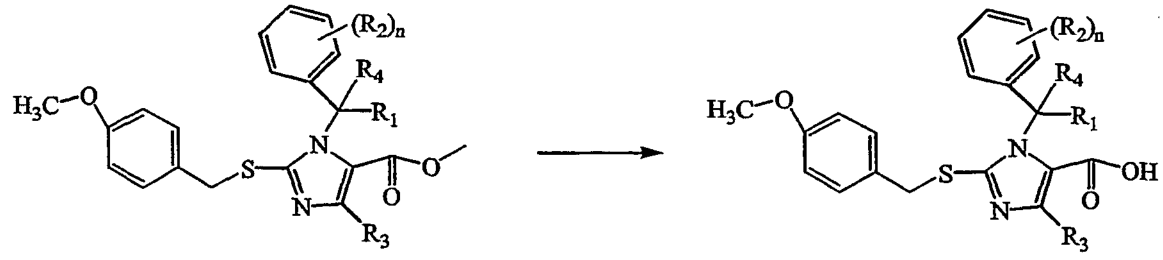

(XXXVII) (XXXVI)

Intermediates of formula (XXXVII) can be prepared by reacting an intermediate of formula (XXXVIII) with 4-methoxy-benzenemethanol in the presence of a suitable acid, such as for example trifluoroacetic acid, and a suitable solvent, such as for example methylene chloride.

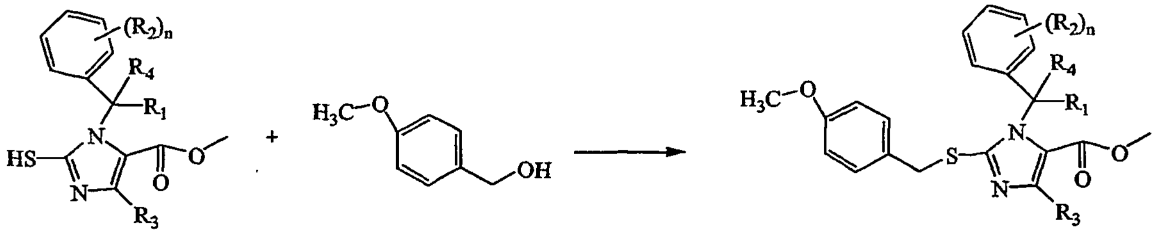

(XXXVIII) (XXXVII)

Intermediates of formula (VI) can be prepared by reacting an intermediate of formula (XXIX) or (VII) with an intermediate of formula (XXXIX) in the presence of a suitable base, such as for example *N,N*-diisopropylethanamine, a suitable diimide, such as for example *N*'-(ethylcarbonimidoyl)-*N,N*-dimethyl-1,3-propanediamine or dicyclohexylcarbondiimide, 1-hydroxy-1*H*-benzotriazole and a suitable solvent, such as for example tetrahydrofuran or *N,N*-dimethylformamide.

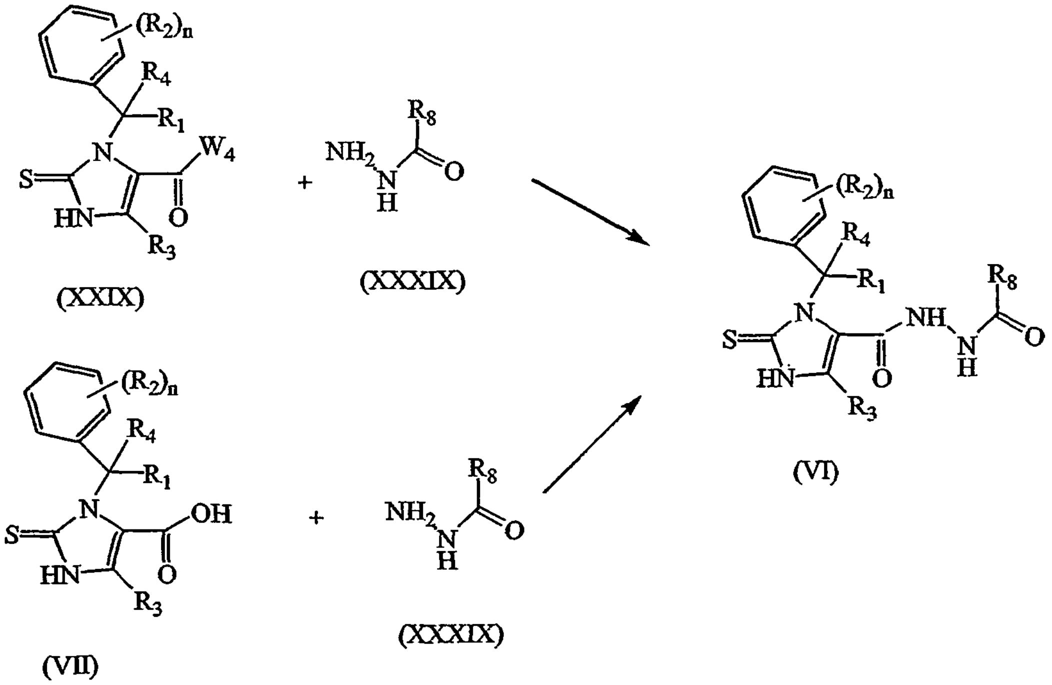

(XXIX) (XXXIX) (VI) (VII) (XXXIX)

In the preparation of the compounds of the present invention, interesting intermediates are intermediates of formula (XII)

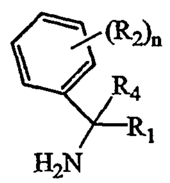

(XII) ,

a *N*-oxide, a pharmaceutically acceptable addition salt, a quaternary amine or stereochemically isomeric form thereof.

As already indicated hereinabove, the intermediates of formula (XII) may contain a chiral center at the carbon atom carrying the $R_1$ and $R_4$ substituent depending on the substituents representing $R_1$ and $R_4$. In case said carbon atom represents a chiral center, a preferred embodiment of the intermediates of formula (XII) are those intermediates wherein the intermediate is stereospecific, i.e. wherein the intermediate has the (R) or (S) configuration at the carbon atom carrying the $R_1$ and $R_4$ substituent (intermediates of formula (XII-b). Particularly preferred are those intermediates of formula (XII-b) which have the (S) configuration (intermediates of formula (XII-b-1).

Thus the present invention also relates to intermediates of formula (XII-b-1)

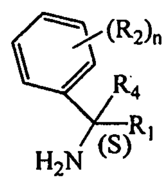

(XII-b-1) ,

a *N*-oxide, a pharmaceutically acceptable addition salt or a quaternary amine thereof.

The present invention also relates to intermediates of formula (XII-b-1) provided that when n=2 and each $R_2$ is chloro and said two chloro substituents are placed in meta and para position, then $R_1$ is other than ethyl.

Another preferred embodiment are those intermediates of formula (XII-b-1) wherein each $R_2$ is independently selected from halo or polyhalo$C_{1-6}$alkyl provided that when n=2 and each $R_2$ is chloro and said two chloro substituents are placed in meta and para position, then $R_1$ is other than ethyl.

A further embodiment are those intermediates of formula (XII-b-1) wherein n is 1, 2, or 3, in particular 2 and provided that when n is 2 and each $R_2$ is chloro and said two chloro substituents are placed in meta and para position, then $R_1$ is other than ethyl.

Again another embodiment are those intermediates of formula (XII-b-1) wherein n is 2 and the two $R_2$ substituents are placed in meta and para position provided that when each $R_2$ is chloro, then $R_1$ is other than ethyl.

Another embodiment are those intermediates of formula (XII-b-1) as described hereinabove wherein $R_4$ is hydrogen.

Also interesting are those intermediates of formula (XII) and (XII-b-1) wherein $R_1$ is methyl, ethyl, *n*-propyl, provided that when n is 2 and $R_2$ represents chloro, and said two chloro substituents are placed in meta and para position and $R_4$ is hydrogen, then $R_1$ is other than ethyl and provided that when n is 2 and said two $R_2$ substituents are placed in meta and para position and $R_2$ in meta position is trifluoromethyl and $R_2$ in para position is fluoro and $R_4$ is hydrogen, then $R_1$ is other than ethyl.

Also interesting are those intermediates of formula (XII) or (XII-b-1) as described hereinabove wherein $R_2$ is chloro, fluoro, or trifluoromethyl, in particular chloro.

Particularly interesting intermediates are those intermediates of formula (XII-a) or (XII-b-1) having the following formula

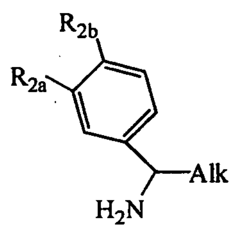
(XII-a-1)

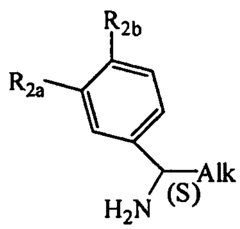
(XII-b-1-1)

a *N*-oxide, a pharmaceutically acceptable addition salt or a quaternary amine thereof, wherein Alk is defined as hereinabove, i.e. Alk represents methyl, ethyl and n-propyl, and each $R_{2a}$ and $R_{2b}$ independently represents chloro, fluoro, trifluoromethyl.

An interesting embodiment are those intermediates of formula (XII-a-1) provided that when $R_{2a}$ and $R_{2b}$ are both chloro or when $R_{2a}$ is trifluoromethyl and $R_{2b}$ is fluoro, then Alk is other than ethyl.

Further interesting intermediates of formula (XII-a-1) are those intermediates of formula (XII-a-1) provided that when $R_{2a}$ and $R_{2b}$ are both chloro, then Alk is other than methyl, ethyl, *n*-propyl and provided that when $R_{2a}$ and $R_{2b}$ are both fluoro or $R_{2a}$ is trifluoromethyl and $R_{2b}$ is fluoro or $R_{2a}$ is fluoro and $R_{2b}$ is trifluoromethyl then Alk is other than ethyl.

Also interesting are those intermediates of formula (XII-b-1-1) provided that when $R_{2a}$ and $R_{2b}$ are both chloro, then Alk is other than ethyl.

The compounds of formula (I) and (I') show CCR2 receptor antagonistic properties.

The C - C chemokine receptor 2 (CCR2) and its ligand monocyte chemoattractant (chemotactic) protein (MCP-1; in new chemokine nomenclature also called CCL2) are recognized to be implicated in both acute and chronic inflammatory processes.

Chemokines (contraction of "chemotactic cytokines") are most important regulators of leukocyte trafficking. This biological role is exerted by interacting - on target cells - with seven-transmembrane-domain receptors that are coupled to heterodimeric G proteins. Chemokines are mainly grouped into 2 major families (C - C or C - X - C family) dependent on the presence of an amino acid (represented by X) between the two conserved cysteine residues (represented by C) near the amino terminus. In general, chemokines from the C - C family attract monocytes, macrophages, T cells and NK cells.

A chemokine, which acts through the CCR2 receptor, is MCP-1 as indicated above. Therefore, the CCR2 receptor is also known as the MCP-1 receptor.

MCP-2, MCP-3 and MCP-4 may also act, at least in part, through this receptor.

It is recognized that the CCR2 receptor and MCP-1 play a role in the pathophysiology of various inflammatory

diseases. Therefore, CCR2 receptor antagonists, which block the CCR2 receptor, have potential as pharmaceutical agents to combat inflammatory conditions such as arthritis, osteoarthritis, rheumatoid arthritis, glomerulonephritis, diabetic nephropathy, lung fibrosis, idiopathic pulmonary fibrosis, sarcoidosis, vasculitis, hepatitis, nonalcoholic steatohepatitis, inflammatory conditions of the brain such as Alzheimer's disease, restenosis, alveolitis, asthma, allergic rhinitis, allergic conjunctivitis, atherosclerosis, psoriasis, delayed-type hypersensitivity reactions of the skin, inflammatory bowel disease, acute or chronic brain inflammation, e.g. multiple sclerosis, autoimmune encephalomyelitis, chronic obstructive pulmonary disease (COPD), uveitis, dermatitis, atopic dermatitis. CCR2 receptor antagonists may also be useful to treat autoimmune diseases such as diabetes or transplant rejection, stroke, reperfusion injury, ischemia, cancer, myocardial infraction, pain, in particular neuropathic pain.

The compounds of the present invention may also be used to inhibit the entry of Human Immunodeficiency Virus (HIV) into monocytes and lymphocytes, thereby having a therapeutic role in the treatment of AIDS (Acquired Immunodeficiency Syndrome).

The CCR2 receptor exists in two isoforms, namely the CCR2A and the CCR2B receptor.

Due to their CCR2 receptor antagonistic activity, in particular their CCR2B receptor antagonistic activity, the compounds of formula (I), their *N*-oxides, pharmaceutically acceptable addition salts, quaternary amines or stereochemically isomeric forms are useful in the treatment or prevention, in particular for the treatment, of diseases or conditions mediated through the activation of the CCR2 receptor, in particular the CCR2B receptor. Diseases or conditions related to an activation of the CCR2 receptor comprise inflammatory conditions such as arthritis, osteoarthritis, rheumatoid arthritis, glomerulonephritis, diabetic nephropathy, lung fibrosis, idiopathic pulmonary fibrosis, sarcoidosis, vasculitis, hepatitis, nonalcoholic steatohepatitis, inflammatory conditions of the brain such as Alzheimer's disease, restenosis, alveolitis, asthma, allergic rhinitis, allergic conjunctivitis, atherosclerosis, psoriasis, delayed-type hypersensitivity reactions of the skin, inflammatory bowel disease, acute or chronic brain inflammation, e.g. multiple sclerosis, autoimmune encephalomyelitis, chronic obstructive pulmonary disease (COPD), uveitis, dermatitis, atopic dermatitis, autoimmune diseases such as diabetes or transplant rejection, stroke, reperfusion injury, ischemia, cancer, myocardial infraction, pain (neuropathic pain). In particular, the compounds of formula (I) are useful in the treatment or prevention of inflammatory diseases and autoimmune diseases, especially rheumatoid arthritis, atherosclerosis, multiple sclerosis, inflammatory bowel disease and chronic obstructive pulmonary disease (COPD). The compounds of formula (I) are also of particular interest in the treatment or prevention of psoriasis, asthma, rheumatoid arthritis or pain (neuropathic pain), more in particular psoriasis, asthma or rheumatoid arthritis.

In view of the above-described pharmacological properties, the compounds of formula (I), their *N*-oxides, pharmaceutically acceptable addition salts, quaternary amines and stereochemically isomeric forms, may be used as a medicine. In particular, the present compounds can be used for the manufacture of a medicament for treating or preventing diseases mediated through activation of the CCR2 receptor, in particular the CCR2B receptor. More in particular, the compounds of the invention can be used for the manufacture of a medicament for treating or preventing inflammatory diseases, especially rheumatoid arthritis, atherosclerosis, multiple sclerosis, inflammatory bowel disease and chronic obstructive pulmonary disease (COPD). The compounds of the invention can also in particular be used for the manufacture of a medicament for treating or preventing psoriasis, asthma, rheumatoid arthritis or pain (neuropathic pain), more in particular psoriasis, asthma or rheumatoid arthritis.

The blockade of the CCR2 receptor by the present compounds of formula (I) inhibits the normal function of MCP-1. Therefore, the present compounds can also be described as MCP-1 inhibitors and hence can be used to prevent or treat diseases mediated through MCP-1.

The present invention also provides compositions for preventing or treating diseases mediated through activation of the CCR2 receptor, in particular the CCR2B receptor. Said compositions comprise a therapeutically effective amount of a compound of formula (I) and a pharmaceutically acceptable carrier or diluent.

The compounds of the present invention may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier

comprises saline solution, glucose solution or a mixture of saline and glucose solution. Injectable suspensions may also be prepared in which case appropriate liquid carriers, suspending agents and the like may be employed. Also included are solid form preparations, which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment.
The compounds of the present invention may also be administered via inhalation or insufflation by means of methods and formulations employed in the art for administration via this way. Thus, in general the compounds of the present invention may be administered to the lungs in the form of a solution, a suspension or a dry powder. Any system developed for the delivery of solutions, suspensions or dry powders via oral or nasal inhalation or insufflation are suitable for the administration of the present compounds.
The compounds of the present invention may also be topically administered in the form of drops, in particular eye drops. Said eye drops may be in the form of a solution or a suspension. Any system developed for the delivery of solutions or suspensions as eye drops are suitable for the administration of the present compounds.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

The exact dosage and frequency of administration depends on the particular compound of formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

The compounds of formula (I) may also be used in combination with other conventional anti-inflammatory or immunosuppressive agents, such as steroids, cyclooxygenase-2 inhibitors, non-steroidal-anti-inflammatory drugs, TNF-a antibodies, such as for example acetyl salicylic acid, bufexamac, diclofenac potassium, sulindac, diclofenac sodium, ketorolac trometamol, tohnetine, ibuprofen, naproxen, naproxen sodium, tiaprofen acid, flurbiprofen, mefenamic acid, nifluminic acid, meclofenamate, indomethacin, proglumetacine, ketoprofen, nabumetone, paracetamol, piroxicam, tenoxicam, nimesulide, fenylbutazon, tramadol, beclomethasone dipropionate, betamethasone, beclamethasone, budesonide, fluticasone, mometasone, dexamethasone, hydrocortisone, methylprednisolone, prednisolone, prednisone, triamcinolone, celecoxib, rofecoxib, valdecoxib, infliximab, leflunomide, etanercept, CPH 82, methotrexate, sulfasalazine, antilymphocytory immunoglobulines, antithymocytory immunoglobulines, azathioprine, cyclosporine, tacrolimus substances, ascomycin, rapamycin, muromonab-CD3.
Thus, the present invention also relates to the combination of a compound of formula (I) and another anti-inflammatory or immunosuppressive agent. Said combination may be used as a medicine. The present invention also relates to a product containing (a) a compound of formula (I), and (b) another anti-inflammatory or immunosuppressive compound, as a combined preparation for simultaneous, separate or sequential use in the treatment of diseases mediated through activation of the CCR2 receptor, in particular mediated through the CCR2B receptor. The different drugs in such products may be combined in a single preparation together with pharmaceutically acceptable carriers. Alternatively, such products may comprise, for example, a kit comprising a container with a suitable composition containing a compound of formula (I) and another container with a composition containing another anti-inflammatory or immunosuppressive compound. Such a product may have the advantage that a physician can select on the basis of the diagnosis of the patient to be treated the appropriate amounts of each component and the sequence and timing of the administration thereof.

The following examples are intended to illustrate the present invention.

## Experimental Part

Hereinafter, "THF" means tetrahydrofuran, "DIPE" means diisopropylether, "DMF" means *N,N*-dimethylformamide and "DMA" means *N,N*-dimethylacetamide.

A. Preparation of the intermediate compounds

Example A1

a. Preparation of intermediate 1

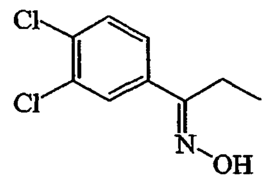

A solution of $Na_2CO_3$ (part of 0.52 mol) in $H_2O$ (150 ml) was added to a stirring mixture of 1-(3,4-dichlorophenyl)-1-propanone (0.345 mol) in ethanol, p.a. (150 ml), then the remainder of $Na_2CO_3$ was added and hydroxylamine monohydrochloride (0.345 mol) was added portionwise while stirring vigorously. The reaction mixture was heated to reflux temperature and extra $H_2O$ (75 ml) was added, then the resulting mixture was stirred and refluxed for 6 hours. Extra hydroxylamine monohydrochloride (2.4 g) was added and the mixture was refluxed further for 18 hours. Again extra hydroxylamine monohydrochloride (3 g) was added; the reaction mixture was refluxed for 24 hours and stirred for 2 days at room temperature. The solids were filtered off, washed with EtOH/$H_2O$ (1/1) and dried (vacuum, stream of air) at 56˚C. Yield: 71.8 g of intermediate 1 (95.4 %).

b. Preparation of intermediate 2 and 3

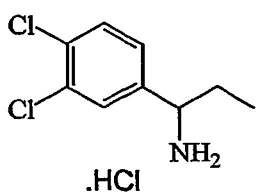

.HCl

Intermediate 2

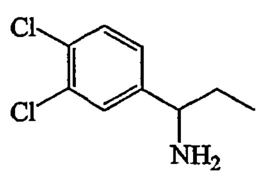

Intermediate 3

A mixture of intermediate 1 (0.3 mol) in $CH_3OH/NH_3$ (7 N) (500 ml) was hydrogenated at 14˚C with Raney Nickel (catalytic quantity) as a catalyst in the presence of thiophene (6 ml). After uptake of $H_2$ (2 equiv.), the catalyst was filtered off and the filtrate was evaporated, then co-evaporated 2 times with toluene. The residue was stirred in boiling 2-propanol (250 ml) and the mixture was filtered off hot. The filtrate was allowed to reach room temperature and HCl/2-propanol (6N, 150 ml) was added slowly while stirring vigorously. The solvent was evaporated and the residue was stirred in DIPE, then filtered off, washed and dried (vacuum) at 60˚C. Yield: 53 g intermediate 2 (73.4 %). A part of this fraction was converted into its free base: Intermediate 2 (18.0 g) was stirred in $CH_2Cl_2$ (200 ml) and a 15 % aqueous $K_2CO_3$ solution was added, then the resulting mixture was stirred for 1 hour and a 50 % NaOH solution was added to increase the pH. The organic layer was separated, washed with $H_2O$, dried ($MgSO_4$), filtered off and the solvent was evaporated. Yield: 12.4 g of intermediate 3.

c. Preparation of intermediate 4

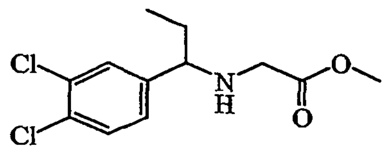

A solution of intermediate 2 (prepared according to A1.b) (0.0748 mol) and chloro acetic acid methyl ester (0.08 mol) in DMF, p.a., dried on molecular sieves, (150 ml) was stirred at room temperature under $N_2$ and $Et_3N$ (0.224 mol) was slowly added, then the reaction mixture was stirred for 20 hours at room temperature and extra chloro acetic acid methyl ester (3.3 ml) was added. The mixture was stirred for another 20 hours at room temperature and again extra chloro acetic acid methyl ester (2 ml) was added. The resulting mixture was stirred for 24 hours and then the solids were filtered off and washed with DMF. $Et_2O$ (800 ml) was added and the mixture was washed 3 times with $H_2O$ (500 ml). The organic layer was separated, dried ($MgSO_4$), filtered off and the solvent was evaporated, then co-evaporated with toluene. The residual oil (23.4 g) was filtered over silica gel (eluent: $CH_2Cl_2/CH_3OH$ 99/1). The product fractions were collected and the solvent was evaporated, finally co-evaporated with toluene. Yield: 20.6 g of intermediate 4 (99.7 %).

d. Preparation of intermediate 5

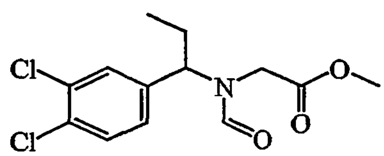

A solution of formic acid (7.5 ml) and intermediate 4 (prepared according to A1.c) (0.0746 mol) in xylene, p.a. (225 ml) was stirred and refluxed for 4 hours and then the reaction mixture was allowed to reach room temperature. The mixture was washed 2 times with $H_2O$ (2 x 200 ml), with a saturated aqueous $NaHCO_3$ solution (200 ml) and with brine (200 ml), then the separated organic layer was dried ($MgSO_4$) and filtered off. Finally, the solvent was evaporated Yield: 21.3 g of intermediate 5 (93.9 %).

e-1. Preparation of intermediate 6

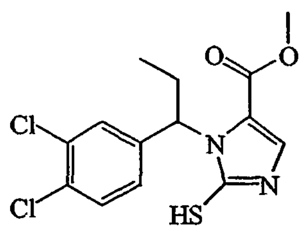

$NaOCH_3$ (0.07 mol) was added to a stirring solution of intermediate 5 (prepared according to A1.d) (0.066 mol) and methyl ester formic acid (0.19 mol) in THF, p.a. (100 ml) under $N_2$ and the reaction mixture was stirred at room temperature for 40 hours. The solvent was evaporated and the crude residue was stirred in water (90 ml). The aqueous layer was washed 2 times with $Et_2O$ (50 ml) and $CH_3OH$ (60 ml) was added. HCl, 36%, p.a. (15.7 ml) was added and the solution was heated on an oil-bath and stirred at 45°C for 24 hours, then KSCN (0.11 mol) was added. The mixture was stirred at room temperature for 40 hours and at 80°C for 5 hours. The reaction mixture was allowed to reach room temperature and the solids were filtered off, washed with $H_2O/CH_3OH$ (2/1), then dried at 60°C (vacuum). Yield: 19.35 g of a fraction which was re-crystallized from $CH_3CN$ (200 ml), filtered off, washed with $CH_3CN$ and dried at 50°C (vacuum). Yield: 17.2 g of intermediate 6 (75.5 %).

e-2. Preparation of intermediate 44

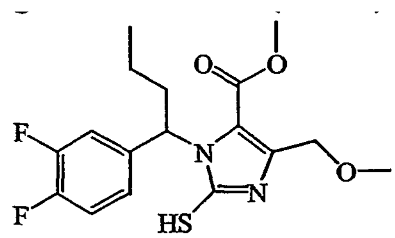

Methyl 2-[[1-(3,4-difluorophenyl)butyl](formyl)amino]acetate (prepared according to A1.d) (15.0 g, 53 mmol) dissolved in 250 ml of absolute ether was cooled at stirring to -78˚C in an argon atmosphere, and 55 ml of 2M solution of lithium diisopropylamide in THF/hexane was added dropwise at -78˚C. The mixture was stirred for an additional 0.5 hour at the same temperature. Methoxyacetic acid chloroanhydride (6.9 g, 63.6 mmol) dissolved in 40 ml of dry THF was added dropwise to the mixture, and the reaction temperature was allowed to adjust to room temperature for 2 hours. A mixture of methanol (270 ml), water (135 ml), KCNS (13.43 g) and 67 ml of conc. HCl was added to the mixture. The resulting mixture was heated to 70˚C and stirred for 18 hours at this temperature. Then, it was allowed to cool to room temperature., and neutralized with an aqueous solution of $NaHCO_3$ and extracted with methylene chloride. The extract was dried over $MgSO_4$ and concentrated *irc-vacuo*. The residue obtained was subjected to flash-chromatography on silica gel 60/100 ($CH_2Cl_2$). The fraction containing the desired product (according to LC/MS) was concentrated, and treated with a mixture of hexane/ether (1/1). Yield: 2.46 g (12.5%) of intermediate 44.

f-1. Preparation of intermediate 7

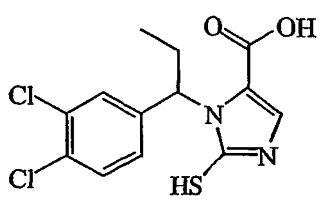

A solution of intermediate 6 (prepared according to A1.e) (0.004 mol) in NaOH (1N) (7.5 ml), methanol, p.a. (10 ml) and THF, p.a. (20 ml) was stirred at room temperature for 20 hours, then stirred for 5 days at 75˚C. Extra NaOH (1N) (7.5 ml) was added and the reaction mixture was stirred for 1 hour at 75˚C, then the mixture was allowed to reach room temperature. $H_2O$ (45 ml), then $Et_2O$ (50 ml) was added and the reaction mixture was stirred for 30 minutes. The aqueous layer was separated, washed with EtOAc/Hexane (2 x 50 ml, 1/1) and acidified with HCl (1N) to pH 3. The mixture was extracted with $CH_2Cl_2/CH_3OH$ (95/5), then the organic layer was separated, dried ($MgSO_4$), filtered and the solvent was evaporated. The residue was dissolved in $Et_2O$/Hexanc/$CH_2Cl_2$ (1/1/1) and concentrated at 60˚C (without vacuum) until crystallisation started, then the mixture stood for 30 minutes. The precipitate was filtered off, washed with Hexane/$Et_2O$ (3/1) and dried (vacuum, 50˚C), followed by drying with vacuo pump for 5 hours). Yield: 0.55 g of intermediate 7 (41.5 %).

f-2. Preparation of intermediate 42

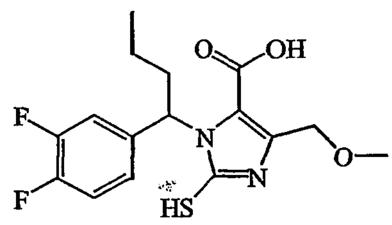

A mixture of intermediate 42 (0.0045 mol) and NaOH 1N (0.025 mol) in methanol (25 ml) was stirred for 24 hours at 60˚C, then extra NaOH 1N (10 ml) was added and the reaction mixture was stirred for 20 hours at 60˚C. A part of the solvent was evaporated and 1N HCl (36 ml) was added to the concentrate. The product was precipitated as an oil and was extracted with $CH_2Cl_2$. The organic layer was separated, dried and filtered off. The solvent was evaporated and the residue was dried Yield: 1.4 g of intermediate 42.

g. Preparation of intermediate 8

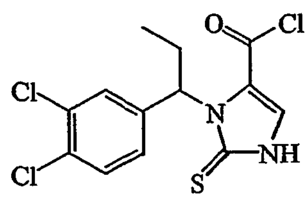

A mixture of intermediate 7 (prepared according to A1.f) (0.001 mol) in $SOCl_2$ (10 ml) was stirred and refluxed for 2 hours and then the solvent was evaporated. Toluene (p.a.) was added 2 times to the residue and the solvent was evaporated after each addition. Yield: 0.35 g of intermediate 8.

h. Preparation of intermediate 9

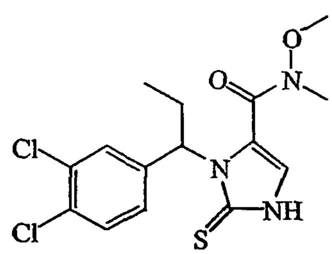

A mixture of *N*-methoxymethanamine .hydrochloride (0.0017 mol) and $Et_3N$ (0.005 mol) in THF (10 ml) was stirred at room temperature and a mixture of intermediate 8 (prepared according to A1.g) (0.0015 mol) in THF (5 ml) was added dropwise over 15 minutes, then the reaction mixture was stirred overnight at room temperature and the solvent was evaporated. The residue was stirred in $H_2O$ and the mixture was extracted with $CH_2Cl_2$. The organic layer was separated, dried ($MgSO_4$), filtered off and the solvent was evaporated. The residue was dissolved in 2-propanone (15 ml, p.a.) and a stream of $SO_2$ was passed through the solution for 20 minutes The solvent was evaporated and the residue was filtered over silica gel (eluent: $CH_2Cl_2$/$CH_3OH$ 95/5). The product fractions were collected and the solvent was evaporated Yield: 0.310 g of intermediate 9.

i. Preparation of intermediate 10

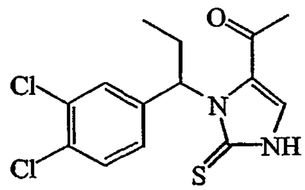

A mixture of intermediate 9 (prepared according to A1.h) (0.0008 mol) in THF (10 ml) was stirred at 0-5˚C on an ice bath and $CH_3MgCl$, 20% in THF (0.002 mol) was added dropwise, then the reaction mixture was stirred for 1 hour and extra $CH_3MgCl$, 20% in THF (0.0044 mol) was added dropwise. The mixture was stirred for 1 hour and again extra $CH_3MgCl$, 20% in THF (0.002 mol) was added dropwise, then the reaction mixture was stirred for 1 hour and 1N HCl (10 ml) was added dropwise under cooling with ice. $H_2O$ was added and the mixture was extracted with $CH_2Cl_2$. The organic layer was separated, dried ($MgSO_4$), filtered off and the solvent was evaporated. The residue was dissolved in 2-propanone (p.a.) and a stream of $SO_2$ was passed through the solution for 20 minutes. The solvent was evaporated and the residue was purified by Flash column chromatography (eluent: EtOAc). The product fractions were collected and dissolved in $CH_2Cl_2/CH_3OH$ (90/10), then the mixture was filtered and the filtrate's solvent was evaporated Yield: 0.060 g of intermediate 10.

j. Preparation of intermediate 11

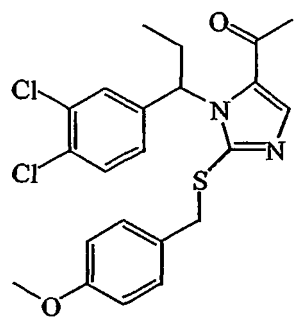

A mixture of intermediate 10 (prepared according to Al.i) (0.005 mol) in dry THF (40 ml) was stirred at room temperature and 60% NaH (0.0055 mol) was added portionwise over 10 minutes, then the mixture was stirred for 30 minutes and a mixture of 1-(chloromethyl)-4-methoxybenzene (0.0055 mol) in dry THF (10 ml) was added at once. The reaction mixture was stirred at room temperature for 6 days and the solvent was evaporated. The residue was stirred in $H_2O$ and extracted with $CH_2Cl_2$. The organic layer was separated, dried ($MgSO_4$), filtered off and the solvent was evaporated. Yield: 2.2 g of intermediate 11.

k. Preparation of intermediate 12

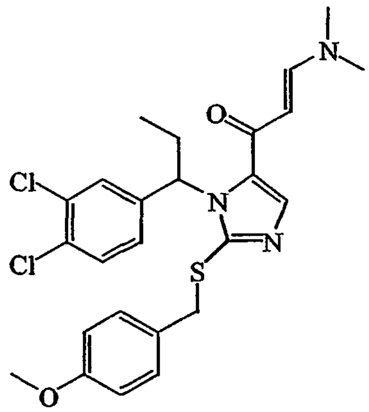

A mixture of intermediate 11 (prepared according to A1.j) (0.00115 mol) in 1,1-dimethoxy-*N,N*-dimethylmethanamine (10 ml) was stirred at 100°C for 18 hours and then the solvent was evaporated. The residue was dissolved in toluene and the solvent was evaporated again. Yield: 0.580 g of intermediate 12.

<u>1-1. Preparation of intermediate 13 and 14</u>

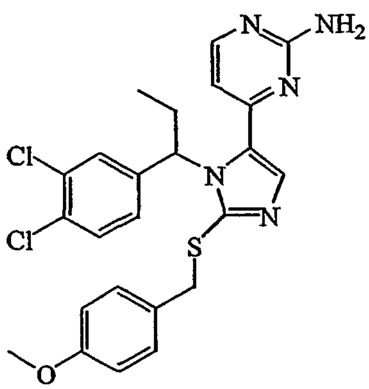

intermediate 13

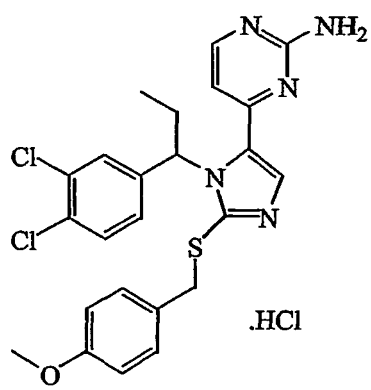

intermediate 14

A mixture of Na (0.0008 mol) in ethanol (4 ml) was stirred under $N_2$ at room temperature until complete dissolution and guanidine (0.0004 mol) was added, then the mixture was stirred for 30 minutes and a mixture of intermediate 12 (prepared according to A1.k) (0.00055 mol) in ethanol (1 ml) was added. The reaction mixture was stirred in a closed vessel at 100°C for 20 hours and evaporated. The crude compound was purified over $SiO_2$ (eluent: $CH_2Cl_2/CH_3OH$ 98/2) and the solvent was evaporated, yielding intermediate 13. The residue was dissolved in 2-propanol and converted into the hydrochloric acid salt (1:1) with HCl/2-propanol. The crystallised salt was filtered off, washed with DIPE and dried. Yield: 0.320 g of intermediate 14.

<u>1-2. Preparation of intermediate 15</u>

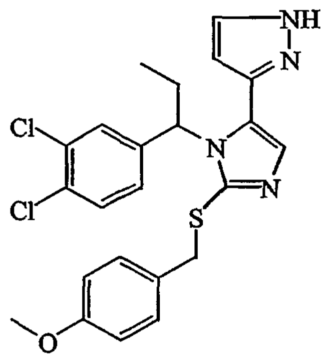

A mixture of intermediate 12 (prepared according to A1.k) (0.00115 mol) and hydrazine monohydrate (0.007 mol) in ethanol (20 ml) was stirred and refluxed on an oil bath for 3 hours. The solvent was evaporated and the residue was filtered over silica gel (eluent: $CH_2Cl_2/CH_3OH$ 98/2). The product fractions were collected and the solvent was evaporated at 50˚C under a stream of $N_2$. Yield: 0.240 g of intermediate 15.

Example A2

a. Preparation of intermediate 16

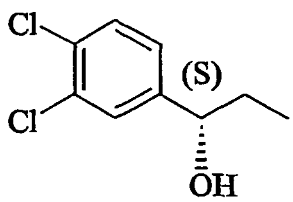

A mixture of *N,N'*-(1R,2R)-1,2-cyclohexanediylbis[1,1,1-trifluoromethanesulfonamide] (0.005 mol) and Ti (*i*-PrO)$_4$ (0.030 mol) in toluene (q.s.) was degassed and placed under Ar-flow, then the reaction mixture was stirred for 20 minutes at 40˚C and cooled to -78˚C. $Et_2Zn$ (0.030 mol) was added dropwise and after 20 minutes, a mixture of 3,4-dichlorobenzaldehyde (0.0250 mol) in toluene (q.s.) was added dropwise. The reaction mixture was allowed to reach 0˚C. The mixture was stirred overnight at room temperature, then quenched with HCl (2N). This mixture was extracted with $CH_2Cl_2$. The separated organic layer was washed, dried, filtered and the solvent evaporated. The residue was purified by column chromatography over silica gel (eluent $CH_2Cl_2$ /$CH_3OH$ 98/2). The product fractions were collected and the solvent was evaporated. Yield: 5.1 g of intermediate 16.
The R isomer can be prepared by the above reaction by using *N,N'*-(1S,2S)-1,2-cyclohexanediylbis[1,1,1-trifluoromethanesulfonamide] as catalyst.

b. Preparation of intermediate 17

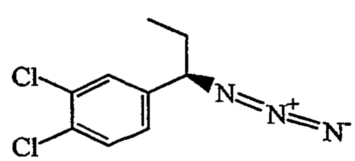

A mixture of intermediate 16 (prepared according to A2.a) (0.025 mol) and diphenylphosphoryl azide (0.030

mol) in toluene (50 ml) was stirred at 0°C and 2,3,4,6,7,8,9,10-octahydropyrimido[1,2-a]azepine (0.030 mol) was added. The reaction mixture was stirred for 2 hours at 0°C, then stirred overnight at room temperature. The mixture was diluted with water and toluene. The organic layer was separated, washed once with water, once with 5% HCl, and the solvent was evaporated, yielding intermediate 17, used in next reaction step.

c. Preparation of intermediate 18

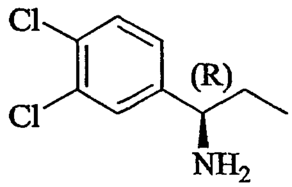

A mixture of intermediate 17 (prepared according to A2.b) (0.025 mol), triphenylphosphine (0.027 mol) in THF (70 ml) and $H_2O$ (20 ml) was stirred overnight at room temperature. The solvent was evaporated. The residue was treated with 10% HCl. The acidic layer was washed with DIPE, then alkalized, followed by an extraction with $CH_2Cl_2$. The separated organic layer was dried, filtered and the solvent evaporated, The residue was purified by column chromatography over silica gel. The product fractions were collected and the solvent was evaporated. Yield: 1.1 g of intermediate 18.

d. Preparation of intermediate 19

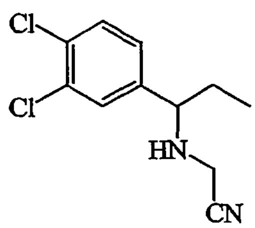

A solution of intermediate 3 (prepared according to A1.b) (0.0116 mol) in $Et_3N$ (0.013 mol) and DMF, p.a., dried on molecular sieves (20 ml) was stirred on an ice bath. A solution of chloroacetonitrile (0.0128 mol) in DMF, p.a., dried on molecular sieves, (2.5 ml) was added dropwise. The reaction mixture was stirred at room temperature for 6 hours. More chloroacetonitrile (0.0063 mol) in DMF, p.a., dried on molecular sieves (1 ml) was added dropwise. The reaction mixture was stirred for another 24 hours. More chloroacetonitrile (0.0063 mol) in DMF, p.a., dried on molecular sieves (1 ml) was added dropwise and the reaction mixture was stirred for another 24 hours. More $Et_3N$ (1 ml) was added, then more chloroacetonitrile(0.0079 mol) in DMF, p.a., dried on molecular sieves, (1 ml) was added dropwise. The reaction mixture was stirred for 20 hours. The precipitate was filtered off. The filtrate was poured out into $Et_2O$ (200 ml) and washed with $H_2O/NaHCO_3$ (10 %;100 ml) and $H_2O$ (2x). The separated organic layer was dried ($MgSO_4$), filtered and the solvent was evaporated and co-evaporated with toluene. The residue was purified over silica gel (eluent : $CH_2Cl_2$/MeOH 99:1). The desired fractions were collected and the solvent was evaporated and co-evaporated with toluene. Yield: 2.3g of intermediate 19 (81.6%).

e. Preparation of intermediate 20

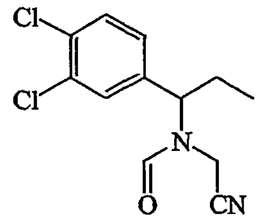

A mixture of intermediate 19 (prepared according to A2.d) (0.021 mol) in *n*-butyl formate (25 ml) was stirred and refluxed for 48 hours and then cooled. The solvent was evaporated and the residue was diluted with $CH_2Cl_2$. The resulting mixture was washed with water, dried and the solvent was evaporated. Yield: 5.25 g of intermediate 20.

f Preparation of intermediate 21

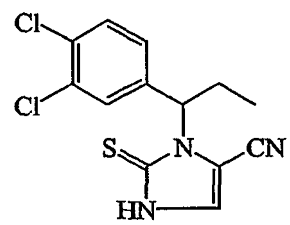

NaOMe (prepared in situ) (q.s.) was added dropwise to a mixture of intermediate 20 (prepared according to A2.e) (0.022 mol) and acetaldehyde (0.022 mol) in THF (100 ml). After 7 hours, the solvent was evaporated and the residue was partitioned between water and ether. The aqueous layer was acidified with concentrated HCl to pH 2-3 and extracted with $CH_2Cl_2$. The extract was evaporated and the residue was dissolved in $CH_3OH/H_2O$. KSCN (0.050 mol) was added, followed by concentrated HCl (5 ml) and then the reaction mixture was stirred and refluxed over the weekend. The mixture was cooled and the solvent was evaporated. The residue was partitioned between water and $CH_2Cl_2$, then the organic layer was separated, dried and the solvent was evaporated. The residue was purified by flash column chromatography over silica gel (eluent: Hexane/EtOAc 80/20). The product fractions were collected and the solvent was evaporated. Yield: 2 g of intermediate 21. Remark: NaOMe was generated in situ with NaH and $CH_3OH$ in THF.

g. Preparation of intermediate 22

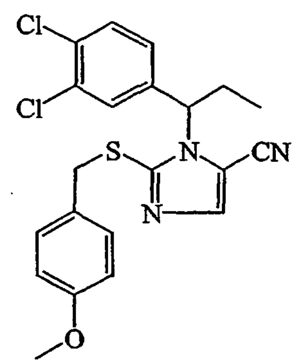

A mixture of intermediate 21 (prepared according to A2.f) (0.0128 mol) in trifluoroacetic acid (5 ml) and $CH_2Cl_2$ (40 ml) was stirred at 0-5˚C on an ice bath and a solution of 4-methoxybenzenemethanol (0.017 mol) in $CH_2Cl_2$ (10 ml) was added dropwise over 30 minutes at 0-5˚C, then the reaction mixture was stirred for 2 hours at 0-5˚C and the solvent was evaporated (vacuum). The residue was dissolved in $CH_2Cl_2$ and washed with $H_2O$ and with $NaHCO_3$. The organic

layer was separated, dried ($MgSO_4$), filtered off and the solvent was evaporated. The residue was purified over silica gel (eluent: $CH_2Cl_2/CH_3OH$ 99/1). The product fractions were collected and the solvent was evaporated. Yield: 4.3 g of intermediate 22.

h. Preparation of intermediate 23

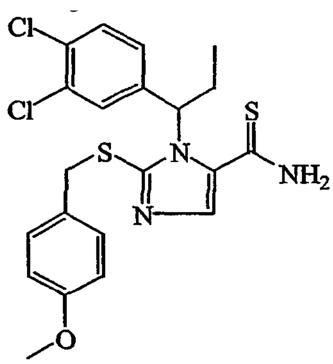

A mixture of intermediate 22 (prepared according to A2.g) (0.01 mol) and *N*-ethyl-*N*-(1-methylethyl)-2-propanamine (0.02 mol) in pyridine (100 ml) was stirred at 80°C and then $H_2S$ (gas) was passed through the solution for 5 hours, then $N_2$ was passed through for 2 hours at 80°C in order to remove the $H_2S$. The solvent was evaporated and the residue was dissolved in $CH_2Cl_2$. The solution was washed with $H_2O$ and with 1N HCl. The organic layer was separated, dried ($MgSO_4$), filtered off and the solvent was evaporated. The residue was purified over silica gel (eluent: $CH_2Cl_2/CH_3OH$ 99/1). The product fractions were collected and the solvent was evaporated. Yield: 3 g of intermediate 23.

i-1. Preparation of intermediate 24

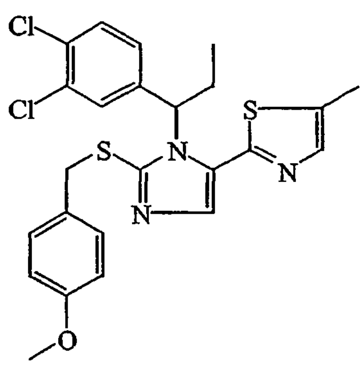

A mixture of intermediate 23 (prepared according to A2.h) (0.0005 mol) and 1-chloro-2-propanone (0.00055 mol) in ethanol (4 ml) was stirred at 80°C for 3 hours, then the mixture was left to stand overnight at room temperature and the solvent was evaporated. The residue was purified over silica gel (eluent: $CH_2Cl_2/CH_3OH$ 97/3). The product fractions were collected and the solvent was evaporated, giving fraction (I). The fractions containing intermediate 23 were collected and the solvent was evaporated. Ethanol (4 ml) and extra 1-chloro-2-propanone (0.04 ml) were added to the residue and the reaction mixture was stirred for 3 hours. The solvent was evaporated and the residue was filtered over silica gel (eluent: $CH_2Cl_2/CH_3OH$ 97/3). The product fractions were collected and the solvent was evaporated, giving fraction (II). Finally, fraction (I) and fraction (II) were combined. Yield: 0.030 g of intermediate 24

i-2. Preparation of intermediate 25

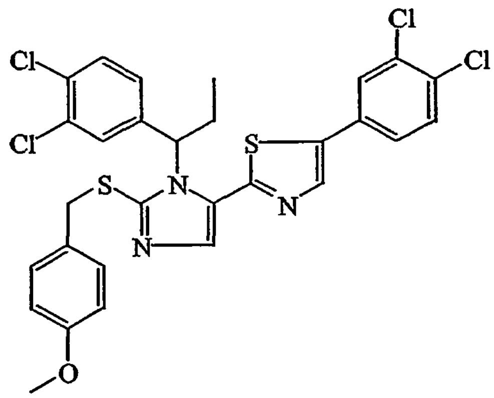

A mixture of intermediate 23 (prepared according to A2.h) (0.0005 mol) and 2-bromo-1-(3,4-dichlorophenyl) ethanone (0.0007 mol) in ethanol (4 ml) was stirred in a closed vessel for 18 hours at room temperature and then the solvent was evaporated. The residue was filtered 2 times (eluent: $CH_2Cl_2/CH_3OH$ 99/1, 100/0), then the product fractions were collected and the solvent was evaporated. Yield: 0.120 g of intermediate 25.

Example A3

a. Preparation of intermediate 26

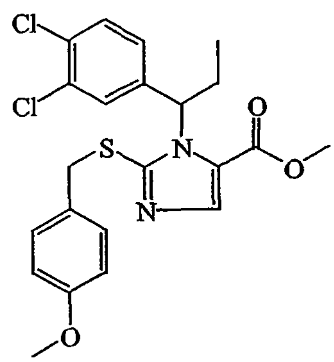

A mixture of intermediate 6 (prepared according to A1.e) (0.03 mol) in trifluoroacetic acid (20 ml) and $CH_2Cl_2$ (150 ml) was stirred at 0-5°C on an ice bath and a mixture of 4-methoxybenzenemethanol (0.033 mol) in $CH_2Cl_2$ (50 ml) was added dropwise over 30 minutes at 0-5°C, then the reaction mixture was stirred for 1 hour at 0-5°C and the solvent was evaporated. The residue was dissolved in $CH_2Cl_2$ and washed with $H_2O$ and with $NaHCO_3$. The organic layer was separated, dried ($MgSO_4$), filtered off and the solvent was evaporated, finally the residue was dried. Yield: 13.9 g of intermediate 26.

b. Preparation of intermediate 27

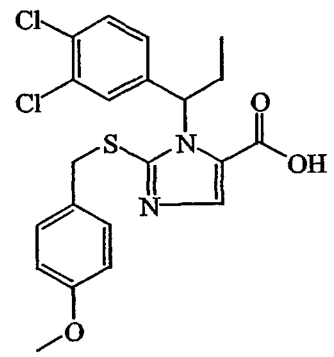

A mixture of intermediate 26 (prepared according to A3.a) (0.03 mol) in 1N NaOH (150 ml) and methanol (150 ml) was stirred at 80˚C for 18 hours and then the organic solvent (methanol) was evaporated. 1N HCl (150 ml) was added to the aqueous concentrate, then the resulting precipitate was filtered off and dried. Yield: 12 g of intermediate 27.

c. Preparation of intermediate 28

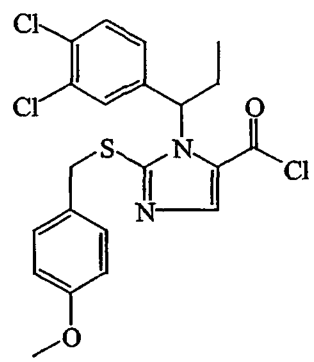

A mixture of intermediate 27 (prepared according to A3.b) (0.026 mol) in $SOCl_2$ (100 ml) was stirred and refluxed for 2 hours, then the solvent was evaporated and co-evaporated 2 times with toluene. Yield: 12.5 g of intermediate 28.

d. Preparation of intermediate 29

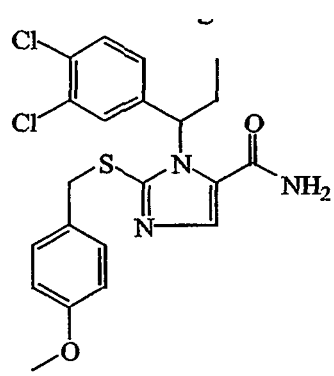

A solution of intermediate 28 (prepared according to A3.c) (0.026 mol) in THF (200 ml) was added dropwise to stirring $NH_3/H_2O$ (50 ml) at room temperature and the reaction mixture was stirred for 2 hours. The solvent was partly

(1/2) evaporated and the concentrate was extracted with $CH_2Cl_2$. The organic layer was separated, dried ($MgSO_4$), filtered off and the solvent was evaporated. The residue was purified over silica gel (eluent: $CH_2Cl_2/CH_3OH$ 95/5). The product fractions were collected and the solvent was evaporated. Yield: 3.5 g of intermediate 29.

e. Preparation of intermediate 30

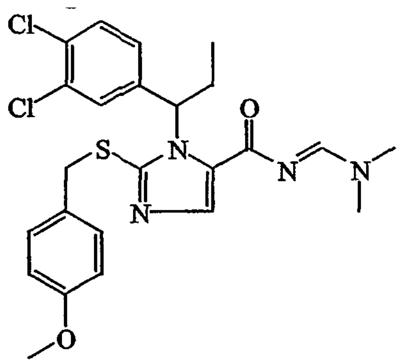

A mixture of intermediate 29 (prepared according to A3.d) (0.0022 mol) in DMF/DMA (25 ml) was stirred at 100°C for 18 hours and then the solvent was evaporated. The residue was filtered 2 times over silica gel (eluent: $CH_2Cl_2/CH_3OH$ 99/1), then the product fractions were collected and the solvent was evaporated. Yield: 0.51 g of intermediate 30.

f Preparation of intermediate 31

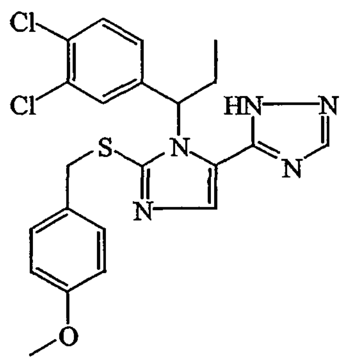

A mixture of intermediate 30 (prepared according to A3.e) (0.001 mol) and hydrazine monohydrate (0.0015 mol) in acetic acid (5 ml) was stirred in a closed vessel for 2 hours at 90°C and then the solvent was evaporated. The residue was dissolved in $CH_2Cl_2$ and washed with $H_2O$ and with $K_2CO_3$. The organic layer was separated, dried ($MgSO_4$), filtered off and the solvent was evaporated. The residue was purified by column chromatography over silica gel (eluent: $CH_2Cl_2/CH_3OH$ 97/3). The product fractions were collected and the solvent was evaporated. Yield: 0.200 g of intermediate 31.

Example A4

a. Preparation of intermediate 32

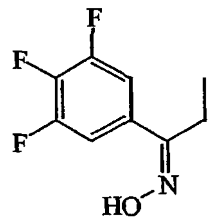

A mixture of 1-(3,4,5-trifluorophenyl)-1-propanone (0.106 mol), hydroxylamine (0.150 mol) and NaOAc (0.150 mol) in methanol (q.s.) was stirred overnight at room temperature, then the reaction mixture was filtered and the filtrate was evaporated. Yield: 21.5 g of intermediate 32.

b. Preparation of intermediate 33

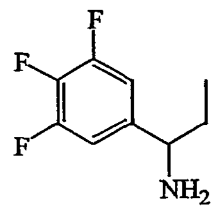

A mixture of intermediate 32 (prepared according to A4.a) (0.11 mol) in $CH_3OH/NH_3$ (500 ml) was hydrogenated with Raney Nickel (2 g) as a catalyst. After uptake of $H_2$ (2 equivalents), the catalyst was filtered off and the filtrate was evaporated. Yield: 20 g of intermediate 33.

c. Preparation of intermediate 34

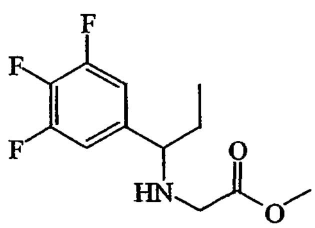

A mixture of intermediate 33 (prepared according to A4.b) (0.105 mol) and bromoacetic acid methyl ester (0.196 mol) in $Et_3N$ (30 ml) was reacted overnight at room temperature and then extra bromoacetic acid methyl ester (10 g) was added. *N,N*-dimethyl-4-pyridinamine (1 g) was added and the reaction mixture was stirred for 3 days at room temperature. The solvent was evaporated and the residue was diluted with $CH_2Cl_2$. The resulting solids were filtered off and dried (vacuum) at 50°C. Yield: 6.8 g of intermediate 34.

d. Preparation of intermediate 35

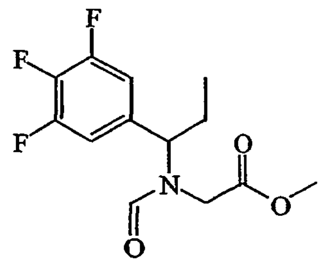

A mixture of acetylchloride (0.1 mol) and $HCO_2Na$ (10 g) in $Et_2O$ (100 ml) was stirred for 6 hours at room temperature and then the solids were filtered off. A mixture of intermediate 34 (prepared according to A4.c) (0.024 mol) in $Et_2O$ (q.s.) was added and the reaction mixture was stirred for 4 hours at room temperature. The solvent was evaporated and the residue was quenched with $NaHCO_3$, then extracted with $CH_2Cl_2$. The organic layer was separated, dried ($MgSO_4$), filtered off and the solvent was evaporated. Yield: 4.4 g of intermediate 35.

e. Preparation of intermediate 36

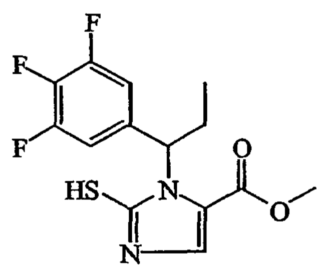

*t*-BuONa (2 g) was added to a mixture of intermediate 35 (prepared according to A4.d) (0.0052 mol) in acetic acid methyl ester (3 ml) and THF (80 ml) at room temperature and the mixture was reacted for 20 minutes, then water (20 ml) was added and the solvent was concentrated until a volume of 30 ml was left. Water (20 ml) and methanol (40 ml) were added to the residue, followed by the addition of KSCN (3 g) and HCl (3 ml). The reaction mixture was heated for 3 hours and quenched with $Na_2CO_3$ (to neutral). The solvent was concentrated until a volume of 40 ml was left and the concentrate was extracted with $CH_2Cl_2$. The organic layer was dried over $MgSO_4$, filtered and evaporated. Yield: 1.5 g of intermediate 36 (88 %).

f. Preparation of intermediate 37

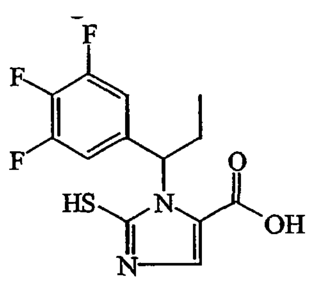

A mixture of intermediate 36 (prepared according to A4.e) (0.0015 mol) in 1M NaOH (5 ml) and methanol (3 ml) was stirred and refluxed for 24 hours at 100°C, then the reaction mixture was cooled and the solvent was evaporated. The residue was quenched with concentrated HCl and the desired product was collected. Yield: 360 mg of intermediate 37.

Example A5

a. Preparation of intermediate 38

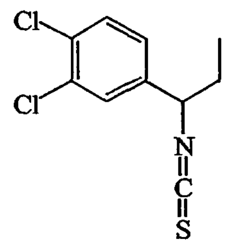

*N*-ethyl-*N*-(1-methylethyl)-2-propanamine (0.1 mol) was added to a stirring mixture of intermediate 3 (prepared according to A1.b) (0.0415 mol) in $CH_2Cl_2$, p.a. (100 ml) under $N_2$. After 15 minutes of stirring, the reaction mixture was put on an ice bath and a solution of carbonothioic dichloride (0.0457 mol) in $CH_2Cl_2$, p.a. (15 ml) was added dropwise at 0˚C. The mixture was stirred at 0˚C for 30 minutes and at room temperature for 18 hours, then extra *N*-ethyl-*N*-(1-methylethyl)-2-propanamine (9 ml) was added and the resulting mixture was stirred for 2 hours. The mixture was washed 2 times with $H_2O$, once with HCl (1N) and again with $H_2O$. The organic layer was separated, dried ($MgSO_4$), filtered off and the solvent was evaporated, then co-evaporated with toluene. The residue was purified by column chromatography over silica gel (eluent: $CH_2Cl_2$/Hexane 15/85). The product fractions were collected and the solvent was evaporated. Yield: 7.4 g of intermediate 38 (72.4 %).

b-1. Preparation of intermediate 39

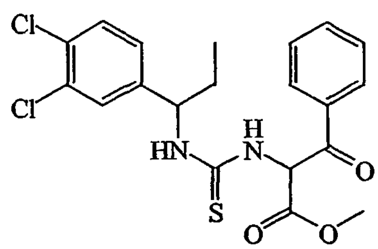

Beta-oxo-phenylalanine methyl ester monohydrochloride (0.00175 mol), followed by $K_2CO_3$ (0.00175 mol) and then $H_2O$ (5 ml) were added to a solution of intermediate 38 (prepared according to A5.a) (0.00175 mol) in THF (20 ml) and the reaction mixture was stirred at room temperature for 18 hours. The mixture was poured out into $H_2O$ (50 ml) and extracted with $CH_2Cl_2$. The organic layer was separated, dried ($MgSO_4$), filtered off and the solvent was evaporated. The residue was purified by flash column chromatography (eluent: $CH_2Cl_2$/$CH_3OH$). The product fractions were collected and the solvent was evaporated. Yield: 0.095 g of intermediate 39 (12.4 %).

b-2. Preparation of intermediate 40

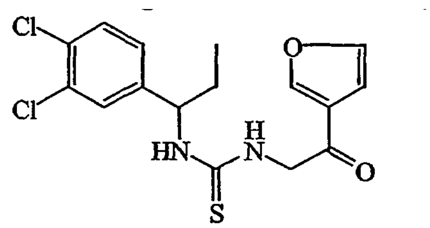

2-amino-1-(2-furanyl)-ethanone monohydrochloride (0.00146 mol) was added to a solution of intermediate 38 (prepared according to A5.a) (0.00122 mol) in 1,4-dioxane, p.a. (6 ml), then $H_2O$ (0.5 ml) was added followed by $K_2CO_3$ (0.00122 mol). The reaction mixture was stirred at room temperature for 18 hours and the solvent was evaporated, yielding intermediate 40. The residue was used further in the next step.

Example A6

Preparation of intermediate 41

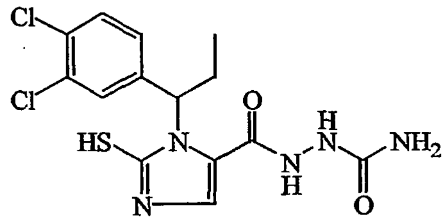

Hydrazinecarboxamide monohydrochloride (0.0078 mol) was added to a solution of intermediate 8 (prepared according to A1.g) (0.0052 mol) in THF, p.a. dried on molecular sieves (40 ml) under $N_2$, then *N*-ethyl-*N*-(1-methylethyl)-2-propanamine (0.016 mol) was added and the reaction mixture was stirred for 18 hours at room temperature. The solvent was evaporated and the residue was stirred in a half saturated aqueous $K_2CO_3$ solution. The resulting mixture was washed with $CH_2Cl_2/CH_3OH$ (95/5) and acidified with concentrated HCl. The formed precipitate was filtered off, washed with $H_2O$ and with DIPE and then dried (vacuum) at 50°C. Yield: 0.42 g of intermediate 41.

Example A7

Preparation of intermediate 43

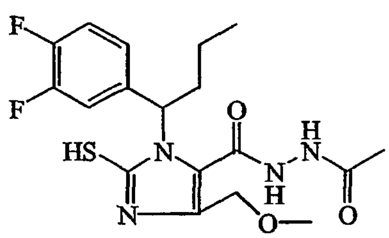

A mixture of intermediate 42 (prepared according to A1.f-2) (0.001 mol), *N*'-(ethylcarbonimidoyl)-*N*,*N*-dimethyl-1,3-propanediamine (0.001 mol) and 1-hydroxy-1*H*-benzotriazole (0.001 mol) in DMF (5 ml) was stirred for 30 minutes at room temperature, then hydrazide acetic acid (0.003 mol) was added and the reaction mixture was stirred overnight at room temperature. The solvent was evaporated, then the residue was stirred in $H_2O$ and the mixture was extracted with $CH_2Cl_2/CH_3OH$ (90/10). The organic layer was separated, dried, filtered off and the solvent was evaporated. The

obtained residue was stirred in $CH_2Cl_2$ and after solidification the desired product was filtered off and dried. Yield: 0.170 g of intermediate 43.

B. Preparation of the final compounds

Example B1

Preparation of compound 1

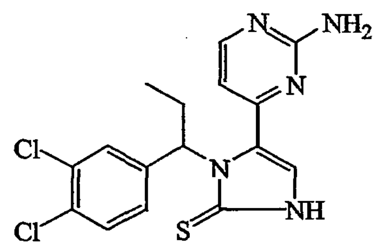

A mixture of intermediate 14 (prepared according to A1.1-1) (0.00055 mol) in trifluoroacetic acid (3 ml) was stirred at 80°C in a closed vessel for 4 hours and then the solvent was evaporated at 80°C under a stream of $N_2$. The residue was stirred in $CH_2Cl_2$ and treated with $H_2O$ and $K_2CO_3$. The organic layer was separated, dried ($MgSO_4$), filtered off and the solvent was evaporated. The residue was stirred in EtOAc (2 ml) and after filtration the desired product was dried. Yield: 0.020 g of compound 1.

Example B2

Preparation of compound 2

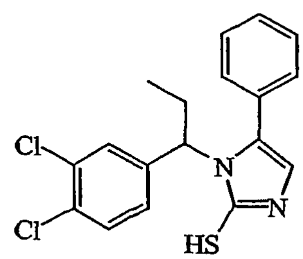

2-amino-2-benzoylacetamide monohydrochloride (0.000158 mol), $H_2O$ (0.5 ml) and $K_2CO_3$ (0.000154 mol) were added to a solution of intermediate 38 (prepared according to A5.a) (0.000154 mol) in 1,4-dioxane, p.a. (2.5 ml) and then the reaction mixture was stirred vigorously for 20 hours. 36% HCl, p.a. (0.5 ml) was added and the resulting mixture was stirred for 4 hours at 75°C and then allowed to reach room temperature, $H_2O$ was added and the mixture was extracted with EtOAc. The organic layer was separated, dried ($MgSO_4$), filtered off and the solvent was evaporated. The residue was purified by reversed phase high-performance liquid chromatography. The desired product fractions were collected and the organic volatiles were evaporated. The product was extracted with $CH_2Cl_2$ and the separated organic layer was evaporated. Yield: 0.0076 g of compound 2.

Example B3

a. Preparation of compound 3

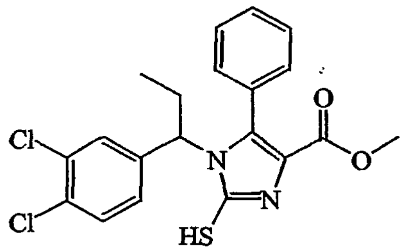

A solution of intermediate 39 (prepared according to A5.b-1) (0.0002 mol) in acetic acid (6 ml) was stirred for 18 hours in a sealed tube at 100°C, then the reaction mixture was allowed to reach room temperature and was poured out into $H_2O$. $CH_2Cl_2$ was added, then a saturated $K_2CO_3$ solution was added until a clear biphasic solution was formed. The organic layer was separated, dried ($MgSO_4$), filtered off and the solvent was evaporated, then co-evaporated with toluene. The residue was purified by high-performance liquid chromatography over RP-18 (eluent: (10 % $NH_4OAc$ in $H_2O$)/$CH_3OH$/$CH_3CN$). The product fractions were collected and the solvent was evaporated for 50%. The concentrate was extracted with $CH_2Cl_2$ and the separated organic layer was evaporated. Yield: 0.011 g of compound 3.

b. Preparation of compound 4

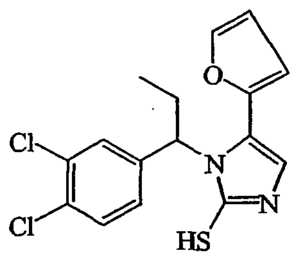

36% HCl, p.a. (2 ml) was added to a solution of intermediate 40 (prepared according to A5.b-2) (0.0012 mol) in 1,4-dioxane, p.a. (12 ml) and the resulting solution was stirred at 65°C for 18 hours. $H_2O$ (10 ml) was added and the mixture was extracted with EtOAc. The organic layer was separated, dried ($MgSO_4$), filtered off and the solvent was evaporated. The residue was dissolved in 2-propanone (15 ml, p.a.) and the solution was treated with $SO_2$ (gas) for 15 minutes. The solvent was evaporated and the residue was purified by flash column chromatography (gradient eluent: $CH_2Cl_2$/$CH_3OH$ 99.8/0.2 -> 99.6/0.4). The product fractions were collected and the solvent was evaporated. Yield: 0.0134 g of compound 4.

Example B4

Preparation of compound 5

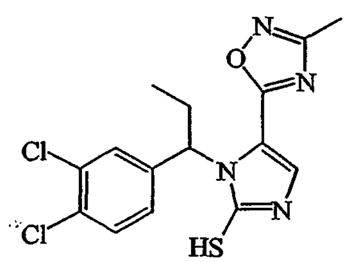

A mixture of intermediate 6 (prepared according to A1.e) (0.00072 mol) and *N*-hydroxy-ethanimidamide (0.00181 mol) in methanol (3 ml) and $NaOCH_3/CH_3OH$ (30%) (1 ml) was reacted at 100°C in a sealed tube for 18 hours and then the reaction mixture was quenched with an aqueous $NH_4Cl$ solution. The solvent was evaporated and the residue was extracted with $CH_2Cl_2$. The organic layer was separated, filtered through a drying cartridge and the solvent was evaporated. The residue was purified by high-performance liquid chromatography. The product fractions were collected and the solvent was evaporated. Yield: 0.110 g of compound 5.

Example B5

Preparation of compound 6

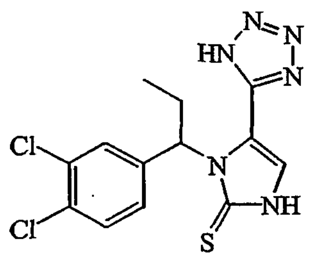

tBuOK (10 g) was added dropwise to a mixture of intermediate 20 (prepared according to A2.e) (0.063 mol) and methyl ester formic acid (0.32 mol) in THF (300 ml). After 7 hours, the solvent was evaporated and the residue was partitioned between water and ether. The aqueous layer was acidified with concentrated HCl to pH 2-3 and extracted with $CH_2Cl_2$. The extract was evaporated and the residue was dissolved in $CH_3OH/H_2O$. KSCN (15 g) was added, followed by concentrated HCl (q.s.) and then the reaction mixture was stirred and refluxed over the weekend. The mixture was cooled and the solvent was evaporated. The residue was partitioned between water and $CH_2Cl_2$, then the organic layer was separated, dried and the solvent was evaporated. The residue was purified by flash column chromatography over silica gel (eluent: Hexane/EtOAc 80/20). The product fractions were collected and the solvent was evaporated. Then the product fractions were collected and the solvent was evaporated, yielding

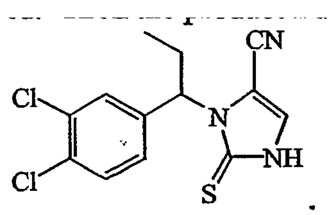

A mixture of 0.200 g of said intermediate, 0.5 g of $Bu_3SnN_3$ in 0.5 ml toluene was heated overnight at 130°C and the mixture was purified by reversed phase high performance liquid chromatography resulting in 20 mg of compound 6.

Example B6

a. Preparation of compound 7

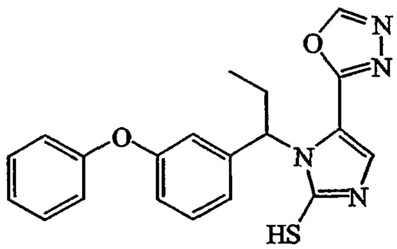

A mixture of intermediate 44.

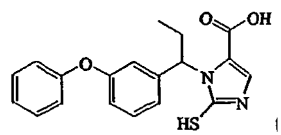

(prepared according to A1.f-1) (0.00037 mol) in $SOCl_2$ (25 ml) was stirred and refluxed for 4 hours, then the reaction mixture was cooled and the solvent was evaporated. The oily residue was diluted with THF (10 ml) and treated with hydrazinecarboxaldehyde (0.0042 mol), then the mixture was stirred for 30 minutes at room temperature and the solvent was evaporated. The residue was diluted with $CH_2Cl_2$ and dried by Solid Phase Extraction. The solvent was evaporated and the residue was diluted with $CH_3CN$. The resulting mixture was treated with $POCl_3$ (10%w) (1 ml) in $CH_3CN$ (5 ml) and heated for 5 hours. The mixture was cooled and the solvent was evaporated. The residue was extracted with $CH_2Cl_2$ and dried by Solid Phase Extraction, then the solvent was evaporated and the residue was purified by Reversed Phase high-performance liquid chromatography. The product fractions were collected and the solvent was evaporated. Yield: 22 mg of compound 7.

<u>b. Preparation of compound 8</u>

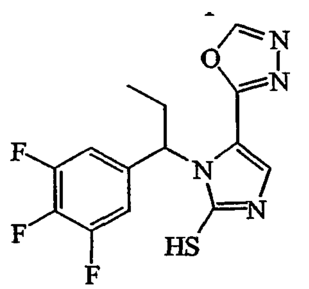

A mixture of intermediate 37 (prepared according to A4.f) (0.001 mol) in $SOCl_2$ (5 ml) was heated at 70°C for 4 hours, then the mixture was cooled and the solvent was evaporated. A part (1/3) of this fraction was taken up in THF (4 ml) and treated with hydrazinecarboxaldehyde (0.0025 mol), then the reaction mixture was heated for 1 hour. The solvent was evaporated and the residue was taken up in water (0.5 ml) and $CH_2Cl_2$. The resulting mixture was extracted with a Solid Phase Extraction cartridge and the solvent was evaporated. The residue was taken up in $CH_3CN$ (5 ml), then the mixture was treated with a 1M $POCl_3$ solution (0.0004 mol) and heated at 80°C for 12 hours. The resulting mixture was cooled and quenched with an aqueous $NaHCO_3$ solution. The solvent was evaporated and the residue was purified by Reversed Phase high-performance liquid chromatography. Finally, the product fractions were collected and the solvent was evaporated. Yield: 20 mg of compound 8.

Example B7

Preparation of compound 9

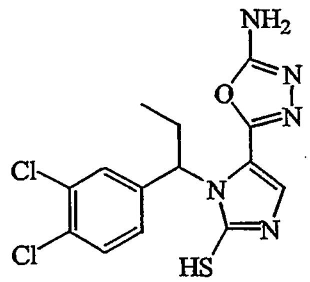

A mixture of intermediate 41 (prepared according to A6) (0.0009 mol) in $CH_3CN$ (15 ml) was stirred on a hot oil bath and phosphorus oxychloride (0.00107 mol) was added at 50°C and then the reaction mixture was stirred further for 2 hours at 90°C. The mixture was allowed to reach room temperature and was poured out into a half saturated, aqueous $NaHCO_3$ solution and extracted with $CH_2Cl_2/CH_3OH$ 98/2. The organic layer was separated, dried ($MgSO_4$), filtered off and the solvent was evaporated. The residue (0.21 g) was purified by reversed phase high-performance liquid chromatography ($NH_4HCO_3$-buffer). The product fractions were collected and the solvent was evaporated. Yield: 0.069 g of compound 9.

Example B8

Preparation of compound 10

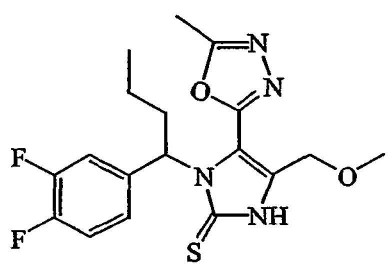

A mixture of intermediate 43 (prepared according to A7) (0.0004 mol) and Burgess'reagent (0.0012 mol) in THF (5 ml) was stirred for 3 hours at 60°C. $CH_2Cl_2$ was added and the reaction mixture was washed with $H_2O$. The organic layer was separated, dried, filtered off and the solvent was evaporated. The residue was filtered over silica gel (eluent: $CH_2Cl_2/CH_3OH$ 98/2). The pure product fractions were collected and the solvent was evaporated The obtained residue was stirred in DIPE, then the desired product was filtered off and dried. Yield: 0.044 g of compound 10 (m.p.: 97.8-97.9°C).

Tables 1 and 2 list the compounds of formula (I) which were prepared according to one of the above samples (Ex. No.)

Table 1

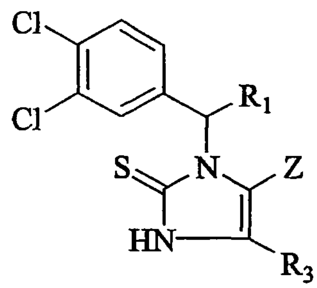

| Comp. No. | Exp. No. | $R_1$ | $R_3$ | Z | Properties |
|---|---|---|---|---|---|
| 11 | B1 | $-CH_2CH_3$ | H | 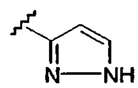 | |
| 12 | B1 | $-CH_2CH_3$ | H | 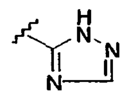 | |
| 6 | B5 | $-CH_2CH_3$ | H | 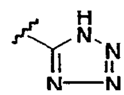 | |
| 4 | B3.b | $-CH_2CH_3$ | H | 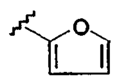 | |
| 13 | B7 | $-CH_2CH_3$ | H | 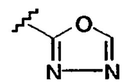 | |
| 5 | B4 | $-CH_2CH_3$ | H | 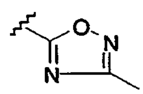 | |
| 14 | B7 | $-CH_2CH_3$ | H | 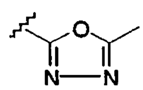 | m.p.227-228.5°C |
| 9 | B7 | $-CH_2CH_3$ | H | 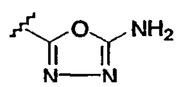 | |
| 15 | B1 | $-CH_2CH_3$ | H | 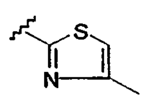 | |
| 16 | B1 | $-CH_2CH_3$ | H | 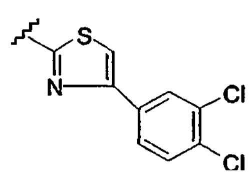 | |
| 2 | B2 | $-CH_2CH_3$ | H | 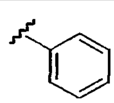 | |

(continued)

| Comp. No. | Exp. No. | $R_1$ | $R_3$ | Z | Properties |
|---|---|---|---|---|---|
| 17 | B3.b | $-CH_2CH_3$ | H | 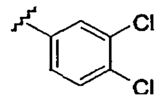 | |
| 18 | B3.b | $-CH_2CH_3$ | H | 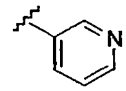 | |
| 19 | | $-CH_2CH_3$ | H | 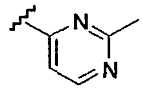 | |
| 1 | B1 | $-CH_2CH_3$ | H | 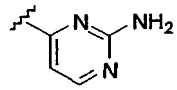 | m.p. 230.2-235.5°C |
| 20 | B1 | $-CH_2CH_3$ | H | 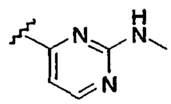 | |
| 21 | B1 | $-CH_2CH_3$ | H | 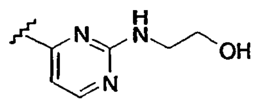 | |
| 22 | B1 | $-CH_2CH_3$ | H | 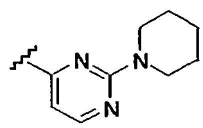 | |
| 23 | B1 | $-CH_2CH_3$ | H | 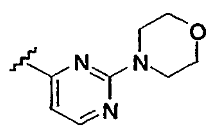 | |
| 24 | B1 | $-CH_2CH_3$ | H | 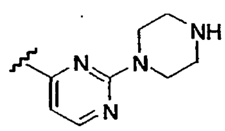 | |
| 25 | B1 | $-CH_2CH_3$ | H | 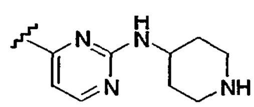 | |
| 3 | B3.a | $-CH_2CH_3$ | 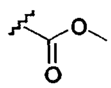 | 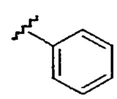 | |

Table 2

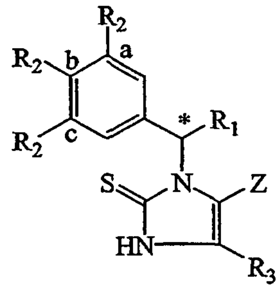

| Comp No. | Exp. No. | R1 | a | R2 b | c | R3 | Z | Properties |
|---|---|---|---|---|---|---|---|---|
| 26 | B1 | $-CH_2CH_3$ | F | $-CF_3$ | H | H | 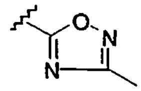 | |
| 27 | B7 | $-CH_2CH_3$ | F | H | F | H | 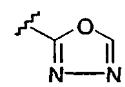 | |
| 28 | B7 | $-CH_2CH_3$ | F | H | F | H | 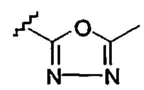 | |
| 8 | B6.b | $-CH_2CH_3$ | F | F | F | H | 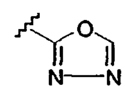 | |
| 7 | B6.a | $-CH_2CH_3$ | H | H | 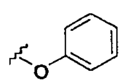 | H | 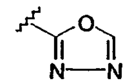 | |
| 10 | B8 | $-CH_2CH_2CH_3$ | F | F | H | $-CH_2-OCH_3$ | 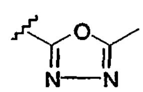 | m.p. 97.8-97.9 |
| 29 | B6.a | $-CH_2CH_2CH_3$ | F | F | H | H | 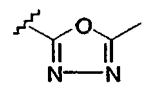 | |

C. Analytical Part

*LCMS conditions 1*

The HPLC gradient was supplied by a Waters Alliance HT 2790 system with a columnheater set at 40˚C. Flow from the column was split to a Waters 996 photodiode array (PDA) detector and a Waters-Micromass ZQ mass spectrometer with an electrospray ionization source operated in positive and negative ionization mode. Reversed phase HPLC was carried out on a Xterra MS C18 column (3.5 μm, 4.6 x 100 mm) (12 minutes column) with a flow rate of 1.6 ml/minutes. Three mobile phases (mobile phase A : 95% 25mM ammoniumacetate + 5% acetonitrile; mobile phase B: acetonitrile; mobile phase C: methanol) were employed to run a gradient condition from 100 % A to 50% B and 50% C in 6.5 minutes, to 100 % B in 1 minute, 100% B for 1 minute and reequilibrate with 100 % A for 1.5 minute. An injection volume of 10 μL was used.

Mass spectra were acquired by scanning from 100 to 1000 in 1s using a dwell time of 0.1 s. The capillary needle voltage

was 3kV and the source temperature was maintained at 140°C. Nitrogen was used as the nebulizer gas. Cone voltage was 10 V for positive ionzation mode and 20 V for negative ionization mode. Data acquisition was performed with a Waters-Micromass MassLynx-Openlynx data system.

Table 3 : LCMS parent peak ([M+] defines the mass of the compound) and retention time (minutes)

| Compound no. | [MH+] | Retention time |
|---|---|---|
| 5 | 368 | 6.11 |
| 26 | 387 | 5.53 |
| 14 | 369 | 5.40 |
| 13 | 355 | 5.31 |
| 28 | 337 | 4.49 |
| 27 | 323 | 4.37 |
| 3 | 421 | 6.06 |
| 11 | 353 | 5.10 |
| 7 | 377 | 6.12 |
| 17 | 433 | 6.58 |
| 4 | 353 | 5.99 |
| 18 | 364 | 5.74 |
| 1 | 380 | 4.97 |
| 2 | 363 | 5.98 |
| 16 | 516 | 6.75 |
| 12 | 354 | 4.78 |
| 15 | 384 | 6.50 |
| 8 | 341 | 4.90 |
| 22 | 448 | 6.73 |
| 20 | 394 | 5.47 |
| 25 | 463 | 4.47 |
| 19 | 379 | 5.72 |
| 23 | 450 | 5.84 |
| 24 | 449 | 4.50 |
| 21 | 424 | 4.86 |
| 9 | 370 | 3.90 |
| * [M+] defines the mass of the compound | | |

D. Pharmacological example

*Inhibition of MCP-1 induced Ca-flux in human THP-1 cells*

MCP-1 binding to the CCR2 receptor induces a rapid and transient intracellular release of $Ca^{2+}$ (secondary messenger) in several cell lines (Charo et al, PNAS 1994). Free $Ca^{2+}$ levels can be measured using a $Ca^{2+}$ sensitive dye. When the CCR2 receptor is blocked with a CCR2 receptor antagonist, the MCP-1 induced release of $Ca^{2+}$ is inhibited.

Human THP-1 cells (monocytic cell line, ATCC TIB-202) were cultured in RPMI 1640 medium supplemented with 10 % fetal calf serum (FCS), 1% L-Glutamine, penicillin (50 U/ml) and streptomycin (50 μg/ml) (all GIBCO BRL, Gent). After centrifugation, cells were loaded for 30 minutes with the $Ca^{2+}$ sensitive fluorescent dye Fluo-3 AM (Molecular Probes, Leiden, Netherlands) (2 million cells/ml in RPMI medium containing 4 μM Fluo-3 AM, 20 mM HEPES, 0.1 %

Bovine Serum Albumin (BSA) and 5 mM probenecid). Excess dye was removed by 3-fold washing with buffer (5 mM HEPES, 140 mM NaCl, 1 mM $MgCl_2$, 5 mM KCl, 10 mM glucose, 2.5 mM probenecid, 1.25 mM $CaCl_2$, 0.1 % BSA; all further incubations were done in this buffer). Cells were plated at a density of 150 000 cells/well in dark-wall 96-well plates (Costar, Cambridge, MA) and sedimented by centrifugation (1 minute). The cells were pre-incubated for 20 minutes with test compound. Then, $10^{-7}$ M hMCP-1 (Bachem, Bubendorf, Switserland) was added. Changes in intracellular free $Ca^{2+}$ concentration were measured using the Fluorescent Imaging Plate Reader (FLIPR, Molecular Devices, Munchen, Germany). Fluorescence was recorded every second from 10 seconds before the addition of the MCP-1 till 2 minutes after the addition (first minute: 60 records with 1 second intervals, second minute 20 records with 3 second intervals). The maximal fluorescence obtained during this time frame was used for further calculations.

Table 4 reports $pIC_{50}$ values obtained in the above-described test for compounds of formula (I). $pIC_{50}$ defines $-\log IC_{50}$ wherein $IC_{50}$ is the molar concentration of the test compound which inhibits 50 % of specific MCP-1 induced $Ca^{2+}$ flux.

Table 4

| Comp. No. | $pIC_{50}$ |
|---|---|
| 5 | 6.7 |
| 26 | 6.66 |
| 14 | 6.38 |
| 11 | 6.14 |
| 1 | 7.45 |
| 12 | 6.4 |
| 20 | 7.38 |
| 19 | 6.08 |
| 24 | 6.2 |
| 21 | 7.46 |
| 9 | 7.48 |

*Radioligand binding assay.*

$^{125}$I-MCP-1 binding assays were performed in 96-well plates with 40 μg of protein per well. Compounds were dissolved and diluted in DMSO to 100X dilutions. A 10X concentration range of compounds was prepared in binding buffer (10% DSMO). Competition binding assays contained the following components in a total volume of 250 μl: 25 μl of the appropriate compound dilution (final concentration of 1% DMSO), 200 μl membranes from CCR2B- transfected CHO cells dissolved in binding buffer and 25 μl $^{125}$I-MCP-1 (Bolton and Hunter labeled, Amersham, specific activity = 2000 Ci/mmol, 0.15 nM final). Binding buffer was composed of 25 mM HEPES, 5 mM $MgCl_2$, 1 mM $CaCl_2$, 0.5% protease-free bovine serum albumin, pH 7.4. After 90 minutes incubation at 25 °C, membranes were harvested on GF/B filters - presoaked in 0.5% polyethylenimine, followed by washing with buffer containing 25 mM HEPES, 5 mM $MgCl_2$, 1 mM $CaCl_2$, 5 mM NaCl, pH 7.4. Filter bound radioactivity was determined by liquid scintillation counting. $EC_{50}$ values (μM) and $K_i$ values (μM) were calculated. The $EC_{50}$ value indicates the concentration of the test compound that competes with MCP-1 for half of the specific binding sites; the $K_i$ value indicates the equilibrium dissociation constant, i.e. the concentration of the test compound that will bind to half of the binding sites at equilibrium in the absence of radioligand or other competitors. $EC_{50}$ values and $K_i$ values were calculated using non-linear regression in Graphpad Prism. Prism calculates the $K_i$ or affinity of the receptor for the competing drug using the equation of Cheng and Prusoff (Biochem. Pharmacol. 1973, 22: 3099-3108). A low $K_i$ indicates a high affinity of the receptor for the test compound.

$$K_i = \frac{EC_{50}}{1 + \frac{[radioligand]}{K_d}}$$

wherein $K_d$ describes the affinity of the radioligand for the receptor, i.e. the concentration of the radioligand that will bind

to half of the binding sites at equilibrium in the absence of competitors.
Compound 1 has a $K_i$ (μM) of 0.17.

*Chemotactic response*

The CCR2 antagonistic activity of the compounds of the present invention can also be determined by measuring the effect of the compounds on the chemotactic response of cells in the presence of a chemokine, such as for example MCP-1.

Mononuclear cells from human heparinized peripheral blood (PBMC) were isolated using Ficoll-Paque gradient centrifugation (Amersham Biosciences). Assays of chemotactic responsiveness were performed using disposable 96-well chemotaxis chambers (ChemoTx, Neuro Probe) with 5-μm pore size polycarbonate (PVP-free) filter membranes. Mononuclear cells were fluorescently labeled with 5 μg/ml Calcein-AM (Molecular Probes, Eugene, OR) at 37°C for 30 minutes. Labeled cells were washed twice and resuspended at $5x10^6$ cells/ml in Hanks' Balanced Salt Solution (Gibco BRL) supplemented with 0.2% bovine serum albumin. Subsequently, cells were pre-incubated for 10 minutes at room temperature with serial dilutions of the compounds in DMSO (dimethylsulfoxide) (final DMSO concentration of 0.2%). Bottom wells of the chemotaxis chamber were loaded with 28 μl medium containing 30 ng/ml recombinant hMCP-1 (R&D) or buffer only. Pre-treated cells (100.000 cells) were added in triplicate to the topside of the filter (20 μl) and incubated at 37°C in humidified air containing 5% $CO_2$. After 105 minutes incubation, the non-migrated cells were removed from the top of the filter by gently wiping the filter with a tissue. The migrated cells were measured using a fluorescent plate reader ($\lambda_{excitation}$ = 485 nm; $\lambda_{emission}$ = 538 nm). The chemotactic response can be expressed as chemotactic index (C.I.), being the ratio of the means of migrated cells in the presence of MCP-1 and the means of migrated cells in the absence of chemokine. Percentage inhibition can be calculated using the formula:

$$\%inhibition = (1 - \frac{F_{sample} - F_{buffer}}{F_{MCP-1} - F_{buffer}})x100$$

with $F_{sample}$, the fluorescence of the cells pre-incubated with 10, 1, 0.1, 0.01 or 0.001 μM compound and migrated to 30 ng/ml MCP-1 in the bottom wells; $F_{MCP-1}$, the fluorescence of the cells pre-incubated with buffer-0.2% DMSO and migrated to 30 ng/ml MCP-1 and $F_{buffer}$, the fluorescence of cells pre-incubated with buffer-0.2% DMSO and spontaneous migrated to buffer in the bottom wells.

## Claims

**1.** A compound of formula (I)

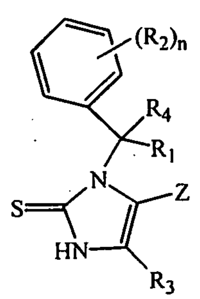

(I)

,

a *N*-oxide, a pharmaceutically acceptable addition salt, a quaternary amine or a stereochemically isomeric form thereof, wherein

$R_1$ represents $C_{1-6}$alkyl;
each $R_2$ independently represents halo or polyhalo$C_{1-6}$alkyl;
$R_3$ represents hydrogen, cyano, $C_{1-6}$alkyl optionally substituted with hydroxy or $C_{1-6}$alkyloxy, C(=O)-O-$R_5$, C(=O)-$NR_{6a}R_{6b}$, C(=S)-$NR_{6a}R_{6b}$, $S(=O)_2$-$NR_{6a}R_{6b}$ or C(=O)-$R_7$;
$R_4$ represents hydrogen;

$R_5$ represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl or aryl;

$R_{6a}$ and $R_{6b}$ each independently represent hydrogen, $C_{1-6}$alkyl, amino, mono- or di($C_{1-4}$alkyl)amino, arylNH-, amino$C_{1-6}$alkyl, mono-or di($C_{1-4}$alkyl)amino-$C_{1-6}$alkyl, $C_{1-6}$alkylcarbonylamino, aminocarbonylamino, $C_{1-6}$alkyloxy, carbonylamino or hydroxy$C_{1-6}$alkyl; or

$R_{6a}$ and $R_{6b}$ taken together with the nitrogen to which they are attached form pyrrolidinyl, imidazolidinyl, pyrazolidinyl, piperidinyl, piperazinyl, morpholinyl, thiomorpholinyl or piperazinyl substituted with $C_{1-6}$alkyl;

$R_7$ represents hydrogen, $C_{1-6}$alkyl, hydroxy$C_{1-6}$alkyl, $C_{2-6}$alkenyl, $C_{2-6}$alkynyl, polyhalo$C_{1-6}$alkyl, $C_{1-6}$alkyloxy$C_{1-6}$alkyl, amino$C_{1-6}$alkyl, mono-or di($C_{1-4}$alkyl)amino$C_{1-6}$alkyl, aminocarbonyl$C_{1-6}$alkyl, mono-or di($C_{1-4}$alkyl)aminocarbonyl$C_{1-6}$alkyl, aryl or heteroaryl;

Z represents a cyclic ring system selected from

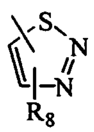
(a-1)

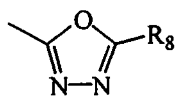
(a-2)

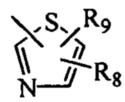
(a-3)

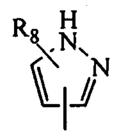
(a-4)

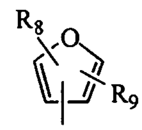
(a-5)

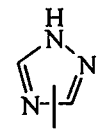
(a-6)

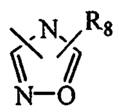
(a-7)

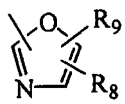
(a-8)

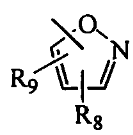
(a-9)

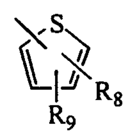
(a-10)

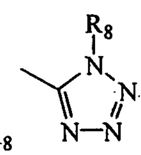
(a-11)

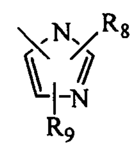
(a-12)

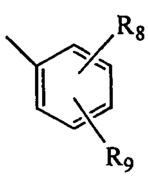
(a-13)

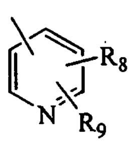
(a-14)

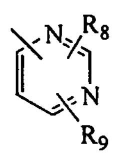
(a-15)

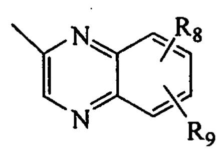
(a-16)

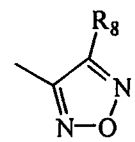
(a-17)

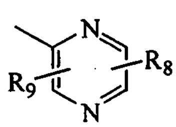
(a-18)

each $R_8$ independently represents hydrogen, halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl) amino, hydroxy$C_{1-6}$alkylamino, aryl, aryloxy, piperidinyl, piperidinylamino, morpholinyl, piperazinyl or nitro;

each $R_9$ independently represents hydrogen, halo or $C_{1-6}$alkyl;

n is 1, 2, 3, 4 or 5;
aryl represents phenyl or phenyl substituted with one, two, three, four or five substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono-or di($C_{1-4}$alkyl)aminocarbonyl, amino, mono-or di($C_{1-4}$alkyl)amino, phenyloxy or nitro;
heteroaryl represents furanyl, thienyl, pyrrolyl, oxazolyl, thiazolyl, imidazolyl, pyrazolyl, isoxazolyl, isothiazolyl, oxadiazolyl, triazolyl,

thiadiazolyl, pyridyl, pyridazinyl, pyrimidinyl, pyrazinyl, each of said heterocycles optionally being substituted with one or two substituents each independently selected from halo, $C_{1-6}$alkyl, $C_{1-6}$alkyloxy, polyhalo$C_{1-6}$alkyl, polyhalo$C_{1-6}$alkyloxy, cyano, aminocarbonyl, mono- or di($C_{1-4}$alkyl)aminocarbonyl, amino, mono-or di($C_{1-4}$alkyl) amino or nitro.

**2.** A compound according to claim 1 wherein $R_2$ represents halo.

**3.** A compound according to claim 1 or 2 wherein Z represents a cyclic ring system selected from (a-2), (a-3), (a-4), (a-5), (a-6), (a-7), (a-11), (a-13), (a-14) or (a-15).

**4.** A compound according to claim 3 wherein Z represents a cyclic ring system selected from (a-2) or (a-15).

**5.** A compound according to any one of the preceding claims wherein $R_3$ represents hydrogen, cyano, C(=O)-O-$R_5$, C(=O)-$NR_{6a}R_{6b}$, C(=S)-$NR_{6a}R_{6b}$, $S(=O)_2$-$NR_{6a}R_{6b}$ or C(=O)-$R_7$.

**6.** A compound according to any one of claims 1 to 4 wherein $R_3$ represents hydrogen, $C_{1-6}$alkyl substituted with $C_{1-6}$alkyloxy or C(=O)-O-$R_5$.

**7.** A compound according to claim 6 wherein $R_3$ represents hydrogen or C(=O)-O-$R_5$.

**8.** A compound according to any one of the preceding claims wherein n is 2.

**9.** A compound according to claim 1 wherein $R_1$ represents $C_{1-6}$alkyl; $R_2$ represents halo or polyhalo$C_{1-6}$alkyl; $R_3$ represents hydrogen, $C_{1-6}$alkyl substituted with $C_{1-6}$alkyloxy, or C(=O)-O-$R_5$; Z represents a cyclic ring system selected from (a-2), (a-3), (a-4), (a-5), (a-6), (a-7), (a-11), (a-13), (a-14) or (a-15); $R_4$ represents hydrogen; n represents 1,2 or 3.

**10.** A compound according to claim 1 wherein the compound is selected from

<table>
<tr><td colspan="3">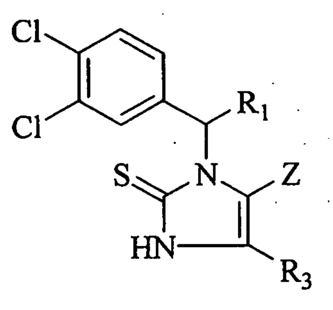</td></tr>
<tr><th>$R_1$</th><th>$R_3$</th><th>Z</th></tr>
<tr><td>$-CH_2CH_3$</td><td>H</td><td>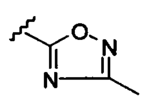</td></tr>
</table>

(continued)

| $R_1$ | $R_3$ | Z |
|---|---|---|
| $-CH_2CH_3$ | H | 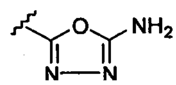 |
| $-CH_2CH_3$ | H | 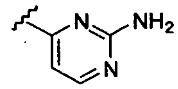 |
| $-CH_2CH_3$ | H | 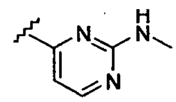 |
| $-CH_2CH_3$ | H | 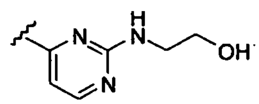 |

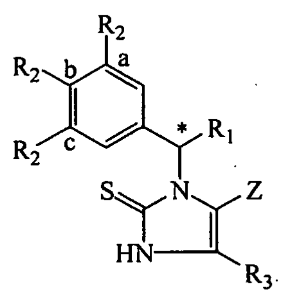

| R1 | a | R2 b | c | R3 | Z |
|---|---|---|---|---|---|
| $-CH_2CH_3$ | F | $-CF_3$ | H | H | 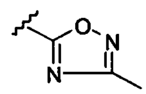 |

**11.** A compound according to any one of the preceding claims wherein the compound is stereochemically pure.

**12.** A compound as claimed in any one of the preceding claims for use as a medicine.

**13.** Use of a compound as claimed in any one of claims 1 to 11 for the manufacture of a medicament for preventing or treating diseases mediated through activation of the CCR2 receptor.

**14.** Use according to claim 13 wherein the disease is an inflammatory disease.

**15.** A pharmaceutical composition comprising a pharmaceutically acceptable carrier, and as active ingredient a therapeutically effective amount of a compound as claimed in any one of claims 1 to 11.

**16.** A process of preparing a composition as claimed in claim 15 **characterized in that** a pharmaceutically acceptable carrier is intimately mixed with a therapeutically effective amount of a compound as claimed in any one of claims 1 to 11.

**17.** A process of preparing a compound as defined in claim 1 **characterized by**

a) reacting an intermediate of formula (II) with an appropriate acid optionally in the presence of a suitable solvent

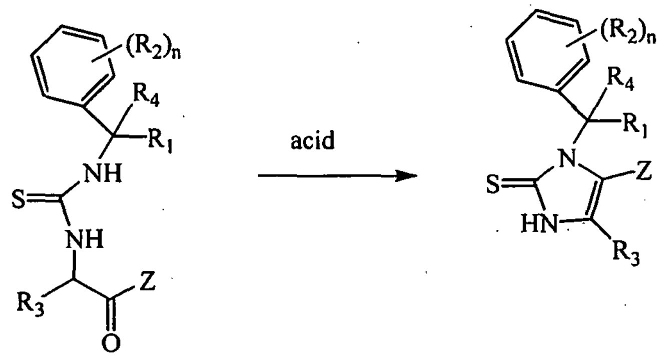


(II) (I)

with $R_1$, $R_2$, $R_3$, $R_4$, Z and n as defined in claim 1;
b) reacting an intermediate of formula (III) with an intermediate of formula (IV) in the presence of a suitable solvent

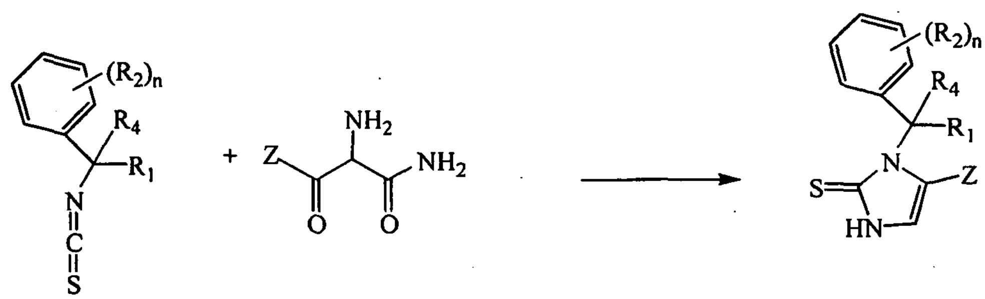

(III) (IV) (I-a)

with $R_1$, $R_2$, $R_4$, Z and n as defined in claim 1;
c) reacting an intermediate of formula (V) with a suitable acid

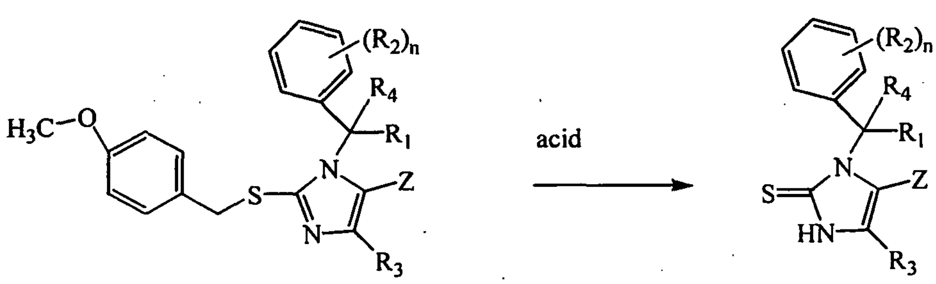


(V) (I)

with $R_1$, $R_2$, $R_3$, $R_4$, Z and n as defined in claim 1;
d) reacting an intermediate of formula (VI) with phosphoric trichloride ($POCl_3$) or Burgess'reagent in the presence of a suitable solvent

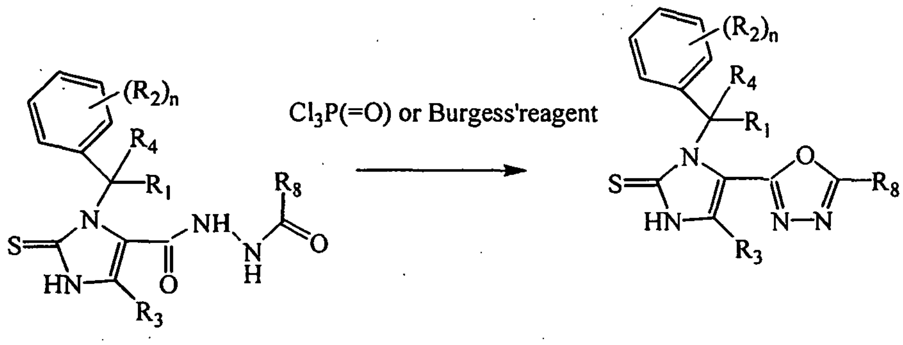


(VI) (I-b)

with $R_1$, $R_2$, $R_3$, $R_4$, $R_8$ and n as defined in claim 1;

e)reacting an intermediate of formula (VII) with $SOCl_2$ and $HC(=O)NH-NH_2$ in the presence of a suitable solvent

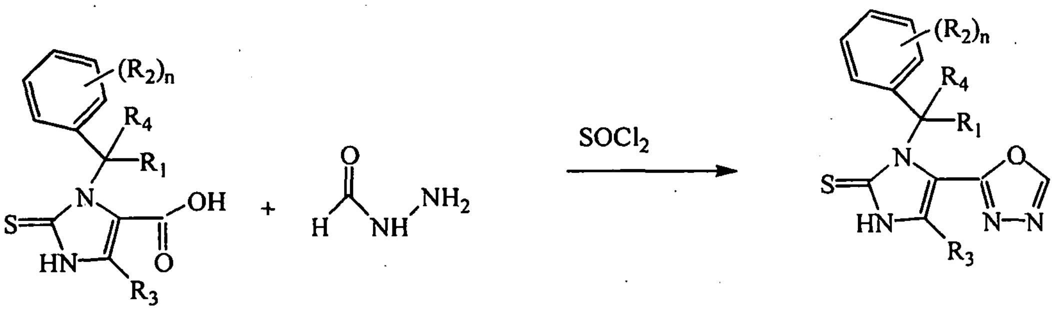


(VII) (I-b-1)

with $R_1$, $R_2$, $R_3$, $R_4$ and n as defined in claim 1;

f) reacting an intermediate of formula (VIII) with an intermediate of formula (IX) in the presence of a suitable base and a suitable solvent

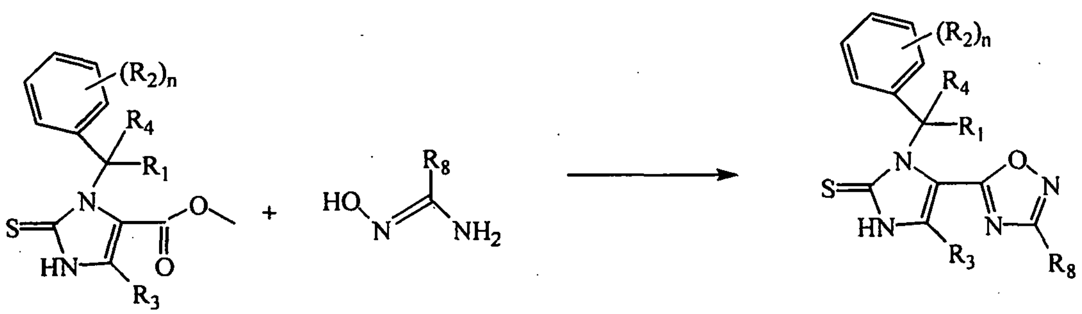

(VIII) (IX) (I-c)

with $R_1$, $R_2$, $R_3$, $R_4$, $R_8$ and n as defined in claim 1;

g) reacting an intermediate of formula (X) with methyl formate, KSCN in the presence of a suitable base, a suitable acid and a suitable solvent, followed by reacting the thus obtained intermediate of formula (X-a) with $Bu_3SnN_3$ in the presence of a suitable solvent

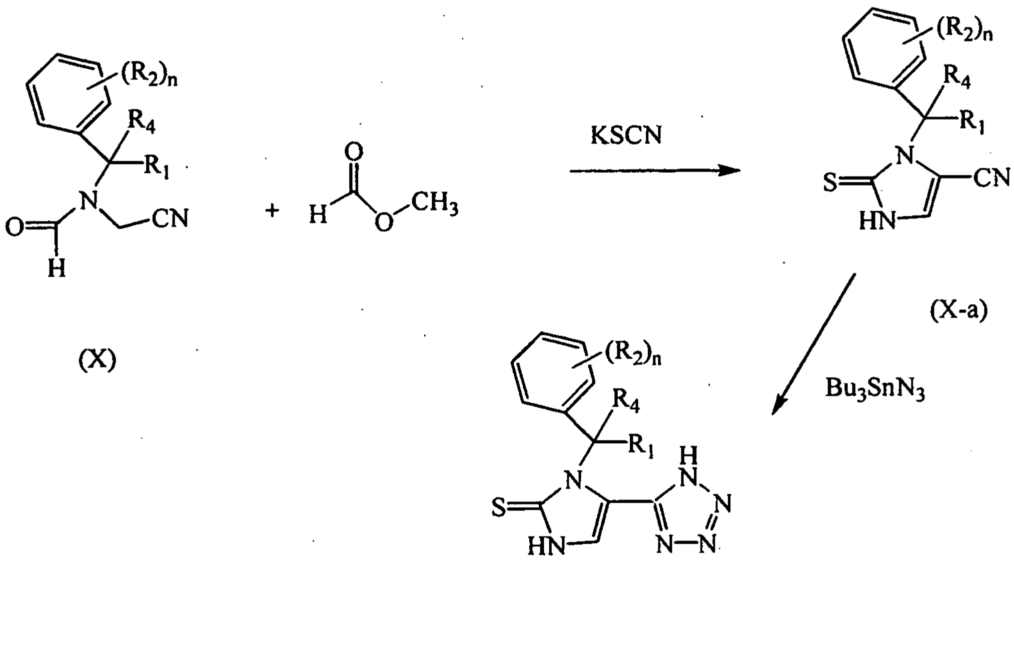


(X)

(X-a)

(I-a-1)

with $R_1$, $R_2$, $R_4$ and n as defined in claim 1;
or, if desired, converting compounds of formula (I) into each other following art-known transformations, and further, if desired, converting the compounds of formula (I), into a therapeutically active non-toxic acid addition salt by treatment with an acid, or into a therapeutically active non-toxic base addition salt by treatment with a base, or conversely, converting the acid addition salt form into the free base by treatment with alkali, or converting the base addition salt into the free acid by treatment with acid; and, if desired, preparing stereochemically isomeric forms, quaternary amines or *N*-oxide forms thereof.

**Patentansprüche**

**1.** Verbindung der Formel (I)

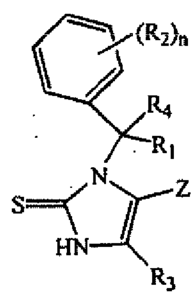

(I)

,

ein N-Oxid, ein pharmazeutisch annehmbares Additionssalz, ein quaternäres Amin oder eine stereochemisch isomere Form davon, wobei

$R_1$ für $C_{1-6}$-Alkyl steht;
$R_2$ jeweils unabhängig voneinander für Halogen oder Polyhalogen-$C_{1-6}$-alkyl steht;
$R_3$ für Wasserstoff, Cyano, gegebenenfalls durch Hydroxy oder $C_{1-6}$-Alkyloxy substituiertes $C_{1-6}$-Alkyl, C(=O)-O-$R_5$, C (=O)-$NR_{6a}R_{6b}$, C(=S)-$NR_{6a}R_{6b}$, $S(=O)_2$-$NR_{6a}R_{6b}$ oder C(=O)-$R_7$ steht;

$R_4$ für Wasserstoff steht;

$R_5$ für Wasserstoff, $C_{1-6}$-Alkyl, Hydroxy-$C_{1-6}$-alkyl, $C_{2-6}$-Alkenyl, $C_{2-6}$-Alkinyl, Polyhalogen-$C_{1-6}$-alkyl, $C_{1-6}$-Alkyloxy-$C_{1-6}$-alkyl, Amino-$C_{1-6}$-alkyl, Mono- oder Di($C_{1-4}$-alkyl)amino-$C_{1-6}$-alkyl, Aminocarbonyl-$C_{1-6}$-alkyl, Mono- oder Di($C_{1-4}$-alkyl)aminocarbonyl-$C_{1-6}$-alkyl oder Aryl steht;

$R_{6a}$ und $R_{6b}$ jeweils unabhängig voneinander für Wasserstoff, $C_{1-6}$-Alkyl, Amino, Mono- oder Di($C_{1-4}$-alkyl) amino, ArylNH-, Amino-$C_{1-6}$-alkyl, Mono- oder Di ($C_{1-4}$-alkyl) amino-$C_{1-6}$-alkyl, $C_{1-6}$-Alkylcarbonylamino, Aminocarbonylamino, $C_{1-6}$-Alkyloxy, Carbonylamino oder Hydroxy-$C_{1-6}$-alkyl stehen; oder

$R_{6a}$ und $R_{6b}$ zusammen mit dem Stickstoff, an den sie gebunden sind, Pyrrolidinyl, Imidazolidinyl, Pyrazolidinyl, Piperidinyl, Piperazinyl, Morpholinyl, Thiomorpholinyl oder Piperazinyl, substituiert durch $C_{1-6}$-Alkyl, bilden;

$R_7$ für Wasserstoff, $C_{1-6}$-Alkyl, Hydroxy-$C_{1-6}$-alkyl, $C_{2-6}$-Alkenyl, $C_{2-6}$-Alkinyl, Polyhalogen-$C_{1-6}$-alkyl, $C_{1-6}$-Alkyloxy-$C_{1-6}$-alkyl, Amino-$C_{1-6}$-alkyl, Mono- oder Di($C_{1-4}$-alkyl)amino-$C_{1-6}$-alkyl, Aminocarbonyl-$C_{1-6}$-alkyl, Mono- oder Di($C_{1-4}$-alkyl)aminocarbonyl-$C_{1-6}$-alkyl, Aryl oder Heteroaryl steht;

Z für ein unter

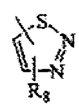
(a-1)

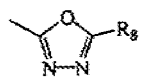
(a-2)

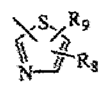
(a-3)

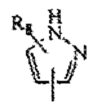
(a-4)

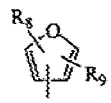
(a-5)

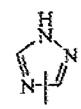
(a-6)

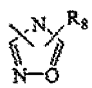
(a-7)

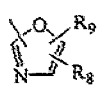
(a-8)

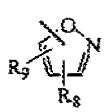
(a-9)

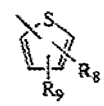
(a-10)

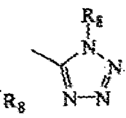
(a-11)

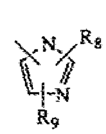
(a-12)

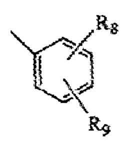
(a-13)

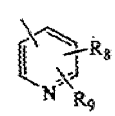
(a-14)

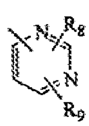
(a-15)

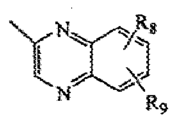
(a-16)

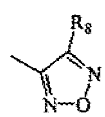
(a-17)

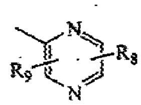
(a-18)

ausgewähltes cyclisches Ringsystem steht;

$R_8$ jeweils unabhängig voneinander für Wasserstoff, Halogen, $C_{1-6}$-Alkyl, $C_{1-6}$-Alkyloxy, Polyhalogen-$C_{1-6}$-alkyl, Polyhalogen-$C_{1-6}$-alkyloxy, Cyano, Aminocarbonyl, Mono- oder Di($C_{1-4}$-alkyl)aminocarbonyl, Amino, Mono- oder Di($C_{1-4}$-alkyl)amino, Hydroxy-$C_{1-6}$-alkylamino, Aryl, Aryloxy, Piperidinyl, Piperidinylamino, Morpholinyl, Piperazinyl oder Nitro steht;

$R_9$ jeweils unabhängig voneinander für Wasserstoff, Halogen oder $C_{1-6}$-Alkyl steht;

n für 1, 2, 3, 4 oder 5 steht;

Aryl für Phenyl oder durch einen, zwei, drei, vier oder fünf Substituenten, die jeweils unabhängig voneinander unter Halogen, $C_{1-6}$-Alkyl, $C_{1-6}$-Alkyloxy, Polyhalogen-$C_{1-6}$-alkyl, Polyhalogen-$C_{1-6}$-alkyloxy, Cyano, Aminocarbonyl, Mono- oder Di($C_{1-4}$-alkyl)aminocarbonyl, Amino, Mono- oder Di($C_{1-4}$-alkyl)amino, Phenyloxy oder Nitro ausgewählt sind, substituiertes Phenyl steht;

Heteroaryl für Furanyl, Thienyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, Pyridyl, Pyridazinyl, Pyrimidinyl oder Pyrazinyl steht, wobei jeder der Heterocyclen gegebenenfalls durch einen oder zwei Substituenten, die jeweils unabhängig voneinander unter Halogen, $C_{1-6}$-Alkyl, $C_{1-6}$-Alkyloxy, Polyhalogen-$C_{1-6}$-alkyl, Polyhalogen-$C_{1-6}$-alkyloxy, Cyano, Aminocarbonyl, Mono- oder Di($C_{1-4}$-alkyl)aminocarbonyl, Amino, Mono- oder Di($C_{1-4}$-alkyl)amino oder Nitro ausgewählt sind, substituiert ist.

**2.** Verbindung nach Anspruch 1, in der $R_2$ für Halogen steht.

**3.** Verbindung nach Anspruch 1 oder 2, in der Z für ein unter (a-2), (a-3), (a-4), (a-5), (a-6), (a-7), (a-11), (a-13), (a-14) oder (a-15) ausgewähltes cyclisches Ringsystem steht.

**4.** Verbindung nach Anspruch 3, in der Z für ein unter (a-2) oder (a-15) ausgewähltes cyclisches Ringsystem steht.

**5.** Verbindung nach einem der vorhergehenden Ansprüche, in der $R_3$ für Wasserstoff, Cyano, C(=O)-O-$R_5$, C(=O)-$NR_{6a}P_{6b}$, C(=S)-$NR_{6a}P_{6b}$, $S(=O)_2$-$NR_{6a}P_{6b}$ oder C(=O)-$R_7$ steht.

**6.** Verbindung nach einem der Ansprüche 1 bis 4, in der $R_3$ für Wasserstoff, durch $C_{1-6}$-Alkyloxy substituiertes $C_{1-6}$-Alkyl oder C(=O)-O-$R_5$ steht.

**7.** Verbindung nach Anspruch 6, in der $R_3$ für Wasserstoff oder C(=O)-O-$R_5$ steht.

**8.** Verbindung nach einem der vorhergehenden Ansprüche, in der n für 2 steht.

**9.** Verbindung nach Anspruch 1, in der $R_1$ für $C_{1-6}$-Alkyl steht; $R_2$ für Halogen oder Polyhalogen-$C_{1-6}$-alkyl steht; $R_3$ für Wasserstoff, durch $C_{1-6}$-Alkyloxy substituiertes $C_{1-6}$-Alkyl oder C(=O)-O-$R_5$ steht; Z für ein unter (a-2), (a-3), (a-4), (a-5), (a-6), (a-7), (a-11), (a-13), (a-14) oder (a-15) ausgewähltes cyclisches Ringsystem steht; $R_4$ für Wasserstoff steht und n für 1, 2 oder 3 steht.

**10.** Verbindung nach Anspruch 1, ausgewählt unter

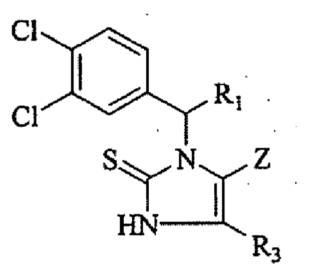

| $R_1$ | $R_2$ | |
|---|---|---|
| $-CH_2CH_3$ | H | 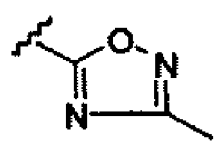 |
| $-CH_2CH_3$ | H | 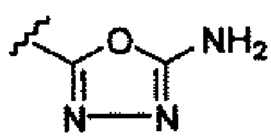 |

(fortgesetzt)

| $R_1$ | $R_2$ | |
|---|---|---|
| $-CH_2CH_3$ | H | 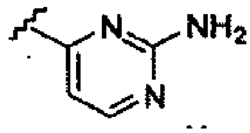 |
| $-CH_2CH_3$ | H | 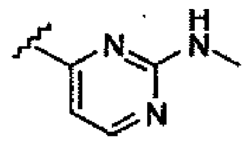 |
| $-CH_2CH_3$ | H | 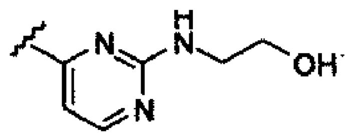 |

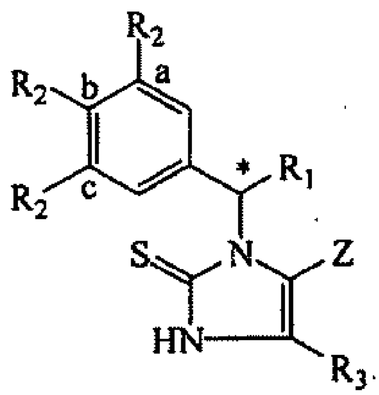

| R1 | | R2 | | R3 | Z |
|---|---|---|---|---|---|
| | a | b | c | | |
| $-CH_2CH_3$ | F | $-CF_3$ | H | H | 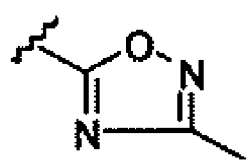 |

**11.** Verbindung nach einem der vorhergehenden Ansprüche, die stereochemisch rein ist.

**12.** Verbindung nach einem der vorhergehenden Ansprüche zur Verwendung als Medizin.

**13.** Verwendung einer Verbindung nach einem der Ansprüche 1 bis 11 zur Herstellung eines Arzneimittels zur Prävention oder Behandlung von Krankheiten, die durch Aktivierung des CCR2-Rezeptors vermittelt werden.

**14.** Verwendung nach Anspruch 13, bei der es sich bei der Krankheit um eine Entzündungskrankheit handelt.

**15.** Pharmazeutische Zusammensetzung, enthaltend einen pharmazeutisch unbedenklichen Träger und als Wirkstoff eine therapeutisch wirksame Menge einer Verbindung nach einem der Ansprüche 1 bis 11.

**16.** Verfahren zur Herstellung einer Zusammensetzung nach Anspruch 15, **dadurch gekennzeichnet, daß** man einen pharmazeutisch unbedenklichen Träger innig mit einer therapeutisch wirksamen Menge einer Verbindung nach einem der Ansprüche 1 bis 11 vermischt.

**17.** Verfahren zur Herstellung einer Verbindung gemäß Anspruch 1, **dadurch gekennzeichnet, daß** man

a) ein Zwischenprodukt der Formel (II) mit einer geeigneten Säure umsetzt, gegebenenfalls in Gegenwart eines geeigneten Lösungsmittels

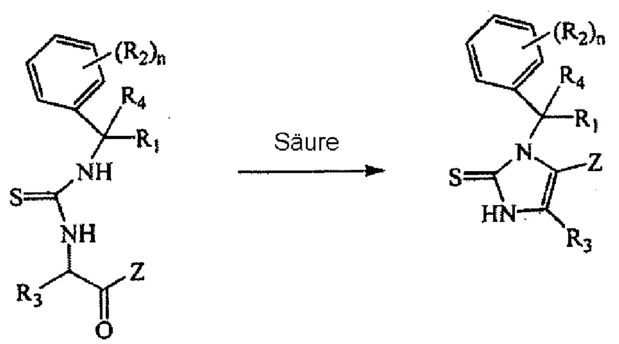


(II) (I)

wobei $R_1$, $R_2$, $R_3$, $R_4$, Z und n die in Anspruch 1 angegebene Bedeutung besitzen;
b) ein Zwischenprodukt der Formel (III) in Gegenwiart eines geeigneten Lösungsmittels mit einem Zwischenprodukt der Formel (IV) umsetzt

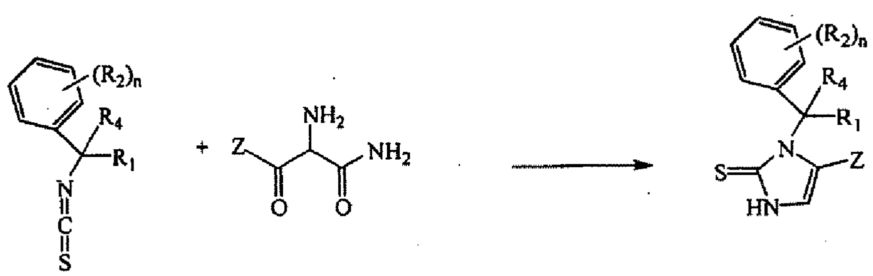

(III) (IV) (I-a)

wobei $R_1$, $R_2$, $R_4$, Z und n die in Anspruch 1 angegebene Bedeutung besitzen;
c) ein Zwischenprodukt der Formel (V) mit einer geeigneten Säure umsetzt

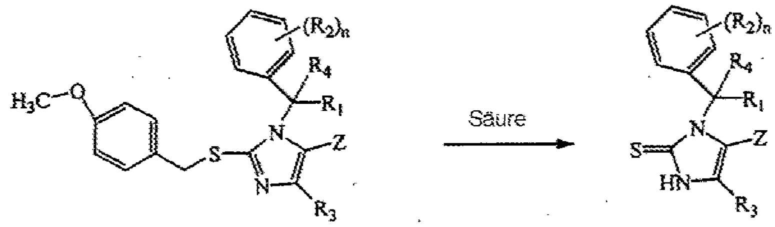


(V) (I)

wobei $R_1$, $R_2$, $R_3$, $R_4$, Z und n die in Anspruch 1 angegebene Bedeutung besitzen;

d) ein Zwischenprodukt der Formel (VI) in Gegenwart eines geeigneten Lösungsmittels mit Phosphoryltrichlorid ($POCl_3$) oder Burgess-Reagenz umsetzt

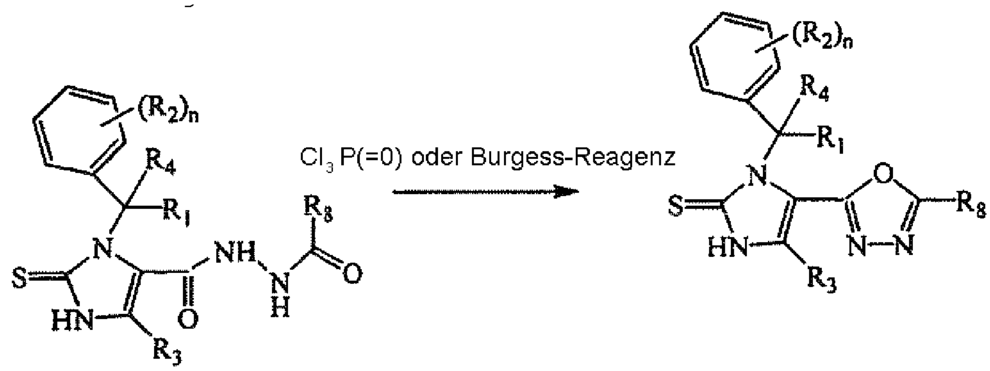


(VI) (I-b)

wobei $R_1$, $R_2$, $R_3$, $R_4$, $R_8$ und n die in Anspruch 1 angegebene Bedeutung besitzen;
e) ein Zwischenprodukt der Formel (VII) in Gegenwart eines geeigneten Lösungsmittels mit $SOCl_2$ und HC(=O) $NH-NH_2$ umsetzt

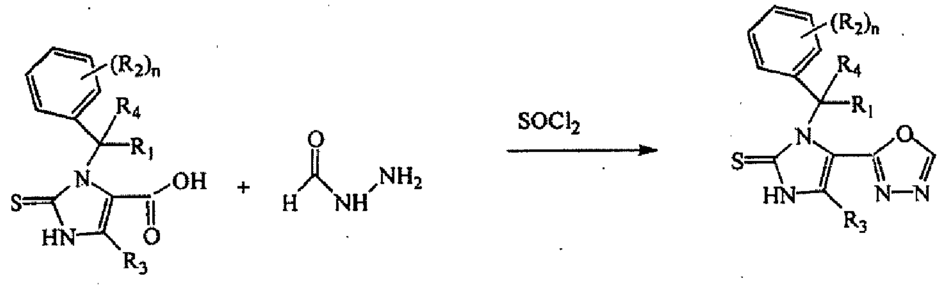


(VII) (I-b-1)

wobei $R_1$, $R_2$, $R_3$, $R_4$ und n die in Anspruch 1 angegebene Bedeutung besitzen;
f) ein Zwischenprodukt der Formel (VIII) in Gegenwart einer geeigneten Base und eines geeigneten Lösungsmittels mit einem Zwischenprodukt der Formel (IX) umsetzt

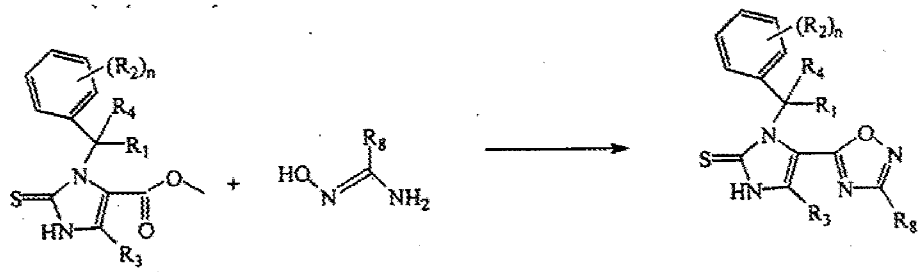

(VIII) (IX) (I-c)

wobei $R_1$, $R_2$, $R_3$, $R_4$, $R_8$ und n die in Anspruch 1 angegebene Bedeutung besitzen;

g) ein Zwischenprodukt der Formel (X) in Gegenwart einer geeigneten Base, einer geeigneten Säure und eines geeigneten Lösungsmittels mit Ameisensäuremethylester und KSCN umsetzt und danach das so erhaltene Zwischenprodukt der Formel (X-a) in Gegenwart eines geeigneten Lösungsmittels mit $Bu_3SnN_3$ umsetzt

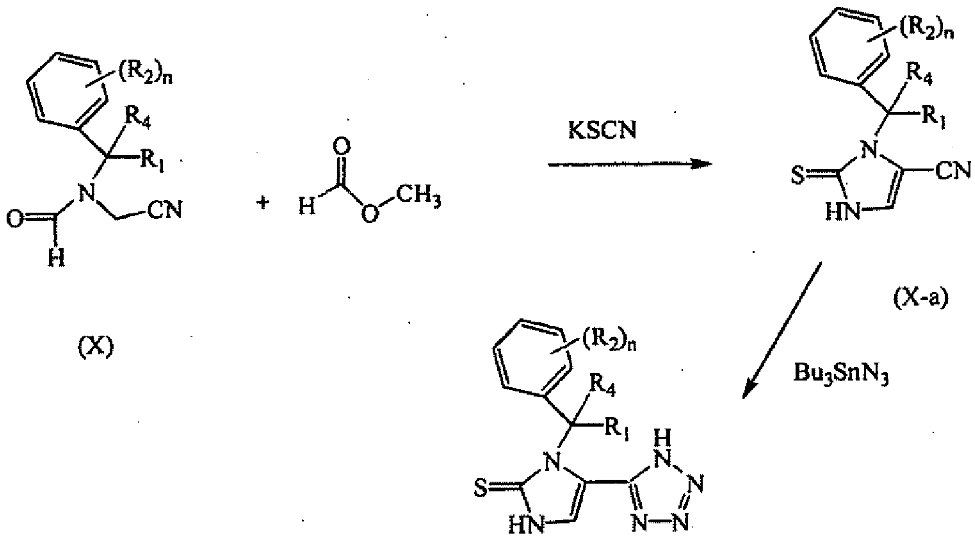


(X) (X-a)

(I-a-1)

wobei R1, R2, R4 und in die in Anspruch 1 angegebene Bedeutung besitzen;
oder gegebenenfalls Verbindungen der Formel (I) durch an sich bekannte Transformationen ineinander umwandelt und weiterhin gegebenenfalls die Verbindungen der Formel (I) durch Behandlung mit einer Säure in ein therapeutisch wirksames nichttoxisches Säureadditionssalz oder durch Behandlung mit Base in ein therapeutisch wirksames nichttoxisches Basenadditionssalz umwandelt oder umgekehrt die Säureadditionssalzform durch Behandlung mit Alkali in die freie Base oder das Basenadditionssalz durch Behandlung mit Säure in die freie Säure umwandelt und gegebenenfalls stereochemisch isomere Formen, quaternäre Amine oder N-Oxidformen davon herstellt.

**Revendications**

1. Composé de la formule

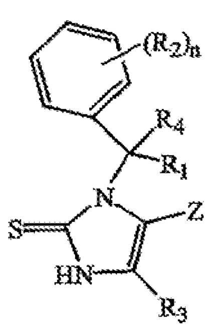

(I)

un N-oxyde, un sel d'addition pharmaceutiquement acceptable, une amine quaternaire ou une forme stéréochimiquement isomère de ceux-ci, dans laquelle

$R_1$ représente un groupe alkyle de $C_1$ à $C_6$ ;

chaque $R_2$ représente indépendamment un groupe halo ou un groupe polyhaloalkyle de $C_1$ à $C_6$ ;
$R_3$ représente un atome d'hydrogène, un groupe cyano, un groupe alkyle de $C_1$ à $C_6$ éventuellement substitué par un groupe hydroxy ou un groupe alkyloxy de $C_1$ à $C_6$, un groupe C(=O)-O-$R_5$, un groupe C(=O)-$NR_{6a}R_{6b}$, un groupe C (=S) -$NR_{6a}R_{6b}$, un groupe S (=O)2-$NR_{6a}R_{6b}$ ou un groupe C(=O)-$R_7$ ;
$R_4$ représente un atome d'hydrogène ;
$R_5$ représente un atome d'hydrogène, un groupe alkyle de $C_1$ à $C_6$, un groupe hydroxyalkyle de $C_1$ à $C_6$, un groupe alcényle de $C_2$ à $C_6$, un groupe alcynyle de $C_2$ à $C_6$, un groupe polyhaloalkyle de $C_1$ à $C_6$, un groupe alkyloxy de $C_1$ à $C_6$-alkyle de $C_1$ à $C_6$, un groupe aminoalkyle de $C_1$ à $C_6$, un groupe mono- ou di (alkyle de $C_1$ à $C_4$) aminoalkyle de $C_1$ à $C_6$, un groupe aminocarbonylalkyle de $C_1$ à $C_6$, un groupe mono- ou di(alkyle de $C_1$ à $C_4$)aminocarbonylalkyle de $C_1$ à $C_6$ ou un groupe aryle ;
$R_{6a}$ et $R_{6b}$ représentent chacun indépendamment un atome d'hydrogène, un groupe alkyle de $C_1$ à $C_6$, un groupe amino, un groupe mono- ou di (alkyle de $C_1$ à $C_4$)amino, un groupe arylNH-, un groupe aminoalkyle de $C_1$ à $C_6$, un groupe mono- ou di (alkyle de $C_1$ à $C_4$) aminoalkyle de $C_1$ à $C_6$, un groupe alkyle de $C_1$ à $C_6$-carbonylamino, un groupe aminocarbonylamino, un groupe alkyloxy de $C_1$ à $C_6$, un groupe carbonylamino ou un groupe hydroxyalkyle de $C_1$ à $C_6$ ; ou
$R_{6a}$ et $R_{6b}$ pris ensemble avec l'atome d'hydrogène auquel ils sont attachés forment un pyrrolidinyle, un imidazolidinyle, un pyrazolidinyle, un pipéridinyle, un pipérazinyle, un morpholinyle, un thiomorpholinyle ou un pipérazinyle substitué par un groupe alkyle de $C_1$ à $C_6$ ;
$R_7$ représente un atome d'hydrogène, un groupe alkyle de $C_1$ à $C_6$, un groupe hydroxyalkyle de $C_1$ à $C_6$, un groupe alcényle de $C_2$ à $C_6$, un groupe alcynyle de $C_2$ à $C_6$, un groupe polyhaloalkyle de $C_1$ à $C_6$, un groupe alcoxy de $C_1$ à $C_6$-alkyle de $C_1$ à $C_6$, un groupe aminoalkyle de $C_1$ à $C_6$, un groupe mono- ou di(alkyle de $C_1$ à $C_4$)aminoalkyle de $C_1$ à $C_6$, un groupe aminocarbonylalkyle de $C_1$ à $C_6$, un groupe mono- ou di (alkyle de $C_1$ à $C_4$)aminocarbonylalkyle de $C_1$ à $C_6$, un groupe aryle ou un groupe hétéroaryle ;
Z représente un système à anneau cyclique choisi parmi

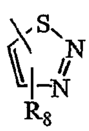
(a-1)

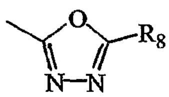
(a-2)

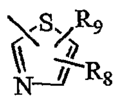
(a-3)

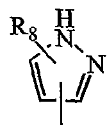
(a-4)

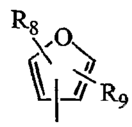
(a-5)

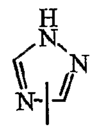
(a-6)

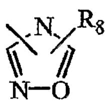
(a-7)

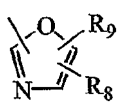
(a-8)

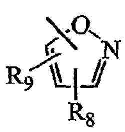
(a-9)

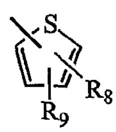
(a-10)

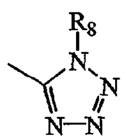
(a-11)

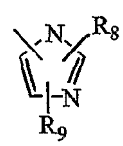
(a-12)

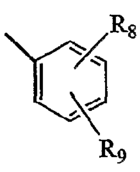
(a-13)

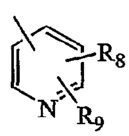
(a-14)

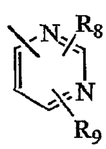
(a-15)

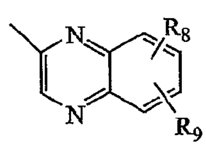
(a-16)

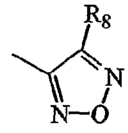
(a-17)

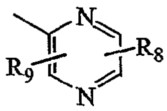

(a-18)

chaque $R_8$ représente indépendamment un atome d'hydrogène, un groupe halo, un groupe alkyle de $C_1$ à $C_6$, un groupe alkyloxy de $C_1$ à $C_6$, un groupe polyhaloalkyle de $C_1$ à $C_6$, un groupe polyhaloalkyloxy de $C_1$ à $C_6$, un groupe cyano, un groupe aminocarbonyle, un groupe mono- ou di(alkyle de $C_1$ à $C_4$)aminocarbonyle, un groupe amino, un groupe mono- ou di(alkyle de $C_1$ à $C_4$)amino, un groupe hydroxyalkylamino de $C_1$ à $C_6$, un groupe aryle, un groupe aryloxy, un groupe pipéridinyle, un groupe pipéridinylamino, un groupe morpholinyle, un groupe piérazinyle ou un groupe nitro ;
chaque $R_9$ représente indépendamment un atome d'hydrogène, un groupe halo ou un groupe alkyle de $C_1$ à $C_6$ ;
n vaut 1, 2, 3, 4 ou 5 ;
un groupe aryle représente un groupe phényle ou un groupe phényle substitué par un, deux, trois, quatre ou cinq substituants, chacun indépendamment choisi parmi un groupe halo, un groupe alkyle de $C_1$ à $C_6$, un groupe alkyloxy de $C_1$ à $C_6$, un groupe polyhaloalkyle de $C_1$ à $C_6$, un groupe polyhaloalkyloxy de $C_1$ à $C_6$, un groupe cyano, un groupe aminocarbonyle, un groupe mono- ou
di (alkyle de $C_1$ à $C_4$)aminocarbonyle, un groupe amino, un groupe mono- ou di(alkyle de $C_1$ à $C_4$)amino, un groupe phényloxy ou un groupe nitro ;
un groupe hétéroaryle représente un groupe furanyle, thiényle, pyrrolyle, oxazolyle, thiazolyle, imidazolyle, pyrazolyle, isoxazolyle, isothiazolyle, oxadiazolyle, triazolyle, thiadiazolyle, pyridyle, pyridazinyle, pyrimidinyle ou pyrazinyle, chacun desdits hétérocycles étant éventuellement substitué par un ou deux substituants chacun indépendamment choisi parmi un groupe halo, un groupe alkyle de $C_1$ à $C_6$, un groupe alkyloxy de $C_1$ à $C_6$, un groupe polyhaloalkyle de $C_1$ à $C_6$, un groupe polyhaloalkyloxy de $C_1$ à $C_6$, un groupe cyano, un groupe aminocarbonyle, un groupe mono- ou di(alkyle de $C_1$ à $C_4$)aminocarbonyle, un groupe amino, un groupe mono- ou di(alkyle de $C_1$ à $C_4$)amino ou un groupe nitro ;

2. Composé selon la revendication 1, dans lequel $R_2$ représente un groupe halo.

3. Composé selon la revendication 1 ou 2, dans lequel Z représente un système à anneau cyclique choisi par les systèmes (a-2), (a-3), (a-4), (a-5), (a-6), (a-7), (a-11), (a-13), (a-14) ou (a-15)

4. Composé selon la revendication 3, dans lequel Z représente un système à anneau cyclique choisi parmi les systèmes (a-2) et (a-15).

5. Composé selon l'une quelconque des revendications précédentes, dans lequel $R_3$ représente un atome d'hydrogène, un groupe cyano, un groupe C(=O)-$OR_5$, un groupe C(=O)-$NR_{6a}R_{6b}$, un groupe C(=S)-$NR_{6a}R_{6b}$, un groupe S(=O)$_2$-$NR_{6a}R_{6b}$ ou un groupe C(=O)-$R_7$.

6. Composé selon l'une quelconque des revendications de 1 à 4, dans lequel $R_3$ représente un atome d'hydrogène, un groupe alkyle de $C_1$ à $C_6$ substitué par un groupe alkyloxy de $C_1$ à $C_6$ ou un groupe C(=O)-$OR_5$.

7. Composé selon la revendication 6, dans lequel $R_3$ représente un atome d'hydrogène ou un groupe C(=O)-O-$R_5$.

8. Composé selon l'une quelconque des revendications précédentes, dans lequel n vaut 2.

9. Composé selon la revendication 1, dans lequel $R_1$ représente un groupe alkyle de $C_1$ à $C_6$ ; $R_2$ représente un groupe halo ou un groupe polyhaloalkyle de $C_1$ à $C_6$ ; $R_3$ représente un atome d'hydrogène, un groupe alkyle de $C_1$ à $C_6$ substitué par un groupe alkyloxy de $C_1$ à $C_6$, ou un groupe C(=O)-O-$R_5$ ; Z représente un système à anneau cyclique choisi par les systèmes (a-2), (a-3), (a-4), (a-5), (a-6), (a-7), (a-11), (a-13), (a-14) ou (a-15) ; $R_4$ représente un atome d'hydrogène ; n vaut 1, 2 ou 3.

10. Composé selon la revendication 1, dans lequel le composé est choisi parmi

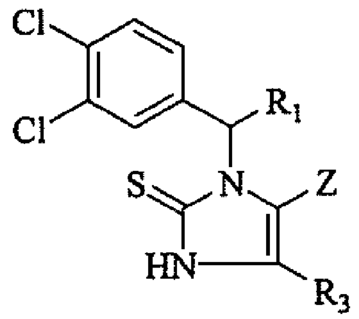

| $R_1$ | $R_3$ | z |
|---|---|---|
| $-CH_2CH_3$ | H | 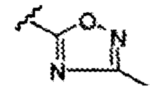 |
| $-CH_2CH_3$ | H | 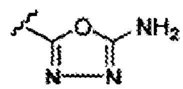 |
| $-CH_2CH_3$ | H | 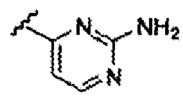 |
| $-CH_2CH_3$ | H | 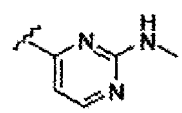 |
| $-CH_2CH_3$ | H | 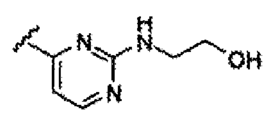 |

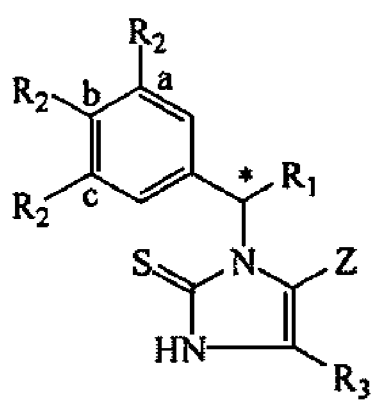

(suite)

| R1 | a b c | R2 R3 | | | Z |
|---|---|---|---|---|---|
| $-CH_2CH_3$ | F | $-CF_3$ | H | H | 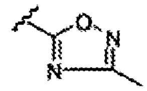 |

**11.** Composé selon l'une quelconque des revendications précédentes, dans lequel le composé est stéréochimiquement pur.

**12.** Composé selon l'une quelconque des revendications précédentes pour utilisation comme médicament.

**13.** Utilisation d'un composé selon l'une quelconque des revendications 1 à 11 pour la fabrication d'un médicament utilisé pour la prévention ou le traitement de maladies induites par une activation du récepteur CCR2.

**14.** Utilisation selon la revendication 13, dans laquelle la maladie est une maladie inflammatoire.

**15.** Composition pharmaceutique comprenant un vecteur pharmaceutiquement acceptable et, comme ingrédient actif, une quantité thérapeutiquement efficace d'un composé selon l'une quelconque des revendications 1 à 11.

**16.** Procédé de préparation d'une composition selon la revendication 15, **caractérisé en ce qu'**un vecteur pharmaceutiquement acceptable est intimement mélangé avec une quantité thérapeutiquement efficace d'un composé selon l'une quelconque des revendications 1 à 11.

**17.** Procédé de préparation d'un composé selon la revendication 1, **caractérisé par**

a) la réaction d'un intermédiaire de la formule (II) avec un acide approprié éventuellement en présence d'un solvant approprié

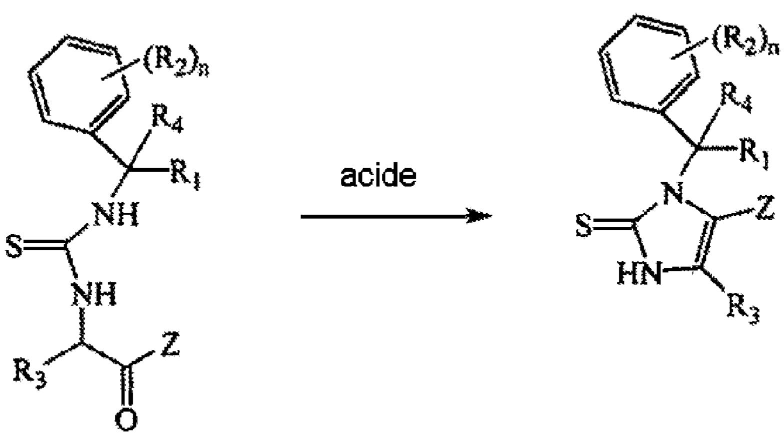


(I)

(II)

avec $R_1$, $R_2$, $R_3$, $R_4$, Z et n tels que définis dans la revendication 1 ;
b) la réaction d'un intermédiaire de la formule (III) avec un intermédiaire de la formule (IV) en présence d'un solvant approprié

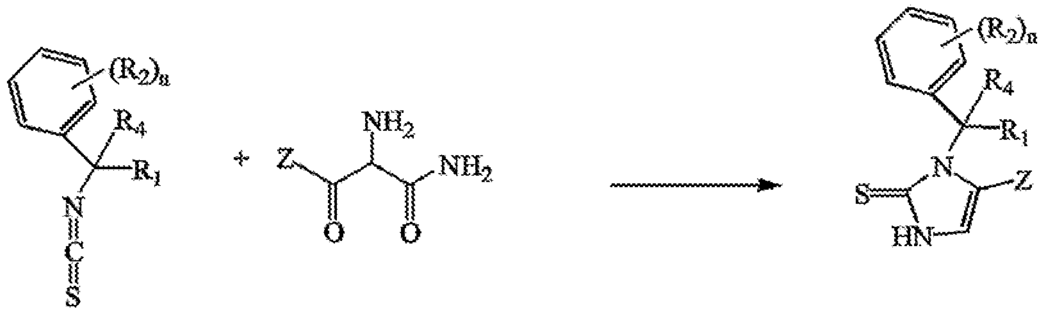

(III) (IV) (I-a)

avec $R_1$, $R_2$, $R_4$, Z et n tels que définis dans la revendication 1 ;
c) la réaction d'un intermédiaire de la formule (V) avec un acide approprié

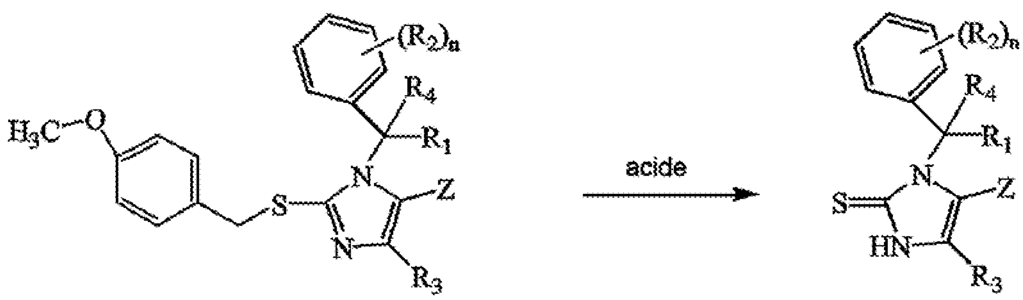


(V) (I)

avec $R_1$, $R_2$, $R_3$, $R_4$, Z et n tels que définis dans la revendication 1 ;
d) la réaction d'un intermédiaire de la formule (VI) avec du trichlorure de phosphore ($POCl_3$) ou un réactif de Burgess en présence d'un solvant approprié

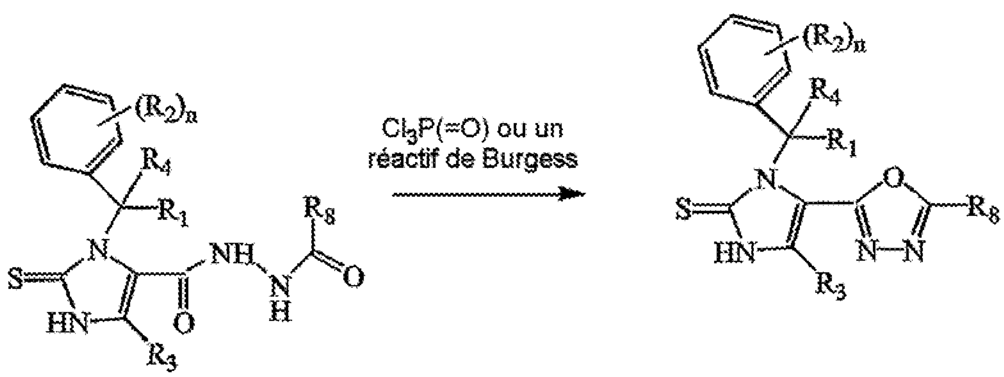


(VI) (I-b)

avec $R_1$, $R_2$, $R_3$, $R_4$, $R_8$ et n tels que définis dans la revendication 1 ;
e) la réaction d'un intermédiaire de la formule (VII) avec du $SOCl_2$ et du $HC(=O)NH-NH_2$ en présence d'un

solvant approprié

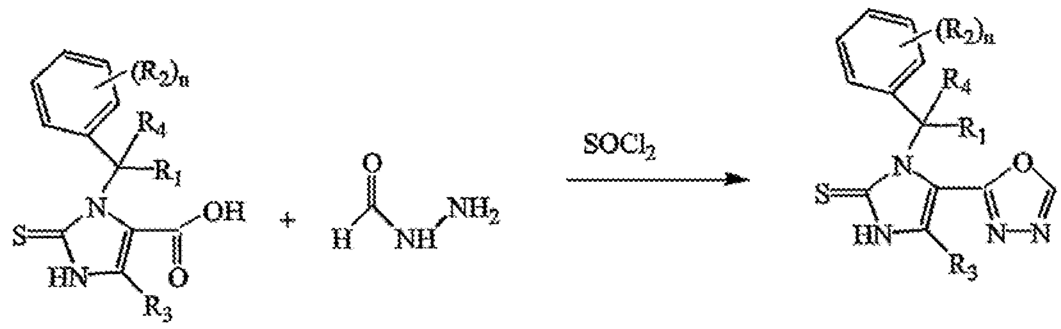


(VII) (I-b-1)

avec $R_1$, $R_2$, $R_3$, $R_4$ et n tels que définis dans la revendication 1 ;

f) la réaction d'un intermédiaire de la formule (VIII) avec un intermédiaire de la formule (IX) en présence d'une base appropriée et d'un solvant approprié

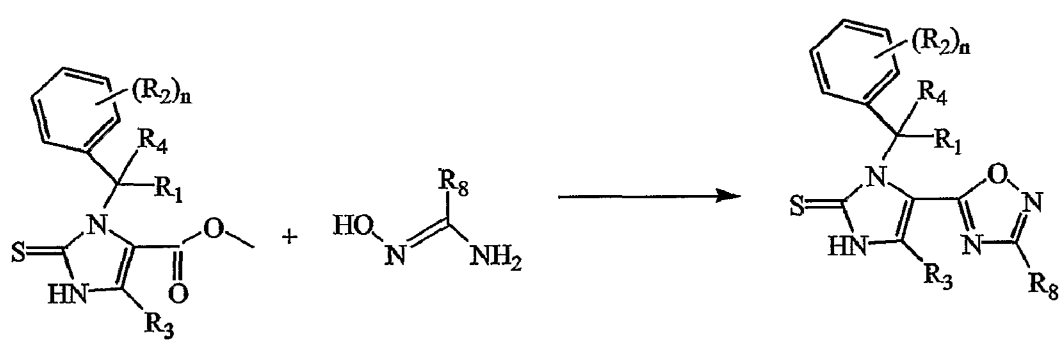

(VIII) (IX) (I-c)

avec $R_1$, $R_2$, $R_3$, $R_4$, $R_8$ et n tels que définis dans la revendication 1 ;

g) la réaction d'un intermédiaire de la formule (X) avec du formate de méthyle, du KSCN en présence d'une base appropriée, un acide approprié et un solvant approprié, suivie de la réaction de l'intermédiaire ainsi obtenu de la formule (X-a) avec du $Bu_3SnN_3$ en présence d'un solvant approprié

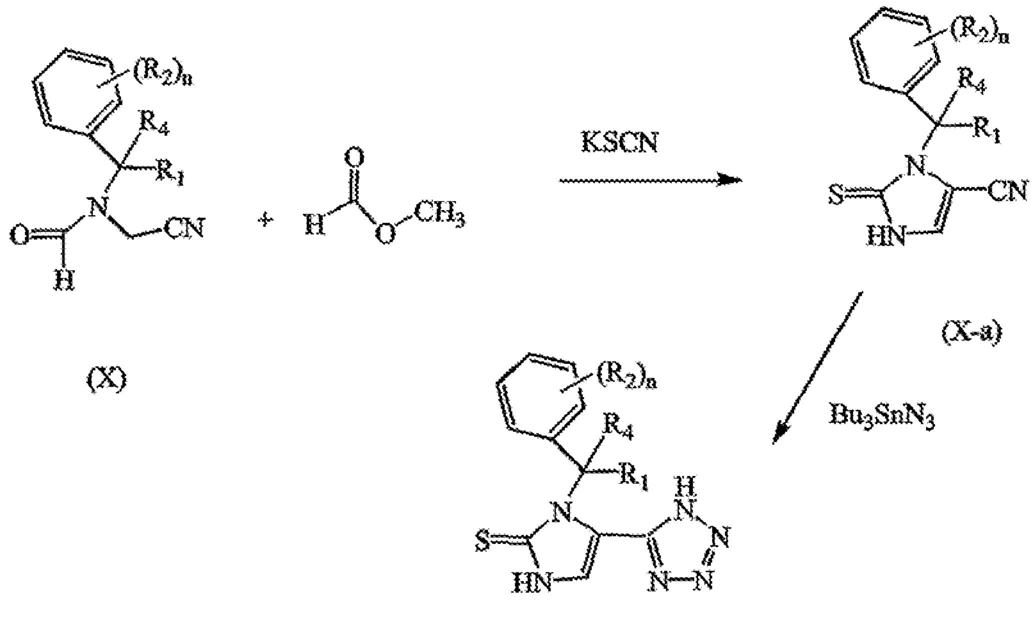


(X)

(X-a)

(I-a-1)

avec $R_1$, $R_2$, $R_4$ et n tels que définis dans la revendication 1 ;

ou, si souhaitée, la conversion de composés de la formule (I) l'un vers l'autre à la suite de transformations connues dans l'art et en outre, si souhaitée, la conversion des composés de la formule (I) en un sel d'addition acide non toxique et thérapeutiquement actif par traitement avec un acide,
ou en un sel d'addition basique non toxique et thérapeutiquement actif par traitement avec une base ou, inversement, la conversion de la forme du sel d'addition acide en la base libre par traitement avec un alcali, ou la conversion du sel d'addition basique en l'acide libre par traitement avec un acide ; et, si souhaitée, la préparation de formes stéréochimiquement isomères, d'amines quaternaires ou de N-oxydes de celles-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02066458 A
- WO 03097633 A
- FR 1487326
- FR 6751 M
- US 3850944 A
- EP 0277384 A
- WO 2004069809 A
- WO 2004069810 A
- US 20030149081 A

### Non-patent literature cited in the description


- **CHARO et al.** *PNAS,* 1994
- **CHENG ; PRUSOFF.** *Biochem. Pharmacol.,* 1973, vol. 22, 3099-3108